# EUROPEAN PATENT APPLICATION

(11) **EP 4 541 444 A1**
(43) Date of publication of application: **23.04.2025**
(21) Application number: 23204843.9
(22) Date of filing: 20.10.2023
(51) Int. Cl.: B01D 15/38, C07K 14/31, C07K 16/00

(54) **FUSION PROTEIN FOR AFFINITY CAPTURE**

(71) Applicant: Cytiva BioProcess R&D AB, 751 84 Uppsala (SE)
(72) Inventor: JONSSON, Andreas, L. M., 751 84 Uppsala (SE); MYHRINDER, Gustav, 751 84 Uppsala (SE); MELIN, Ellen, 751 84 Uppsala (SE); JÄRVER, Peter, 751 84 Uppsala (SE); ÅSTRAND, Mikael, C., 751 84 Uppsala (SE)
(74) Representative: Spjuth, Nora Sofia

(57) **Abstract**

A fusion protein comprising at least one first polypeptide moiety and at least one second polypeptide moiety, wherein the first polypeptide moiety is a single-chain polypeptide capable of binding a target entity, and the second polypeptide moiety is a stabilizing polypeptide and comprises a single-chain α-helix-containing domain, wherein the second polypeptide moiety does not have binding affinity for said target entity. The presence of the second polypeptide moiety can improve at least one property of the fusion protein as compared to the property of the first polypeptide moiety alone, wherein said improved property is selected from the group consisting of: alkaline stability, recombinant protein expression and coupling to a support.

## Description

### TECHNICAL FIELD

The present invention relates to a fusion protein capable of affinity interaction with a target entity, and methods of use thereof for *in vitro* or industrial applications e.g. for the purpose of affinity separation. The invention further relates to an adsorbent material comprising the fusion protein and to a separation method.

### BACKGROUND

Binding events between molecules based on affinity, where a ligand and a target entity interact in a "lock-key" fashion, are utilized in numerous therapeutic and non-therapeutic applications. Non-therapeutic applications include e.g. *in vitro* detection, analysis and separation of a target or analyte present in a sample.

An affinity ligand is capable of selectively and reversibly binding the target entity. General examples of interactions are e.g., enzyme-substrate interaction, biotin-avidin interaction, antibody-antigen interaction, etc. In many applications, the affinity ligand can be immobilized on a support and used for capture of the target entity, e.g. for the purpose of detection or purification.

Affinity chromatography is a specific mode of chromatography which utilizes the selectivity of the interaction between the ligand and the target entity. The affinity ligand is immobilized on a chromatographic support material, also referred to as the stationary phase. When contacted with a sample containing the target entity under binding conditions, the ligand selectively binds the target entity, while other species of the sample can be washed away. The captured target entity can then be eluted, typically by changing buffer conditions, such as conductivity or salt concentration, and/or pH. After subsequent cleaning and regeneration of the chromatography material, the chromatography material can be used for a new cycle of affinity purification. Affinity chromatography can potentially yield a target entity of very high purity.

Affinity chromatography is frequently used in the purification of biomolecules, including biopharmaceuticals and such as monoclonal antibodies, antibody fragments and recombinant proteins, as well as nucleic acids and viral particles for use in vaccine production or gene therapy, for instance.

Preparative chromatography, as well as many analytical applications using a support having the affinity ligand immobilized thereon, require comprehensive attention to definite removal of contaminants from the support between capture/purification cycles. Such contaminants can for example be non-eluted molecules adsorbed to the support or stationary phase, such as non-desired biomolecules or microorganisms, including for example proteins, carbohydrates, lipids, bacteria and viruses. In affinity chromatography, the removal of such contaminants from the support is usually performed after a first elution of the desired product in order to regenerate the stationary phase before subsequent use. Such removal usually involves a procedure known as cleaning-in-place (ClP), wherein agents capable of eluting contaminants from the stationary phase are used. One such class of agents often used is alkaline solutions that are passed over said stationary phase. At present the most extensively used cleaning and sanitizing agent is NaOH, and the concentration thereof can range from 0.05 M up to e.g. 1 M, depending on the degree and nature of contamination. This strategy is associated with exposing the stationary phase to solutions with pH values of about 13 and above. For many affinity chromatography materials containing proteinaceous affinity ligands such alkaline environment is a very harsh condition and consequently results in decreased capacities owing to instability of the ligand to the high pH involved.

Affinity reagents based on staphylococcal protein A (SpA), which is the most widely used affinity medium for isolation of immunoglobulins and their fragments, have been developed to better withstand alkaline conditions (see for example WO2003080655A1, WO 2016/079033A1 and WO2022/013272). Meanwhile, there is a great need in the art for improved affinity ligands capable of binding to bioentities other than immunoglobulins.

### SUMMARY OF THE INVENTION

It is an object of the invention to overcome or at least partly alleviate drawbacks of the prior art.

Accordingly, it is an object of the invention to provide improved affinity ligands useful for affinity capture of various target entities, such as for the purpose of detection or separation.

This and other objects are achieved by a fusion protein, wherein a polypeptide moiety that is capable of binding a target entity is fused to a second polypeptide moiety.

Accordingly, in one aspect, the invention provides a fusion protein comprising at least one first polypeptide moiety and at least one second polypeptide moiety, wherein the first polypeptide moiety is a single-chain polypeptide capable of binding a target entity, and the second polypeptide moiety comprises a single-chain α-helix-containing domain, wherein the second polypeptide moiety does not have binding affinity for said target entity.

The presence of the second polypeptide moiety can improve at least one property of the fusion protein as compared to the property of the first polypeptide moiety alone. The improved property may optionally be selected from the group consisting of: alkaline stability, recombinant protein expression and coupling to a support. The second polypeptide moiety may be a stabilizing polypeptide. The second moiety may be an alkaline stable polypeptide, as defined herein.

The first polypeptide moiety may be a natural or synthetic antibody domain or antibody fragment or a modified variant thereof. such as is a single-chain variable fragment or a single-domain antibody (sdAb), or a variant thereof. Typically the first polypeptide moiety alone has an alkali stability that is lower than the second polypeptide moiety alone.

The second polypeptide moiety may be an α-helix bundle domain, and optionally is or is derived from a protein domain of SpA. The second polypeptide moiety may have reduced affinity the Fc and VH3 regions of trastuzumab.

The fusion protein may be provided as a multimeric fusion protein comprising at least two of said first polypeptide moiety, and/or at least two of said second polypeptide moiety.

In another aspect the invention provides the use of an alkaline stable polypeptide comprising a single-chain α-helix-containing domain to improve the *in vitro* alkaline stability of a single-chain polypeptide capable of binding a target entity. The use involves fusion of the polypeptide comprising a single-chain α-helix-containing domain, optionally via a linker peptide, to the C-terminus of said single-chain polypeptide capable of binding a target entity.

In another aspect the invention provides an adsorbent material comprising the fusion protein as described herein coupled to a support. The support may be selected from the group consisting of a chip, a plate, a well, a sheet, a fiber, a particle, a bead, a fibrous matrix, a membrane, a filter and a porous monolith. The support may be a chromatography matrix.

In other aspects the invention provides the use of the fusion protein or use of the adsorbent material, for detection or separation of a target entity, e.g. separation from a sample comprising the target entity and other components.

In another aspect there is provided a method of separation comprising the steps of: providing an adsorbent material as described herein, wherein the fusion protein has a binding capacity for a target entity; contacting the adsorbent material with a liquid sample comprising said target entity under conditions allowing the target entity to bind to the first polypeptide of the fusion protein; optionally washing the adsorbent material; eluting the target entity from the adsorbent material; and cleaning the adsorbent material with a cleaning liquid. The cleaning liquid is typically alkaline, and may e.g. comprise from 0.05 to 0.5 M of NaOH or KOH.

In other aspects the disclosure provides an isolated nucleic acid sequence encoding the fusion protein, an expression vector comprising the nucleic acid sequence, and a recombinant cell comprising the expression vector, as well as a method of producing the fusion protein.

Preferred aspects of the present disclosure are described below in the detailed description, examples, list of itemized embodiments, and in the dependent claims. It is noted that the invention relates to all possible combinations of features recited in the claims.

### BRIEF DESCRIPTION OF THE DRAWINGS

These and other aspects of the present invention will now be described in more detail, with reference to the appended drawings in which:
Fig. 1A-B are schematic illustrations of a fusion protein according to embodiments of the invention.
Fig. 2A-B are schematic illustrations of multimeric fusion proteins according to various embodiments of the invention.
Fig. 3A is a graph plotting the AAV9 binding response of a fusion protein (solid line) and a reference (dashed line), respectively, against the number of binding cycles on Biacore, each cycle involving 0.5 M NaOH exposure. The binding response was normalized against the response of the second cycle (i.e., first cycle excluded). Fig. 3B shows the binding response from the same experiment, normalized to the response of the first cycles. (Example 2A)
Fig. 4A is a picture of a PAGE gel demonstrating stronger protein expression of multimeric fusion proteins having a stabilizing polypeptide, over a reference lacking a stabilizing polypeptide. Fig. 4B is a graph plotting the AAV9 binding response of the multimeric fusion proteins and the reference, respectively, against the number of binding cycles on Biacore, each cycle involving 0.3 M NaOH exposure. The binding response was normalized against the response of the second cycle (i.e., first cycle excluded). (Example 2B)
Fig. 5A-C show the target binding responses obtained for increasing number of cycles involving NaOH exposure for fusion proteins capable of binding AAV9 (Fig. 5A), GFP (Fig. 5B) or EGFR (Fig. 5C), respectively. The responses were normalized to the response of the first cycle. (Example 4A)
Fig. 6A-C show target binding responses obtained for increasing number of cycles involving NaOH exposure for fusion proteins capable of binding AAV9 (Fig. 6A), GFP (Fig. 6B) or EGFR (Fig. 6C), respectively. The responses were normalized to the response of the first cycle. (Example 4B)
Fig. 7A is a bar chart showing the binding response to target (GFP), and immunoglobulins (trastuzumab, Gammanorm) obtained for increasing number of cycles involving NaOH exposure for a fusion protein according to the invention. Fig. 7B shows the average values for a group of fusion proteins having variations in the amino acid sequence of the stabilizing polypeptide. (Example 3)
Fig. 8 is a graph showing the target binding response (not normalized) obtained for increasing number of cycles involving NaOH exposure for a fusion protein comprising an IgG-binding polypeptide domain as the first polypeptide moiety (Example 5).
Fig. 9A-C are graphs showing the target binding response (not normalized) obtained for increasing number of cycles involving NaOH exposure for fusion proteins comprising IgG-binding polypeptide domains (SpA domain C, D, E) as the first polypeptide moiety (Example 6).
Fig. 10A-B are graphs showing the target binding response (not normalized) obtained for increasing number of cycles involving NaOH exposure for fusion proteins having an scFv as the first polypeptide moiety (Example 7).
Fig. 11A is a chromatogram showing the elution peak from a chromatography column using a fusion protein according to embodiments of the present invention as affinity ligand immobilized on the chromatography matrix. Fig. 11B is a closer view of the elution peak.
Fig. 12 is a photograph of an SDS-PAGE gel showing the protein content of the eluate fractions from the chromatography run of Example 8.
Fig. 13 is a graph plotting the dynamic binding capacity as evaluated in Example 8.

As illustrated in the figures, some features and parts may be exaggerated for illustrative purposes and, thus, are provided to illustrate the general structures of embodiments of the present invention. Like reference numerals refer to like elements throughout.

### DEFINITIONS

As used herein, the terms "peptide" and "polypeptide" are used synonymously herein to refer to compounds formed of sequences of amino acids, without restriction as to size. "Protein" may be used to refer to the larger compounds of this class. Amino acid sequences are written left to right in the direction from the amino (N) to the carboxy (C) terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V). "Peptides" include any oligopeptide, polypeptide, gene product, expression product, or protein. A peptide is comprised of consecutive amino acids and encompasses naturally occurring or synthetic molecules. In addition, as used herein, the term "peptide" refers to amino acids joined to each other by peptide bonds or modified peptide bonds, e.g., peptide isosteres, etc. and may contain modified amino acids other than the 20 gene-encoded amino acids. The peptides can be modified by either natural processes, such as post-translational processing, or by chemical modification techniques which are well known in the art.

A "single-chain polypeptide" herein refers to a polypeptide that is formed of a single amino acid sequence, in which the amino acid residues are connected via peptide bonds. A single-chain polypeptide may, upon folding, additionally form other internal bonds such as disulfide bonds.

The expression "antigen-binding polypeptide" generally refers to a polypeptide that possesses at least one binding region, such as at least two, such as three binding regions, such that the polypeptide has a binding affinity for a molecule (referred to as antigen or "target"). An antigen-binding polypeptide may have any protein structure, as long as there is a binding affinity for the antigen. However, some classes of antigen-binding polypeptides that have frequently been exploited and used as a basis for engineered antigen-binding polypeptides often have a scaffold or framework structure that is generally conserved between antigen-binding polypeptides of the same class but which bind to different antigens. The binding region(s) of such an antigen-binding molecule can typically be engineered or evolved to bind to a particular antigen, while the scaffold or framework structure remains essentially the same. Non-limiting examples of antigen-binding polypeptides include natural or engineered (i) antibodies, such as monoclonal antibodies, (ii) antibody fragments that contain the light chain and/or heavy chain variable region(s) with complementarity determining regions (CDRs), such as Fab (fragment antigen-binding), Fv (variable fragment), scFab (single chain antigen-binding fragment) and scFv (single chain variable fragment), iii) single-domain antibodies, and iv) polypeptides having a scaffold derived from bacteria, such as immunoglobulin-binding bacterial proteins (such as *Finegoldia magna* protein L or staphylococcal protein A and protein G) or domains thereof, including the wild types as well as variants in which one or more of the antigen binding regions have been engineered.

The expression "single-domain polypeptide" or "single-domain amino acid sequence" refers to a polypeptide which forms an individual protein domain, and which does not comprise any other individual protein domain. Examples of single-domain polypeptides include antibody fragments such as heavy chain variable domain and light chain variable domain, single-domain antibodies (sdAb), albumin binding domain (ABD) and polypeptides derived from a bacterial protein domain, such as the A, B, C, D, E or Z domain of SpA or a domain of protein L. Single-domain antibodies are particularly contemplated. A single-domain polypeptide typically consists of a single-chain polypeptide. A single-domain polypeptide is in itself monomeric, but it may form part of a multimer, as described elsewhere herein. A single-domain polypeptide may be joined to another polypeptide, for example via a peptide bond or via a disulfide bond. Preferably, in the context of the present disclosure, an antigen-binding polypeptide may consist of a single polypeptide chain.

The term "single-domain antibody" refers to a variable domain which is or is derived from a heavy chain variable domain (VH) of an antibody devoid of light chains. Hence, a single domain antibody lacks antibody light chains entirely, including the light chain variable domain (VL). A single-domain antibody may also lack such structural features that are needed for a functional VH/VL interaction, and which are present in, e.g., the conventional VH of IgG1. Hence, a single domain antibody does not form part of a dimeric structure with an antibody light chain variable domain. Single-domain antibodies include antibodies naturally devoid of antibody light chains, such as sdAb derived from IgG2 or IgG3 of camelids, e.g. dromedary, camel, llama and alpaca, which are also referred to as VHH (heavy chain variable domain of a heavy chain antibody). Single domain antibodies naturally devoid of light chains also include so-called VNARs (variable new antigen receptors), which are antibodies derived from cartilaginous fishes, such as sharks. Alternatively, single domain antibodies may be synthetic variants based on an amino acid sequence of an antibody heavy chain variable domain from a species that naturally does not produce sdAb (e.g. cow, rat, mouse or rabbit) and which are modified e.g. with respect to amino acids in the natural VH/VL interaction of such antibodies, so as to mimic a natural sdAb. Such modification may involve substitution of amino acids of the VH/VL interface region to increase the hydrophilicity or solubility.

Generally, terms such as "polypeptide", "protein", "antibody", "single-domain polypeptide", "single-domain antibody", etc. also include synthetic variants that are recombinantly produced and whose amino acid sequence may have been modified in relation to the amino acid sequence of a naturally occurring counterpart. Where such modified variants are particularly intended, the term "variant" may also be used. The term "wild type" or "wt" refers to the typical naturally occurring form.

Complementary determining regions (CDRs) are hypervariable regions of an antibody or part thereof which participate in binding to a target epitope. The CDRs are typically defined by their separate amino acid sequences, although in an antibody, the CDRs together form a three-dimensional binding site for the target antigen. A variable domain of an antibody heavy chain (VH) has three CDRs, referred to as CDR1, CDR2 and CDR3, as positioned from the N terminus of the polypeptide chain. The sequence length of CDRs may vary, and CDR1, CDR2 and especially CDR3 of a VH may be of different lengths.

The portions of an antibody VH not forming the CDRs are referred to as framework regions. The framework regions are typically four and referred to as framework region (FW) 1 to 4, as counted from the N terminus. The framework regions are responsible for the general secondary and tertiary structure of the domain and thus for positioning and orientation of the CDR regions. The framework regions may be referred to as a scaffold structure. Although part of the variable domain, the framework regions are less variable than the CDRs. Generally, amino acid sequence variations that do not significantly alter the secondary or tertiary structure of the framework may however be tolerated. Some parts or amino acid positions of the framework regions may be conserved. The general stability of the framework regions allows for a high degree of variation in the CDRs.

As used herein, the term "ligand" is a molecule that has a known or unknown affinity for a given entity. The term "ligand" may herein be used interchangeably with the terms "affinity ligand", "selective binding molecule", "selective binding partner", "capturing molecule" and "capturing agent". "Affinity ligand" refers to a moiety or molecule that binds reversibly and selectively or preferentially with high affinity to a target entity through a specific interaction with a binding site of the component. In the context of the present invention, an affinity ligand is a polypeptide, and may also be referred to as an "affinity protein". An affinity ligand may be immobilized to a solid support such as a resin.

The term "target entity" herein refers to an entity that forms a specific binding partner to the ligand, and may also be referred to as an "analyte".

As used herein, "affinity" in the context of polypeptides denotes the strength of interaction between two molecules, wherein at least one molecule is a polypeptide. The interaction is selective, i.e. discriminates between the affinity binding partner and other molecules present. Binding affinity is also expressed as the dissociation equilibrium constant (KD).

As used herein, the term "binding capacity" refers to the capability of a ligand to bind the target molecules when the ligand is immobilized on a surface. The binding capacity of a ligand can differ depending on the type of surface on which it is immobilized. For the purpose of the present invention, the binding capacity can be measured by surface plasmon resonance (SPR) technology using e.g. a Biacore instrument as described in the present examples. The binding capacity for a certain concentration of analyte is influenced by the density of immobilized ligands on the surface. In the context of the present invention the binding capacity is preferably determined at a ligand density of at least 3000 response units (RU), such as in the range of from 3000-4000 RU.

The term "dynamic binding capacity" (abbreviated "DBC") of a chromatography column in the context of protein purification is the binding capacity under operating conditions, i.e., in a packed affinity chromatography column during sample application. The DBC of a chromatography resin is expressed as the amount of analyte that binds to the resin under given flow conditions before a significant breakthrough of unbound analyte occurs. DBC is determined by loading a sample containing a known concentration of the analyte and monitoring the flow-through. The analyte will bind to the resin to a certain break point before unbound analyte will flow through the column. The DBC can be determined on the breakthrough curve at a loss of, for example, 10% analyte. This is referred to as the QB10% value. A sample is applied to a chromatography resin column during a specific residence time and the dynamic binding capacity for each resin is calculated at 10% of the breakthrough capacity i.e., the amount of analyte sample that is loaded onto the column until the concentration of analyte in the column effluent is 10% of the analyte sample concentration in the feed. If the dynamic binding capacity for each resin is calculated at 80% of the breakthrough capacity, this is referred to as the QB80% value.

The term "alkaline stability" as used herein, refers to a property of a polypeptide which relates to its ability to withstand alkali exposure without deleterious effect on structure and/or function of the polypeptide. For polypeptides capable of binding a target entity, the alkaline stability is determined based on the affinity for the target entity after exposure to an alkaline agent, typically NaOH. The alkaline stability of an antigen-binding polypeptide can be evaluated immobilizing the polypeptide on a support and measuring the target binding capacity before and after one or more cycles of exposure to NaOH, using methods described in the Examples below. In particular the evaluation is made on an SPR chip onto which the polypeptide is immobilized by covalent coupling with thiol, N-hydroxysuccinimide, streptavidin-biotin binding, or another coupling that is alkali resistant in itself, at a ligand density corresponding to at least 1000 RU, preferably at least about 3000 RU, and for a predetermined analyte concentration. Often, the first alkali cleaning cycle may have a unique, large impact on binding capacity, e.g. due to removal of non-covalently bound polypeptide from the support surface, and therefore the binding capacity is sometimes represented as normalized to the binding capacity measured after the first cycle of alkali exposure (i.e. normalized to the binding response of the second cycle).

In the present context, a polypeptide can be considered to be alkaline stable if, after 10 cleaning cycles, preferably after 15 cleaning cycles, the antigen-binding polypeptide of the fusion protein retains at least 50 % of the target binding capacity compared to its binding capacity after the first cleaning cycle (i.e. disregarding the binding capacity of the first cycle, prior to the first alkali exposure event), where a cleaning cycle involves 600 s of exposure to at least 0.3 M NaOH, such as at least 0.5 M NaOH.

Any polypeptide capable of binding to an antigen and which can be immobilized on a support can be assessed in this way with regard to alkaline stability. For a polypeptide which does not have a binding affinity for other entities (such as a stabilizing polypeptide according to some embodiments of the present invention based on an SpA protein domain for which IgG binding capability has been abolished), it is envisaged that the alkaline stability of a stabilizing polypeptide which does not have a binding affinity for another entity such as IgG can be assessed by providing a separate affinity binder capable of binding the stabilizing polypeptide, immobilizing said separate affinity binder on a support (e.g., an SPR chip or a chromatography matrix) and assessing the interaction (e.g., binding response or dynamic binding capacity QB10%) with the stabilizing polypeptide as the analyte before and after NaOH exposure similarly to what is described elsewhere herein.

The term "solid support" herein refers to a non-aqueous matrix of a solid phase material. Suitable solid phase materials include, but are not limited to, glass, silica (e.g., silica gel), polysaccharides (e.g., a polysaccharide matrix) such as agarose and cellulose, organic polymers such as polyacrylamide, methylmethacrylate, and polystyrenedivinylbenzene copolymers. The solid phase can be of porous or nonporous character and can be compressible or incompressible. For example, the solid phase can be a polymeric matrix or an agarose particle or bead. Preferred solid support materials will be physically and chemically resilient to the conditions employed in a separation process including pumping and cross-flow filtration, and temperatures, pH, and other aspects of the liquids employed.

The term "surface" herein means all external surfaces of a solid structure. In the case of a porous support, such as a bead used e.g. in the field of chromatography, the term "surface" includes outer surfaces as well as pore surfaces. In the case of a fibrous membrane, the term "surface" encompasses the external surface of individual fibers.

The term "separation matrix" is used herein to denote a material comprising a solid support to which one or more ligand(s) have been coupled. The ligand(s) are capable of binding target entities herein also called analytes, which are to be separated from their surrounding, e.g. a liquid sample, and/or which are to be separated from other components present in the liquid sample. A separation matrix may be employed in various contexts, including analytical assays and purification of target for analytical or preparative purposes. The type of support may be selected depending on the intended use.

A separation matrix may further comprise a compound which couples the ligand(s) to the support. The terms "spacer", "extender", and "surface extender" may be used to describe such a compound, as further described herein. The term "resin" is sometimes used for a separation matrix in this field. The terms "chromatography material" and "chromatography matrix" are used herein to denote a type of separation matrix. An affinity separation matrix is a separation matrix where the ligand is an affinity ligand.

The term "spacer" refers to a peptide or other chemical linkage or element that extends the structure of an entity. A spacer attached to a polypeptide, in particular an amino acid spacer, may be provided at the N terminus or the C terminus of the polypeptide. A spacer may connect a polypeptide to a solid support. Suitable spacers for coupling a polypeptide to a support may generally be any spacer used in the art to connect peptides, proteins or other organic molecules to a support. A spacer may also serve to connect two polypeptides or polypeptide domains. A spacer connecting two polypeptide moieties may also be referred to as a linker.

The term "fusion protein" refers to a protein formed of two, or more than two, separate proteins (fusion partners) which are produced by recombinant protein expression as a single polypeptide containing the fusion partners. The fusion partners may be joined sequentially one after the other. The genes encoding the respective proteins are joined at the genetic level. The fusion partners typically do not naturally occur as fused to each other. A fusion protein may contain additional amino acids sequences, such as linkers, in between the fusion partners.

As used herein a "linker" refers to a peptide or other chemical linkage that functions to link otherwise independent functional domains. A linker may be located between two polypeptide units, monomers or domains, such as between a ligand (e.g., a sdAb) and another polypeptide component containing an otherwise independent functional or structural domain, or between two ligands. Suitable linkers for coupling two or more linked units may generally be any linker used in the art to link peptides, proteins or other organic molecules. A linker peptide may be a stretch of amino acids preferably ranging from 1 to 20 amino acids, such as 2-15, such as 2-8, 2-4 or 4-12.

The term "% identity" or "% sequence identity", as used throughout the disclosure, may for example be calculated as follows. The query sequence is aligned to the target sequence using the CLUSTAL W algorithm (Thompson et al, Nucleic Acids Research, 22: 4673-4680 (1994)). A comparison is made over the window corresponding to the shortest of the aligned sequences. The shortest of the aligned sequences may in some instances be the target sequence. In other instances, the query sequence may constitute the shortest of the aligned sequences. The amino acid residues at each position are compared and the percentage of positions in the query sequence that have identical correspondences in the target sequence is reported as % identity.

### DETAILED DESCRIPTION

The present inventors surprisingly found that the alkaline stability of an otherwise alkalisensitive affinity ligand could potentially be improved by fusion with an α-helix-containing polypeptide domain, such as a polypeptide domain corresponding to or based on a protein domain of SpA. In particular, alkali stabilized variants of SpA domains B, C and Z are contemplated for this purpose. Such modified variants are described in WO2003080655A1 and WO2016079033A1, incorporated herein in their entireties.

As demonstrated herein, a fusion partner, referred to as a second polypeptide moiety, may improve the alkaline stability of an affinity ligand polypeptide, such that the resulting fusion protein has an alkaline stability that is higher than the alkaline stability of the affinity ligand (herein referred to as the first polypeptide moiety) alone. The second polypeptide moiety may thus be referred to as a stabilizing polypeptide.

Another benefit of the present fusion protein is that the second polypeptide moiety may facilitate or increase protein expression of the fusion protein in a recombinant host cell, especially in cases where the first polypeptide moiety alone is difficult to express.

Furthermore, the second polypeptide moiety also has the potential to function as a purification tag, in particular for purification by affinity chromatography using a ligand with affinity for the second polypeptide moiety, and/or a means for coupling of the first polypeptide moiety to a support, potentially with the function of a spacer, and/or to provide a means of detection, e.g. by offering a binding site for a labelled binding partner. The second moiety may hence also in more general terms be referred to as a helper protein.

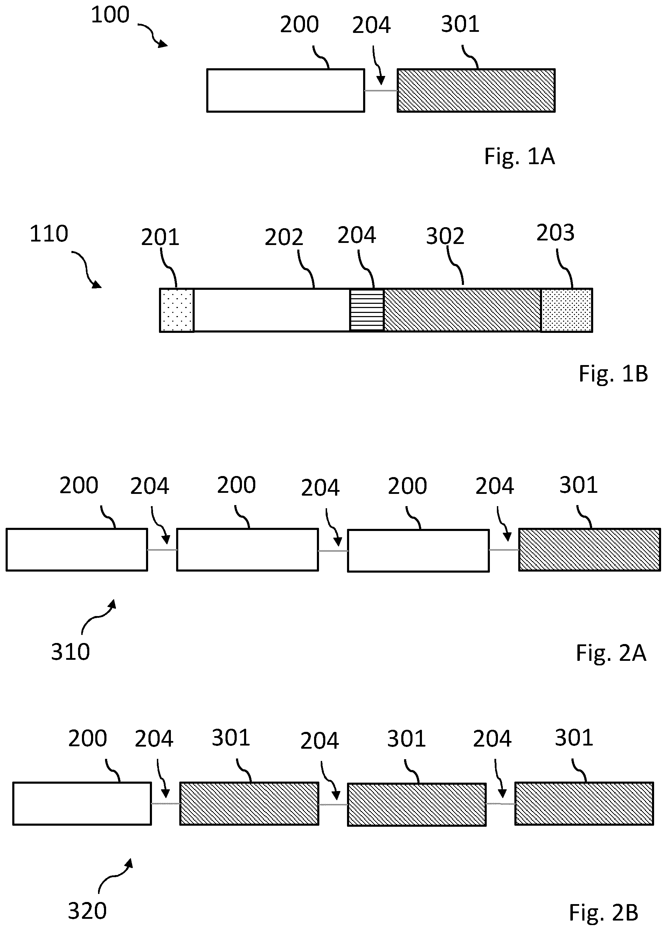

Fig. 1A schematically illustrates a fusion protein 100 comprising, in the N- to C-terminal direction, a first polypeptide moiety 200 and a second polypeptide moiety 301, joined by an optional peptide linker 204. In this figure, the second polypeptide moiety 301 is located C-terminally of the first polypeptide moiety 200. It is also contemplated that the second polypeptide moiety may be located N-terminally of the first polypeptide moiety.

Fig. 1B is an alternative illustration of a fusion protein according to embodiments of the invention. The fusion protein 110 comprises N-terminal additional amino acids 201, such as a leader peptide, and a single-chain polypeptide 202 capable of binding a target entity. At the C-terminus of the single-chain polypeptide 202, a linker 204 is provided followed by a stabilizing polypeptide 302, ending in an optional C-terminal amino acid sequence 203, which can be e.g. a purification tag and may include a linker (not shown in the figure) linking the sequence 203 to the stabilizing polypeptide 302.

The first polypeptide moiety 200 may comprise or consist of only a single-chain polypeptide 202 as illustrated in Fig. 1B, or may comprise or consist of a single-chain polypeptide 202 and additional amino acids, such as the additional amino acid sequence 201 and/or linker 204. Similarly, the second polypeptide moiety 301 of Fig. 1A may comprise or consist of a stabilizing polypeptide 202, or may comprise or consist of a stabilizing polypeptide 202 and additional N- or C-terminal amino acids, such a linker 204 and/or the C-terminal sequence 203.

The fusion protein of the present disclosure may be a multimeric protein, meaning that it may comprise more than one affinity ligand, or more than stabilizing polypeptide. Multimeric variants containing multiple copies of an affinity protein (the first polypeptide moiety) may be advantageous in that they can provide an increased binding capacity in relation to a fusion protein containing only one copy. A multimeric fusion protein containing multiple stabilizing polypeptides may provide an improved stability, such as alkaline stability, in relation to a fusion protein comprising a single stabilizing polypeptide.

Thus, the fusion protein of the present disclosure may optionally be provided in the form of a multimeric fusion protein ("multimer") containing at least two of said first polypeptide moiety, and/or at least two of said second polypeptide moiety, as schematically illustrated in Fig. 2A-B. In Fig. 2A, a multimeric fusion protein 310 comprises multiple units of the first polypeptide moiety 200 arranged sequentially in the N-to C-terminal direction, and which may optionally be joined to one another by linker sequences 204, which may be the same or different for each occurrence. The first polypeptide moieties 200 may be identical, or may be different from one another with regard to their amino acid sequence. Fig. 2A shows three polypeptide moieties 200 ("trimer"), but it is envisaged that the fusion protein may comprise any suitable number of said first polypeptide moiety 200. For example, the fusion protein may contain at least two of the first polypeptide moiety, or at least 3, 4, 5 or 6 units of the first polypeptide moiety. The fusion protein may contain e.g. up to 10 first polypeptide moieties 200. The fusion protein may optionally comprise N-terminal and/or C-terminal amino acids as described elsewhere herein.

Furthermore, the fusion protein may contain at least 2, such as 3, 4, 5 or 6 units, e.g. up to 10 units, of the second polypeptide moiety. Fig. 2B illustrates a fusion protein 320 containing one first polypeptide moiety 200 and three second polypeptide moieties 301. Linkers 204 are optional, and may be the same or different for each occurrence. The second polypeptide moieties 301 may be identical, or may be different with regard to their amino acid sequences. In other embodiments, the fusion protein may contain two stabilizing polypeptides and any suitable number of the first polypeptide moiety 200 as described above. For example, the fusion protein may contain one first polypeptide moiety flanked by two stabilizing polypeptides. Where a fusion protein comprises multiple first polypeptide moieties and multiple stabilizing polypeptides, for example 2 or 3 of each, it is envisaged that the different polypeptide moieties 200, 301 may be arranged in an alternating fashion instead of polypeptides of the same kind being arranged sequentially as depicted in Fig. 2A (for the first polypeptide moiety 200) and Fig. 2B (for the stabilizing polypeptide 301). For example, a multimeric fusion protein containing two units of each polypeptide may have the following general structure: [first polypeptide moiety]-[second polypeptide moiety]-[first polypeptide moiety]-[second polypeptide moiety]. In embodiments where a fusion protein comprises at least two target-binding polypeptides, i.e., at least two first polypeptide moieties, the target-binding polypeptides may be the same or different, and if different, may be directed against (capable of binding) different targets.

In examples where the fusion protein comprises at least two second polypeptide moieties, it may be preferred that at least one is located at the C terminus of the fusion protein. Optionally, at least two second polypeptide moieties may be arranged sequentially at the C terminus of the fusion protein.

At the N-terminus of a fusion protein, such as a multimeric fusion protein, there may be a short amino acid sequence, such as a leader or signal peptide. At the C-terminus a fusion protein, such as a multimeric fusion protein, a tag or spacer, or another short amino acid sequence may optionally be provided as desired.

In certain embodiments, the second polypeptide moiety is not located at or near the N-terminus of the fusion protein.

For clarity, any reference herein to the fusion protein of the present invention encompasses also multimeric variants thereof, unless stated otherwise.

The first polypeptide moiety of the fusion protein is capable of binding a target entity, and may be referred to as an affinity ligand or an affinity binder for said target or directed to said target. The first polypeptide moiety may also be referred to as an antigen-binding polypeptide or protein, or a target-binding polypeptide or protein. Thus, the fusion protein is capable of binding said target entity, by interaction between the first polypeptide moiety and said target entity.

The target entity may be a biological entity such as a biomolecule or a larger structure. The target entity may a therapeutic molecule or entity, intended for use in a pharmaceutical product or in therapy. The target entity may be intended as a vehicle, such as a cell or gene therapy vector. For example, the biological entity can be an antibody or a part thereof, such as a monoclonal antibody, an antibody fragment or an antibody domain. As another example, the biological entity may be a protein, such as a recombinant protein. Alternatively, the biological entity can be a virus particle or a viral vector, such as an adenovirus, a retrovirus (y-retroviruses and lentiviruses), a poxvirus, an adeno-associated virus (AAV), a baculovirus, or a herpes simplex virus. For example, the target entity may be an adeno-associated virus (AAV), such as an AAV serotype 1, 2, 3, 4, 5, 6, 7, 8, 9, 10 (AAVrh10), 11 or 12. Other examples of biological entities include exosomes and lipid nanoparticles. In yet other examples, the biological entity may be a nucleic acid molecule, such as DNA or RNA, e.g., mRNA. The first polypeptide moiety may have an affinity for a molecule exposed on the surface of a biological entity, for example a viral capsid or envelope protein, a membrane receptor, or a surface exposed marker.

Generally, antigen binding polypeptides directed to a desired target can be identified or generated by any method known to persons of skill in the art, including but not limited to immunization of animals (e.g., mice, rabbits or camelids) using the antigen of interest, by phage display, ribosome display, cell surface display or bacterial display, by simulation *in silico,* and combinations thereof. When an antigen-binding polypeptide has been identified, the amino acid sequence, optionally modified, can be used to design a fusion protein construct together with the second polypeptide moiety as disclosed herein. For example, methods for generating high affinity antigen-binding single-domain antibodies are described in, for instance, Schmitz et al, 2013, Structure 21, 1214-1224, and in Fleetwood et al., Cell.Mol. Life Sci. (2013) 70:1081-1093.

The first polypeptide moiety is typically a single-chain polypeptide, and may form a polypeptide domain.

In some embodiments, the first polypeptide moiety may have a secondary structure comprising or predominantly containing β-strands, β-sheets and/or a β-sandwich. However, having a structure dominated by β-strand or β-sheets does not exclude the presence of other structural elements such as α-helices, loops or unstructured regions. Examples of protein domains characterized by a secondary structure of β-strands or β-sheets include antibody variable domains. Thus, the first polypeptide moiety may comprise a natural or synthetic variant of an antibody heavy chain variable domain and/or an antibody light chain variable domain, such as selected from: a heavy chain variable domain of a heavy chain antibody (VHH) and variants thereof, a VNAR and variants thereof, and an immunoglobulin single-chain variable fragments (scFv). The first polypeptide may thus be or be derived from a single domain antibody (sdAb). Single-domain antibodies lack the light chains of full size antibodies, and also lacks the heavy chain constant domains. Where the first polypeptide moiety is based on an antibody, it may lack antibody heavy chain constant regions.

In some embodiments, the first polypeptide moiety may have a secondary structure comprising one or more α-helices. Some polypeptides may have a mixed structure, comprising at least one beta sheet and at least one α-helix, examples of such polypeptides including Protein L protein domains and Protein G protein domains.

In other embodiments, the first polypeptide moiety may be or comprise an α-helix bundle protein domain. Examples of α-helix bundle domains include SpA protein domains and albumin binding domain (ABD). In particular, the first polypeptide moiety may comprise a polypeptide which is or is derived from a protein domain of SpA, such as domain A (SEQ ID NO: 1) domain B (SEQ ID NO:2), domain C (SEQ ID NO:3), domain D (SEQ ID NO: 4) or domain E (SEQ ID NO:5). For example, the first polypeptide moiety may comprise an amino acid sequence selected from the group consisting of SEQ ID NOs:1-7 and amino acid sequences having at least 80 % identity to any one of SEQ ID NOs:1-7.

The first polypeptide may or may not be modified to increase its alkaline stability. Hence, the first polypeptide moiety is not necessarily alkaline stable in itself.

The first polypeptide moiety, and also the fusion protein as a whole, has an affinity for a target entity. Hence, the presence second polypeptide moiety should not interfere with the binding capacity of the first polypeptide for the target molecule.

The ability of the first polypeptide moiety to bind a target entity is provided by one or more binding regions. For example, the first polypeptide moiety may comprise a single-chain polypeptide having a complementarity determining region 1 (CDR1), a complementarity determining region 2 (CDR2), and a complementarity determining region 3 (CDR3). The CDR1, CDR2 and CDR3 may be the CDRs of a natural or synthetic antibody heavy chain variable domain. The CDRs may optionally be engineered to provide desirable binding characteristics.

For example, the first polypeptide moiety may have an amino acid sequence based on an sdAb which comprises framework regions (FWRs) and complementary determining regions (CDRs), provided as follows in the N-to C-terminal direction:
[FWR1]-[CDR1]-[FWR2]-[CDR2]-[FWR3]-[CDR3]-[FWR4]

The framework regions may collectively be referred to as the "framework".

Table 1 outlines the FWRs and CDRs as defined herein in relation to the Kabat amino acid numbering system (Kabat et al., 1991, J. Immunol. 147(5), 1709-1719). For an sdAb according to embodiments of the present disclosure, Framework region 1 (FWR1) is formed of the amino acids in positions 1 to 26. Next, amino acids in Kabat positions 27 to 35d form CDR1, Kabat positions 35a-d being optional. Framework region 2 (FWR2) is formed of the amino acids in Kabat positions 36 to 49. CDR2 is formed of the amino acids in Kabat positions 50 to 58 (52a being optional). Framework region 3 (FWR3) is formed of the amino acids in Kabat positions 59 to 94. CDR3 is formed of the amino acids in Kabat positions 95 to 102 (100a-j being optional). Lastly, framework region 4 (FWR4) is formed of amino acids in Kabat positions 103 to 113.

**Table 1**

| Amino acid positions (Kabat numbering) | 1-26 | 27-35d | 36-49 | 50-58 | 59-94 | 95-102 | 103-113 |
|---|---|---|---|---|---|---|---|
| Region | FWR1 | CDR1 | FWR2 | CDR2 | FWR3 | CDR3 | FWR4 |
| Example sdAb SEQ ID NO:127 | SEQ ID NO:156 | | SEQ ID NO:178 | | SEQ ID NO:203 | | SEQ ID NO:231 |
| Example sdAb SEQ ID NO: 133 | SEQ ID NO:157 | | SEQ ID NO:179 | | SEQ ID NO:204 | | SEQ ID NO:231 |
| Example sdAb SEQ ID NO: 137 | SEQ ID NO:160 | | SEQ ID NO:182 | | SEQ ID NO:207 | | SEQ ID NO:231 |

Where the first polypeptide moiety comprises an sdAb variant, each of the framework regions may have an amino acid sequence having a certain similarity, e.g. at least 70 %, such as at least 75%, such as at least 80 %, such as at least 85 % sequence identity, to a corresponding framework region of SEQ ID NO:127, 133 or 137. The SEQ ID NOs of the individual framework regions of these sdAbs are indicated in Table 1 above.

Parts of the framework regions of an sdAb are relatively conserved and it may be preferable to make only few or no modifications in these parts in relation to e.g. SEQ ID NO: 127, 133, 137 or 139. The conserved parts are herein defined by Kabat positions 1-5, 7-14, 25-26, 41-42, 46, 48-49, 59, 61-75, 77-78, 80-82a, 82c-94 and 103-113. Optionally, each of FWR1-3, and optionally also FWR4, may have at least 80 %, such as at least 85 %, such as at least 90 %, sequence identity with the corresponding framework region of SEQ ID NO: 127, 133, 137 or 139 over the conserved amino acid positions (i.e., omitting the other positions from the comparison). As an example, FWR1 contains 26 aa, whereof 21 amino acids represent the conserved positions as defined above. Substituting 2 of these amino acids may thus result in 90.5 % identity, whereas substituting 3 amino acids may result in 85.7 % identity.

An sdAb variant may have an amino acid sequence wherein Kabat positions 1-5, 7-13, 25-26, 36-39, 41-42, 46, 48-49, 59, 61-75, 77-78, 80-82a and 82c-94 include up to 15, such as up to 14, such as up to 13, such as 12, 11, or 10 amino acid residues that are substituted in relation to at least one of SEQ ID NOs: 127, 133, 137 or 139. For example, in FWR1, positions 1-5, 7-13 and 25-26 (21 residues) may together have up to 4, such as up to 3 amino acid substitutions. In FWR2, Kabat positions 36-39, 41-42, 46 and 48-49 (14 residues) may together have 1 or 2 amino acid substitutions. In FWR3, Kabat positions 59, 61-75, 77-78, 80-82a, 82c-94 (34 residues) may have up to 6 amino acid substitutions.

For example, the first polypeptide moiety may comprise an sdAb framework having
- FWR1 selected from the group consisting of SEQ ID NOs:156-177 (FWR1 variants); and/or
- FWR2 selected from the group consisting of SEQ ID NOs:178-202 (FWR2 variants); and/or
- FWR3 selected from the group consisting of SEQ ID NOs:203-230 (FWR3 variants).

Optionally, an sdAb variant FWR1 may have E in Kabat position 1. Hence, any sdAb amino acid sequence disclosed herein in which Kabat position 1 is not E (e.g., is Q), may instead have E in Kabat position 1.

Optionally, one or more of the framework regions may be modified in relation to a corresponding natural framework region (e.g. a framework region of SEQ ID NO:127 or of SEQ ID NO:133) to provide improved properties, such as improved alkaline stability, of the framework or sdAb as such.

For example, it has been found that in some amino acid positions of the framework regions of an sdAb, certain mutations of the amino acid residue in that position are not only tolerated without compromising functionality, but may even lead to improved performance, especially an improved alkaline stability, of the resulting sdAb variant. In particular the following amino acid positions according to the Kabat numbering system have been identified as variable and potentially useful to improve alkaline stability of an sdAb variant: position 6, 14, 19, 23, 24, 40, 43, 44, 45, 47, 60, 76, 79, 82b and 89. Thus, in embodiments, the first polypeptide of the present fusion protein comprises an single-domain antibody variant comprising an amino acid sequence in which at least one of the Kabat amino acid positions 6,14,19,23,24,40,43,44,45,47,60,76,79,82b and 89 is modified in relation to the corresponding amino acid of SEQ ID NO: 127 and where the sdAb framework has increased alkaline stability compared to SEQ ID NO: 127.

Stabilizing modifications are outlined in Table 2. Thus, the first polypeptide moiety of the present fusion protein may optionally comprise a single-domain antibody variant comprising an amino acid sequence in which at least one, such as at least two, such as three, four, five, six, seven, eight, nine or ten, and preferably at least eight, of the residues in Kabat positions 19, 23, 24, 40, 43, 44, 45, 76, 79 and/or 89 are selected from the stabilizing amino acids indicated in Table 2.

**Table 2. Exemplary framework stabilizing modifications**

| Kabat position | Stabilizing amino acids | Preferred stabilizing amino acid(s) |
|---|---|---|
| 19 | R, S, K, T | R, T |
| 23 | T, S, V, A | T |
| 24 | I, V, S | |
| 40 | R, I, K, T | R, I |
| 43 | R, K, Q, E, G | R, K |
| 44 | E, Q, A, D, G | |
| 45 | R, I, L | R |
| 76 | N, Q | |
| 79 | Y, A, W, F | Y, W, F |
| 89 | V, I, A, L | V, I |

In embodiments, the first polypeptide moiety may comprise an sdAb framework having
- FWR1 selected from the group consisting of SEQ ID NOs:158-177 (FWR1 stabilized variants); and/or
- FWR2 selected from the group consisting of SEQ ID NOs:178-183 and 185-199 (FWR2 stabilized variants); and/or
- FWR3 selected from the group consisting of SEQ ID NOs: 205-211, 216-217, 220-225, 227 and 229-230 (FWR3 stabilized variants).

FWR2 stabilized variants are particularly contemplated.

Notwithstanding the above, it is envisaged that the use of a second polypeptide moiety may be very useful where the first polypeptide moiety as such has a relatively poor alkaline stability, or at least an alkaline stability that is inferior to the alkaline stability of the second polypeptide moiety as such. This may be for example when the first polypeptide moiety alone retains 75 % or less, such as 50 % or less, of its target binding capacity after at most 20 hours, such as after 10 hours, of exposure to 0.5 M NaOH, or after at most 120 cycles, such as after 60 cycles, of repeated NaOH exposure wherein a cycle of NaOH exposure involved contact with 0.5 M NaOH for 10 minutes.

The second polypeptide moiety has the ability to stabilize or enhance the first polypeptide moiety in some respect, such as with regard to alkaline stability. Hence, where the first polypeptide moiety has an alkali stability that is undesirably low for an intended use, fusion of the second polypeptide moiety to the first polypeptide moiety can provide a fusion protein that has an alkaline stability that is higher than the alkaline stability of the first polypeptide moiety alone. For this purpose, the second polypeptide moiety taken alone has an alkaline stability that is higher than the alkaline stability of the first polypeptide alone.

Alkaline stability is a great benefit for many applications, in particular where the fusion protein is to be immobilized on a solid support and subjected to alkaline conditions as part of the intended use. This is the case e.g. in affinity chromatography, as the chromatography material is conventionally subjected to cleaning using an alkaline agent (such as NaOH or KOH) between the purification cycles. Increased alkaline stability of the affinity ligand means that the chromatography material can be used for more purification cycles before binding capacity becomes unacceptably low.

The alkaline stability is usually determined based on the binding capacity after a certain extent of alkali exposure, often a certain number of alkali treatment cycles In this context, the terms "treatment", "exposure" and "cleaning" are used interchangeably. For example, "improved alkaline stability" can mean that a polypeptide can withstand a higher number of alkali cleaning cycles, or the same number of cycles but with harsher alkali conditions, without a deterioration in target binding capacity. Alternatively, "improved alkaline stability" can mean that the polypeptide will retain a higher percentage of the binding capacity after an equal number of cleaning cycles using the same conditions. The alkaline stability may be particularly relevant for a polypeptide intended to be immobilized on a solid support.

The alkali treatment may involve contact or incubation with 0.01-1 M NaOH, such as 0.05-0.5 M, 0.1-0.5 M or 0.3-0.5 M NaOH for a time period of 5-15 minutes, such as 10 minutes (600 seconds) or about 10 minutes. The milder treatment conditions, e.g. 0.01-0.1 M, may be used especially in cases where the first polypeptide moiety as such is very sensitive to alkaline conditions.

The alkaline stability of the fusion protein may in part depend on the alkaline stability of the first polypeptide moiety as such. For example, where the first polypeptide moiety alone has poor stability, the present fusion protein may provide a small yet valuable improvement, or a relatively large improvement, to a level where a more acceptable stability is obtained. For a first polypeptide moiety having medium or good alkaline stability in itself, the fusion protein may provide a smaller improvement, in relative terms. However, in such a case, the improvement may still be such as to result in excellent alkaline stability.

Notably, a fusion protein comprising a first polypeptide moiety that in itself is poorly stable might not achieve the same level of alkaline stability as a fusion protein based on a first polypeptide having a higher alkaline stability in itself. Therefore, if a very high alkaline stability is required, the alkaline stability of the first polypeptide moiety as such should not be too low.

For example, where the present fusion protein is based on a first polypeptide moiety that is highly sensitive to alkaline conditions, e.g. a first polypeptide moiety that in itself retains 50 % of its target binding capacity for very few cleaning cycles, such as only 1 or 2 cycles, the stabilizing polypeptide may serve to increase the alkaline stability such that the fusion protein retains at least 50 % binding capacity for the double amount of cycles, or retains at least 50 % binding capacity for at least 2 cycles, such as at least 3, 4, 6 or 8 cycles provided it represents an increase. A higher number of cycles with at least 50 % binding capability retained indicates an improved alkaline stability. It will be understood that each cycle involves target binding followed by a cleaning step. A cleaning step may be for example approximately 5-15 minutes incubation in contact with 0.1-0.5 M NaOH, such as for example approximately 5 minutes or 10 minutes or 15 minutes. For example, the time of incubation in contact with 0.5 M NaOH may be 5±0.5 minutes, 6±0.5 minutes, 7±0.5 minutes, or 8±0.5 minutes, 9±0.5 minutes, 10±0.5 minutes, 11±0.5 minutes, 12±0.5 minutes, 13±0.5 minutes, 14±0.5 minutes or 15±0.5 minutes. Said incubation may be for example at 22 ± 2 °C.

In some embodiments, the present fusion protein may retain a relatively high target binding capacity after repeated cleaning cycles involving 600 s exposure to NaOH of at least 0.01 M, such as 0.1 M, 0.3 M or 0.5 M. For example, compared to the target binding capacity after the first cycle of NaOH exposure (i.e., excluding the binding capacity recorded for the first cycle from the comparison), a fusion protein may retain at least 50 % of the target binding capability after at least 10 repeated cycles of binding and cleaning NaOH, such as at least 0.1 M NaOH or at least 0.3 M NaOH. In embodiments, the fusion protein may retain at least 50 % binding capability after at least 15 cycles, such as 20 cycles.

The second polypeptide moiety is an α-helix-containing protein domain, and may contain at least one α-helix, such as at least 2 α-helices, such as 3, 4, 5 or 6 α-helices, such as up 10 α-helices. In particular, the second polypeptide moiety may be an α-helix bundle domain, such as a three-helix bundle domain. In an α-helix bundle domain, the α-helices are spatially grouped together and form the major part of the protein domain, which may lack any other prominent unstructured or beta-structured regions. Examples of α-helix bundle domains include SpA protein domains and albumin binding domain (ABD). Hence, the second polypeptide moiety of the present disclosure may comprise a sequence derived from SpA domain A (SEQ ID NO:1), domain B (SEQ ID NO:2), domain C (SEQ ID NO:3), domain D (SEQ ID NO:4) or domain E (SEQ ID NO:5). In this context, "an amino acid sequence derived from an SpA protein domain" means an amino acid sequence which has at least 70 % identity to any sequence selected from the group consisting of: amino acid residues 9-28 in any one of SEQ ID NOs:1-3, residues 12-31 in SEQ ID NO:4, residues 7-26 in SEQ ID NO:5, residues 29-51 in any one of SEQ ID NOs:1-3, residues 30-54 in SEQ ID NO:4 and residues 27-49 in SEQ ID NO:5. Polypeptides derived from SpA protein domains include SEQ ID NOs: 6-110. In particular, the second polypeptide may comprise an α-helix bundle domain having an amino acid sequence selected from the group consisting of SEQ ID NOs: 1-110 and derivatives thereof having at least 70 % identity to any one of said sequences, and such as at least 80 %, at least 85 %, at least 90 % or at least 95 % identity to any one of said sequences and in particular to SEQ ID NO: 8 or 64. In embodiments, the second polypeptide moiety may comprise an α-helix bundle domain having an amino acid sequence having at least 85 % identity to amino acid residues 9-28 of SEQ ID NO:8 or to amino acid 29-51 of SEQ ID NO:8.

The second polypeptide moiety may be an alkali stable protein. For example, the second polypeptide moiety may comprise an amino acid sequence that is derived from a natural protein but modified in relation thereto. The modification may provide increased alkaline stability, reduced interaction with a natural binding partner, or both. For example, the second polypeptide moiety may comprise an SpA derived protein domain, which has optionally been modified to improve its alkaline stability. Preferably, the second polypeptide moiety may in itself have an alkali stability, measured as the number of alkali cleaning cycles with at least 50 % retained target binding capacity (or ability to be bound by an affinity ligand as described above for non-binding second polypeptide moieties), represented by at least 10 cycles, and preferably at least 20 cycles, such as at least 30 cycles, 40 cycles or 50 cycles.

Any modifications made in relation to a natural amino acids sequence of an α-helix-containing protein domain, such as an SpA domain, should not disrupt the helical structure in question. The amino acid variations suggested herein for SpA derived polypeptides take into account that certain amino acids are prone to formation of α-helix structures, and hence may be expected to preserve such a structure.

The first polypeptide moiety and the second polypeptide moiety are different from one another. Hence, in embodiments where the first polypeptide moiety comprises an α-helix-containing protein domain, such as an α-helix bundle domain, the amino acid sequences of the α-helix containing domains are not identical. In such embodiments, the second polypeptide moiety has higher alkaline stability than the first polypeptide moiety.

Examples of SpA derived, alkaline stable polypeptides particularly useful as the stabilizing polypeptide of the present invention include the amino acid sequences of SEQ ID NO:7-9, 28-30 and 61-110. Thus, the second polypeptide moiety may comprise an amino acid sequence selected from the group consisting of SEQ ID NOs: 7-9, 28-30 and 61-110 and derivatives thereof having at least 80 % identity to any one of said sequences, preferably at least 85, 90 or 95 % identity to any one of said sequences, such as to SEQ ID NO: 8 or 64. In embodiments, the second polypeptide may comprise an alkaline stable α-helix bundle domain having an amino acid sequence having at least 85 % identity to amino acid residues 9-28 of SEQ ID NO:8 or to amino acid 29-51 of SEQ ID NO:8.

To avoid interfering with the affinity capture function of the first polypeptide moiety, the second polypeptide moiety should not have binding affinity for the target entity intended to bind to the first polypeptide moiety. Hence, in embodiments the second polypeptide is not capable of binding to said target entity.

Furthermore, in order to avoid unwanted interactions, such as formation of dimers or aggregates, between the first polypeptide moiety and the second polypeptide moiety, the first polypeptide moiety preferably does not have a binding affinity for the second polypeptide moiety, and the second polypeptide moiety does not have affinity for the first polypeptide moiety. Furthermore, in cases where the second polypeptide is not intended to connect the fusion protein to another moiety (e.g. during purification or for coupling the fusion protein to a surface), it may be preferred that it does not have a binding affinity for another molecule, or at least not for any polypeptide present in a process of producing a protein of interest, in order to avoid competing interactions which might adversely affect the interaction between the first polypeptide moiety and its target antigen.

For example, it may be advantageous if the second polypeptide moiety does not have a binding affinity for immunoglobulins. Therefore, where the second polypeptide is derived from a protein domain of SpA which naturally binds to the Fc and/or a VH portion of immunoglobulins, the amino acid sequence of the second polypeptide may be such as to abolish binding to Fc or VH3 or both. In embodiments, the second polypeptide moiety may thus be incapable of binding to either Fc or VH3, or both, of immunoglobulins.

However, it is contemplated that binding to IgG could be desirable in certain applications, and in such cases the second polypeptide may be capable of binding to any desired portion of IgG, in particular the Fc and/or VH3 portions. For example, the second polypeptide may bind to the Fc portion, but not the VH3 of IgG. Alternatively, the second polypeptide may bind to VH3, but not the Fc portion of IgG.

The second polypeptide moiety may be derived from a protein domain of SpA as described above, and modified to reduce or abolish binding to VH3 by introducing mutations in positions X₃₃, X₄₀ and X₅₁, as described in WO 2023/046886. The designations X₃₃, X₄₀ and X₅₁ refer to positions 33, 40 and 51 in an SpA domain represented by any one of SEQ ID NOs: 1-3. Certain mutations in positions X₃₃, X₄₀ and X₅₁ of these polypeptides significantly reduce or abolish binding affinity for VH3. In such polypeptides, X₃₃ is selected from T, S, G, Q, A, E, H, R, P, D, K and N; X₄₀ is selected from E, G, R, D, K, Q, N, H and S, and X₅₁ is selected from L, V, S, I, R and G; with the proviso that when X₅₁ is L then X₃₃X₄₀ is selected from AD, HK, EG, ER, GR, AK, AR, PK, RR and KK and when X₅₁ is G then X₃₃X₄₀ is TK.

Reduced VH affinity can be defined as the second polypeptide moiety having binding affinity for an Fc region of an immunoglobulin, and having lower binding affinity for a VH3 region of trastuzumab compared to the binding affinity of SEQ ID NO: 6 (corresponding to SEQ ID NO:59 of WO2023/046886) for the same VH3 region. WO2023/046886 describes polypeptides having reduced VH affinity and is incorporated herein in its entirety. Trastuzumab is a monoclonal antibody also known under trade names Herceptin^{™} (Roche) and Trazimera^{™} (Pfizer).

The second polypeptide moiety of the present fusion protein may have X₃₃ selected from T, S, G, Q, A, E, H and R; X₄₀ selected from E, G, R, D, K, Q, H and S, and X₅₁ selected from L, V, S, I, R and G; with the proviso that when X₅₁ is L then X₃₃X₄₀ is selected from AD, HK, EG, ER, GR, AK, AR and RR and when X₅₁ is G then X₃₃X₄₀ is TK.

Alternatively or additionally, the second polypeptide moiety may be derived from a protein domain of SpA (as described above) and modified to reduce at least a significant part of the affinity for Fc while retaining the affinity for the VH3 region. This can be done e.g. by providing selected amino acids in positions 9-11, 13-14, 17-18, 26, 28 and 29 of an SpA domain, such as represented by any one of SEQ ID NOs: 1-3. International application no. PCT/EP23/056401 describes polypeptides having reduced Fc affinity and is incorporated herein in its entirety. Reduced Fc affinity can be defined as the second polypeptide having binding affinity for an VH3 region of an immunoglobulin, and having lower binding affinity for a Fc region of trastuzumab compared to the binding affinity of SEQ ID NO: 8 for the same Fc region.

The second polypeptide moiety may comprise an amino acid sequence derived from an SpA protein domain, which amino acid sequence has reduced or abolished binding to both VH3 and Fc. In such variants, the mutations described above for reducing VH3 affinity and Fc affinity, respectively, may be combined.

For example, the second polypeptide moiety may comprise, or may be, an amino acid sequence derived from a protein domain of SpA in which
the amino acid residue in position 9 of SEQ ID NO:64 is selected from L, E, R, A and Y, such as is A;
the amino acid residue in position 10 of SEQ ID NO:64 is selected from L, E, R, A and Y, such as is Y
the amino acid residue in position 11 of SEQ ID NO:64 is selected from L, E, R, A and Y, such as is R;
the amino acid residue in position 13 of SEQ ID NO:64 is selected from L, E, R, A and Q, such as is L, R, A or Q;
the amino acid residue in position 14 of SEQ ID NO:64 is selected from L, E, R, A, Q and K, such as is A or R;
the amino acid residue in position 17 of SEQ ID NO:64 is selected from L, E, R and A, such as is A;
the amino acid residue in position 18 of SEQ ID NO:64 is selected from L, E, R and A, such as is R;
the amino acid residue in position 28 of SEQ ID NO:64 is selected from L, E, R and A, such as is R or A;
the amino acid residue in position 29 of SEQ ID NO:64 is selected from L, E, R and A, such as is R or A;
the amino acid residue in position 33 of SEQ ID NO:64 is selected from T, S, G, Q, A, E, H and R, such as is S;
the amino acid residue in position 40 of SEQ ID NO:64 is selected from E, G, R, D, K, Q, H and S, such as is G; and
the amino acid residue in position 51 of SEQ ID NO:64 is selected from L, V, S, I, R and G, such as is V.

For example, the second polypeptide moiety comprises an amino acid sequence according to
VDAKFDKEX₉X₁₀X₁₁AX₁₃X₁₄EIX₁₇X₁₈LPNLTEEQRX₂₈X₂₉FIQX₃₃LKDDPSX₄₀SKAILAEAKKX₅₁NDAQAPK (SEQ ID NO: 235)
in which, independently, X₉, X₁₀, X₁₁, X₁₃, X₁₄, X₁₇, X₁₈, X₂₈, X₂₉, X₃₃, X₄₀ and X₅₁ are selected as described herein.

For clarity, in said variants, the expression "the amino acid residue in position X" refers to the position in the second polypeptide moiety corresponding to position X in SEQ ID NO: 64.

The amino acid residues suggested above for positions 9-11, 13-14, 17-18, 28-29, 33, 40 and 51 are expected to preserve the helical structure of the α-helix-containing polypeptide domain, and have been found to provide functional variants of a stabilizing polypeptide.

In some variants the amino acids in positions 9-11 are AYR. In other variants, positions 9-11 are independently selected from L, E, R, A and Y, and positions 28, 29, 33, 40 and 41 are varied in relation to SEQ ID NO: 64, i.e. are not A, A, S, G, and V, respectively. In some variants, positions 13-14 are selected from LA, RA, AR and QA.

For example, the second polypeptide moiety may comprise an amino acid sequence selected from the group consisting of SEQ ID NOs:64-110.

In some embodiments, the second polypeptide moiety may comprise an amino acid sequence based on a protein domain of SpA as defined above, wherein the amino acid in the position corresponding to position 31 of any one of SEQ ID NOs:8-10 is isoleucine.

In other embodiments, the second polypeptide moiety may comprise an amino acid sequence based on a protein domain of SpA as defined above, wherein the amino acid in the position corresponding to position 8 of any one of SEQ ID NO:8-10 is not isoleucine, or is not an amino acid selected from the group consisting of Ile, Leu, Val, Tyr, Phe and Trp. In such embodiments the first polypeptide moiety may be capable of binding IgG.

In embodiments, neither the second polypeptide moiety, nor the first polypeptide moiety have affinity for trastuzumab.

In particular, the second polypeptide moiety may consist of an SpA-derived polypeptide as herein and optionally additional amino acids, such as a short N-terminal amino acid sequence and/or optionally a short C-terminal amino acid sequence.

More generally, the fusion protein of the present disclosure may comprise additional amino acids as mentioned above e.g. in relation to Figures 1 and 2, at the N-terminus of the fusion protein, at the C-terminus of the fusion protein, and/or between the different polypeptide units or moieties. The fusion protein as defined herein may comprise any suitable number of additional amino acid residues, for example one, two, three, four, five, six, seven, eight, nine, ten or more, such as up to 20, additional amino acid residues, optionally in a consecutive sequence. For example, a sequence of additional amino acids may consist of from 1 to 12 amino acids, and there may be several such sequences in the fusion protein. The additional amino acids may be a legacy from recombinant protein expression, a leader peptide, a signal peptide, a purification tag, a peptide intended for coupling to support, or the like. The additional amino acid may be a spacer or linker as defined elsewhere herein, or have the ability to serve as spacer or linker. The additional amino acid residues may individually or collectively improve production, purification, stabilization *in vitro* or coupling of the polypeptide to substrates of interest, for example to a solid support, such as a solid support described in connection to the aspect of an adsorbent material or separation matrix.

The fusion protein or a multimer as defined herein may comprise at least one linker. For example, a linker is present between each monomer within the multimer, each linker comprising up to 15 or 30 amino acids, such as 1-5, 1-10 or 5-10 amino acids. Where there are multiple linkers present, the linkers may be the same or different. The skilled person is aware of different kinds of linkers with different properties, such as flexible peptide linkers, rigid peptide linkers and cleavable peptide linkers. Peptide linkers may be structured or unstructured. Typically, unstructured linkers are more flexible and less rigid than structured linkers. Linkers may be used in order to for example increase stability or improve folding of fusion proteins. A linker may also ensure a certain spatial distance between the polypeptide moieties that it links, which may be beneficial for the access of an antigen to the binding surface of an antigen-binding polypeptide. The presence of a linker within a fusion protein generally does not substantially affect the target binding capacity of a correctly folded antigen-binding polypeptide moiety thereof. Thus, the fusion protein disclosed herein, or moieties thereof, may be linked to each directly by peptide bonds between the C-terminal and N-terminal ends of the polypeptides.

The nature of a linker should preferably not destabilize the spatial conformation of the protein units, that is of the monomers within the multimer. This can e.g. be achieved by avoiding the presence of proline in the linkers. Furthermore, said linkers should preferably also be sufficiently stable in alkaline environments not to impair the properties of the protein units. For this purpose, it is advantageous if the linkers do not contain asparagine. It can additionally be advantageous if the linker does not contain glutamine.

In some embodiments, the fusion protein, as disclosed above, comprises one or more coupling elements, selected from the group consisting of at least one cysteine residue, a plurality of lysine residues and a plurality of histidine residues, and combinations thereof. Such coupling elements may be provided anywhere in the fusion protein, but may optionally be arranged close to or at the C-terminal or N-terminal end of the polypeptide. A coupling element may e.g. be a single cysteine at the C-terminal end of the fusion protein. The coupling element(s) may be directly linked to the C- or N-terminal end, or may be linked via a linker comprising up to 15 amino acids, such as 1-5, 1-10 or 5-10 amino acids. Such an amino acid sequence should preferably also be sufficiently stable in alkaline environments not to impair the properties of the fusion protein. For this purpose, it is advantageous if the sequence does not contain asparagine. It can additionally be advantageous if the sequence does not contain glutamine. An advantage of having a C-terminal cysteine is that endpoint coupling of the protein can be achieved through reaction of the cysteine thiol with an electrophilic group on a support. This provides excellent mobility of the coupled protein.

The fusion protein of the present invention can be produced by conventional biotechnological methods, by recombinant protein production using genetically engineered host cells.

Thus, in further aspects, the present disclosure provides a polynucleotide encoding fusion protein as described herein; an expression vector comprising said polynucleotide; and a host cell comprising said expression vector.

Also encompassed by this disclosure is a method of producing the fusion protein as described herein, comprising culturing said host cell under conditions allowing expression of said fusion protein from its expression vector, and isolating the fusion protein.

The host cells may be prokaryotic cells, such as bacteria, or eukaryotic cells. Exemplary prokaryotic host cells are *Escherichia coli.* Suitable eukaryotic cells may be yeast cells, such as *Saccharomyces cerevisiae* and *Pichia pastoris,* or animal cells, such as insect cells or mammalian cells commonly used in the field of biotechnology for the production of proteins, e.g. Chinese Hamster Ovary (CHO) cells.

Following isolation of the fusion protein, the fusion protein is purified, e.g. by conventional means known in the art. For example, where the fusion protein comprises a histidine tag, a step of purifying the fusion protein may comprise immobilized metal affinity chromatography (IMAC). Other methods of purification that may be used include affinity chromatography using a ligand binding either the first polypeptide moiety or the second polypeptide moiety. For example, an affinity ligand that binds to a framework region of the first polypeptide may be used, or the affinity chromatography may use an affinity ligand based on a domain of SpA, wherein the affinity ligand binds to a VH region, such as VH3, of the single-chain polypeptide of the first polypeptide moiety. Alternatively, the affinity ligand may bind to the second polypeptide moiety of the fusion protein, such as to an affinity tag included in the amino acid sequence of the second polypeptide moiety.

The fusion protein as disclosed herein may alternatively be produced by *in vitro* translation, or by non-biological peptide synthesis using amino acids and/or amino acid derivatives having protected reactive side-chains. Non-biological peptide synthesis may comprise:
- step-wise coupling of the amino acids and/or the amino acid derivatives to form a polypeptide having protected reactive side-chains,
- removal of the protecting groups from the reactive side-chains of the polypeptide, and
- folding of the polypeptide in aqueous solution.

In general, the present fusion protein may be used for capture of the target entity for which the first polypeptide moiety has an affinity. However, preferably, the fusion protein as such is not intended for use as a therapeutic compound, and is preferably not intended for use *in vivo.* Hence, the present fusion protein may be used for *in vitro* capture of the target entity.

The capture may be for the detection of the target entity within a sample, or for separation of the target entity from other components in a sample. The fusion protein may be used for analytical separation of the target entity, or it may be used for preparative purification of the target entity. The term "preparative" refers to the process of preparing a purified target entity, in particular during the manufacture of a product, such as a pharmaceutical product, which comprises the target entity. Separation, detection and/or quantification using the fusion protein of the present disclosure may alternatively be performed in the context of a sensor application.

In particular, the fusion protein of the present invention may be used in applications involving interaction between the first polypeptide moiety and its target entity, where at least one of the fusion protein and the target entity is coupled to a support. When the fusion protein is coupled or immobilized to a support, the first polypeptide moiety is still capable of binding its target entity.

Where the fusion protein is coupled to a support, this may be referred to as an adsorbent material or an affinity capture material. Adsorbent materials may be used in various industrial or laboratory applications, including separation, purification, detection and/or quantification of the target entity. A separation matrix as described herein is an adsorbent material intended for use in separation of a target entity from other components. The coupling of the fusion protein to the support may be provided via the stabilizing polypeptide. For example, where the second polypeptide moiety is located C-terminally of the first polypeptide moiety, the second polypeptide moiety may have a C-terminal amino acid sequence suitable for coupling to a support. For example, a tag or spacer ending with a cysteine (C) may be provided for coupling via a thioether bond.

Where the fusion protein is not coupled to a support, it may be used e.g. in a detection assay where the target or analyte is present on a surface and is contacted with the fusion protein which is present in a solution. In such a case, it is envisaged that a reporter entity capable of binding to the second polypeptide moiety may be used for generating a detectable signal. The reporter entity may be e.g. a fluorescent labelled or radiolabelled entity as known in the art. If the second polypeptide moiety is capable of binding to IgG, the reporter entity may be based on IgG.

Because of the stabilizing effect of the second polypeptide moiety, the fusion protein is especially useful in applications where it may be exposed to alkaline conditions, such as treatment with NaOH. Such treatments are frequently used in the field of chromatography, to strip a chromatography matrix of bound impurities before the next purification cycle (cleaning-in-place, as described above). However, alkali exposure or cleaning of a binding surface may be used also in other contexts.

The support may be a solid support and may optionally be a porous material.

The support may be or comprise a surface onto which the fusion protein is coupled. Examples of such support materials include conventional protein-binding support and surfaces such as a chip, a plate, a well and a sheet. The support may optionally be provided in other forms such as a fiber, a membrane, a fibrous matrix, a filter, a porous monolith, a particle or a bead, such as a gel bead as used in chromatography resins. Particles or beads can be porous or non-porous. Particles or beads may include magnetic beads. Supports in the form of beads or particles can be used as a packed bed or in a suspended form. Suspended forms include those known as expanded beds and pure suspensions, in which the particles or beads are free to move. In case of monoliths, packed bed and expanded beds, a separation procedure commonly follows conventional chromatography with a concentration gradient. In case of pure suspension, batch-wise mode will be used.

The support may be made of any suitable material, as described in more detail below. As a non-limiting example, a conventional affinity separation matrix is often of organic nature and based on polymers that expose a hydrophilic surface to the aqueous media used, i.e. expose hydroxy (-OH), carboxy (-COOH), carboxamido (-CONH₂, possibly in N- substituted forms), amino (-NH₂, possibly in substituted form), oligo- or polyethylenoxy groups on their external and, if present, also on internal surfaces.

The fusion protein may be attached to the support via known coupling techniques utilizing e.g. thiol, amino and/or carboxy groups present in the fusion protein. Bisepoxides, epichlorohydrin, CNBr, N-hydroxysuccinimide (NHS) etc. are well-known coupling reagents. Between the support and the fusion protein, a spacer molecule can be introduced, which improves the availability of the first polypeptide moiety and/or facilitates the chemical coupling of the fusion protein to the support. Depending on the nature of the fusion protein and the coupling conditions, the coupling may be a random or multipoint coupling (e.g. via a plurality of lysines or histidines) or a single point coupling (e.g. via a single cysteine). Alternatively, the fusion protein may be attached to the support by noncovalent bonding, such as physical adsorption or biospecific adsorption.

The fusion protein may be coupled to the support via thioether bonds. Methods for performing such coupling are well-known in this field and easily performed by the skilled person in this field using standard techniques and equipment. Thioether bonds are flexible and stable and generally suited for use in affinity chromatography. In particular when the thioether bond is via a terminal or near-terminal cysteine residue on the fusion protein, the mobility of the coupled fusion protein is enhanced which provides improved binding capacity and binding kinetics. In some embodiments the fusion protein is coupled via a C-terminal cysteine provided on the protein as described above. This allows for efficient coupling of the cysteine thiol to electrophilic groups, e.g. epoxide groups, halohydrin groups etc. on a support, resulting in a thioether bridge coupling.

With regard to specific materials, the support may comprise a polymeric material. Polymeric materials include materials of natural or synthetic polymers, and combinations thereof. For example, the support may comprise a polyhydroxy polymer, such as a polysaccharide. Examples of polysaccharides include e.g. dextran, starch, cellulose, pullulan, agar, agarose etc, including derivatives thereof. Polysaccharides are inherently hydrophilic with low degrees of nonspecific interactions, they provide a high content of reactive (activatable) hydroxyl groups and they are generally stable towards alkaline cleaning solutions used in bioprocessing. The support may comprise agar or agarose, such as crosslinked agarose. Such supports used in the present invention can easily be prepared according to standard methods, such as inverse suspension gelation (S Hjerten: Biochim Biophys Acta 79(2), 393-398 (1964)). Alternatively, the base matrices are commercially available products, such as crosslinked agarose beads sold under the name of SEPHAROSE^{™} FF (Cytiva^{™}). In an embodiment, which is especially advantageous for large-scale separations, the support has been adapted to increase its rigidity using the methods described in US6602990 or US7396467, which are hereby incorporated by reference in their entirety, and hence renders the matrix more suitable for high flow rates.

Synthetic polymers useful as material for the support include polyvinyl alcohol, polyhydroxyalkyl acrylates, polyhydroxyalkyl methacrylates, polyacrylamides, polymethacrylamides etc. In case of hydrophobic polymers, such as matrices based on divinyl and monovinyl-substituted benzenes, the surface of the matrix can be hydrophilised to expose hydrophilic groups as defined above to a surrounding aqueous liquid. Such polymers are easily produced according to standard methods. As an alternative, a commercially available product, such as SOURCE^{™} (Cytiva^{™}) may be used.

Alternatively, the solid support according to the invention comprises a support of inorganic nature, e.g. silica, zirconium oxide etc.

In embodiments, the fusion protein may be coupled to a support which is a convection-based chromatography matrix. Such convection-based chromatography matrix may comprise a porous polymer membrane, a filter, a fibrous matrix, or a porous monolith. Examples of a porous polymer membrane include Mustang^{™} membranes (Cytiva) and Sartobind^{™} membranes (Sartorius). A fibrous support may be based on electrospun polymeric fibers or cellulose fibers, optionally non-woven fibers. A fibrous matrix may thus be a fibrous non-woven matrix. The fibers may have a cross-sectional diameter of 10-1000 nm, such as 200-800 nm, 200-400 nm or 300-400 nm. Such a fibrous support can be found in a HiTrap Fibro^{™} unit (Cytiva^{™}). Alternative fibrous supports are disclosed in e.g. WO2019/137869 and WO2018/011600.

An adsorbent material or separation matrix as described herein may be used for the same purposes as mentioned above for the fusion protein.

Thus in one aspect, the invention provides a chromatography material comprising an adsorbent material or separation matrix as disclosed herein, as well as chromatography columns or devices incorporating such adsorbent material or separation matrix.

In another aspect, the adsorbent material may be a sensor surface intended for use in a sensor or other detection or quantification devices.

The present invention provides a method of separating or isolating a target entity, wherein an adsorbent material or separation matrix as disclosed herein is used. In some embodiments, the method comprises contacting a liquid sample comprising the target entity with an adsorbent material as disclosed herein. The contacting is performed under conditions under which the target entity can bind to the first polypeptide moiety. The method may furthermore comprise washing said adsorbent material with a washing liquid, eluting the target entity from the adsorbent material with an elution liquid, and optionally cleaning the adsorbent material with a cleaning liquid. The cleaning liquid can alternatively be called a cleaning-in-place (CIP) liquid. The cleaning liquid typically is an alkaline solution, such as comprising NaOH or KOH of at least 0.05 M, such as 0.05-1 M, such as 0.05-0.5 M, such as at least 0.1 M, such as 0.1-0.5 M, e.g. 0.3 M or 0.5 M. The contact (incubation) time may be at least 10 min. The binding, eluting and cleaning steps may advantageously be repeated at least 10 times, such as at least 20 times.

A skilled person will understand that the liquid sample to be purified may be any sample comprising the target entity in an environment from which it is to be purified or separated. The sample may be obtained from a cell culture, such as a clarified cell culture harvest, and may have been subjected to one or more conventional steps of filtration, concentration, dilution and/or buffer exchange, and optionally one of more initial chromatography steps, in particular a step which is not based on affinity chromatography, such as ion exchange chromatography, hydrophobic interaction chromatography, multimodal chromatography, or size exclusion chromatography. For example, the sample may be a clarified and filtered cell culture harvest. The sample may have been subjected to one or more steps of filtration by tangential flow filtration (TFF).

Prior to contacting with the present adsorbent material, the liquid sample contains the target entity and at least one impurity, such as host cell protein (HCP) or host cell nucleic acids. The present adsorbent material is useful for separating the target entity from such impurities, and after performing the above process, the eluate containing the target entity has a reduced level of at least one such impurity.

Optionally, after performing a separation based on affinity capture as described herein, the eluate containing purified target entity may be subjected to one or more steps of filtration, concentration, dilution or buffer exchange, or chromatography step(s) not based on affinity chromatography, such as ion exchange chromatography, hydrophobic interaction chromatography, multimodal chromatography, or size exclusion chromatography. A further chromatography step performed after an affinity separation step of the present disclosure may be referred to as a polishing step.

While the invention is described herein with respect to exemplary embodiments, the skilled person will appreciate that the invention is not limited to these. In the claims, any reference signs placed between parentheses shall not be construed as limiting the claim.

Use of the verb "to comprise" and its conjugations does not exclude the presence of elements or steps other than those stated. For example, a polypeptide "comprising" framework regions FWR1, FWR2 and FWR3 can also have further framework regions, such as an FWR4, and may have other regions, such as CDRs, and optionally other structures or sequences. The article "a" or "an" preceding an element does not exclude the presence of a plurality of such elements. The mere fact that certain measures are recited in mutually different dependent claims does not indicate that a combination of these measures cannot be used to advantage.

### EXAMPLES

### Example 1A: Preparation of glycerol stock solutions from IDT oligo

This example describes the generation of glycerol stock solution for the production of candidate polypeptides used in Example 4B.

Materials and equipment used were as follows: vector plasmid with kanamycin resistance; G-block polypeptide candidates (IDT^{™}); restriction enzymes Kpnl-HF and Hindlll-HF (NEB^{™}); Antarcic phosphatase (NEB^{™}); GFX^{™} PCR DNA and Gel Band Purification Kit (Cytiva^{™}); T4 DNA ligase (New England Biolabs); Top10 *E. coli* cells (ThermoFisher^{™}); chemically competent BL21(DE3) *E. coli* cells (prepared in-house); chemically competent K12-017 *E. coli* cells (prepared in-house); SOC-media (Invitrogen^{™}); stock of kanamycin 50 mg/ml (PanReac AppliChem^{™}); KCM buffer (5X) 0.5M KCI, 0.15M CaCl₂, 0.25M MgCl₂; Luria-Bertani (LB) culture medium (Invitrogen^{™}); agar plate supplemented with kanamycin (50 µg/ml); 14 ml Falcon^{™} Round-Bottom tube (Corning^{™}); PlasmidPrep Mini Spin Kit (Cytiva ^{™}); Infors HT Shaking incubator.

The vector plasmid and G-blocks containing the DNA sequences for all candidates were digested with restriction enzymes Kpnl-HF and Hindlll-HF. The plasmid was dephosphorylated using Antarctic phosphatase and gel purified according to manufacturer's protocols.

Restriction enzyme digested G-blocks were ligated into the gel purified plasmid using T4 DNA ligase for 2h at room temperature and transformed into Top10 *E. coli* cells. Briefly, cells and ligation mix were incubated for 20 min on ice and 10 min at room temperature in KCM-buffer. Cells were then transferred to SOC media and incubated at 37°C for 1h and then seeded on agar plates containing kanamycin 50 µl/ml and incubated at 37°C over night. A single colony was used to inoculate 4 mL LB media supplemented with 50 µg/ml kanamycin in a 14 ml round-bottom tube and grown over night at 37°C, 200 rpm. The plasmid was prepared according to manufacturer's protocols and the purified plasmid was transformed into either KCM competent K12-017 *E. coli* or KCM competent BL21(DE3) *E. coli.* Cells and plasmid were incubated for 20 min on ice and 10 min at room temperature in KCM-buffer. Cells were then transferred to SOC media and incubated at 37°C for 1h and then seeded on agar plates containing kanamycin 50 µl/ml and incubated at 37°C over night. A single colony was used to inoculate 4 mL LB media supplemented with 50 µg/ml kanamycin in a 14 ml round-bottom tube and grown over night at 37°C, 200 rpm. A glycerol stock of each variant was prepared with 900 µl overnight culture and 500 µl 50% glycerol and stored at -80°C.

### Example 1B: Preparation of glycerol stock solutions from IDT plasmids

This example describes the generation of glycerol stock solution for the production of candidate polypeptides used in Examples 2A, 2B, 4A and 5-7.

Materials and equipment used were as follows: plasmid DNA for the candidate polypeptides to be produced; chemically competent BL21(DE3) *E. coli* (prepared in-house), KCM buffer (5X) 0.5M KCI, 0.15M CaCl₂, 0.25M MgCl₂, LB culture medium (Invitrogen^{™}), carbenicillin (100 mg/ml), agar plates (BD BACTO^{™} Agar) supplemented with carbenicillin (100 µg/ml) (PanReac AppliChem^{™}), 14 ml Falcon^{™} Round-Bottom (Corning^{™}), Infors HT Shaking incubator.

Desired amino acid sequences of candidate polypeptides were back-translated to DNA and optimized to remove rare codons. Constructs were ordered as plasmid DNA based on an expression vector with T5 promotor, OmpA signal peptide, ampicillin resistance and pUC origin of replication. Plasmid DNA was used for transformation into chemically competent BL21(DE3) *E. coli,* plated on agar plate supplemented with 100 µg/ml carbenicillin and grown over night at 37°C. For each candidate, a single colony was used to inoculate 4 mL LB media supplemented with 100 µg/ml carbenicillin in a 14 ml round-bottom tube and grown over night at 37°C, 200 rpm agitation. A glycerol stock of each variant was prepared with 900 µl overnight culture and 500 µl glycerol 50% and stored at -80°C until further use.

### Example 2A: AAV9 binding polypeptide with Z fusion - monomeric constructs

A single-domain antibody capable of binding AAV9 was fused to a stabilizing polypeptide and the impact on protein expression, antigen binding capacity and alkaline stability was investigated.

The polypeptides tested are identified in Table 3. A single-domain antibody was compared to a fusion of the same single-domain antibody with a stabilizing polypeptide. The single domain antibody (denoted "sdAb") was an AAV9-binding VHH variant (SEQ ID NO: 139). The stabilizing polypeptide (denoted "Z") was an SpA domain Z variant (SEQ ID NO:64) in which binding to the Fc and VH3 portions of IgG had been abolished. Each construct had a C-terminal linker followed by a (His)₆ tag.

**Table 3.**

| Candidate no. | Construct description | Construct sequence |
|---|---|---|
| 2A-1 | [sdAb] | SEQ ID NO:140 |
| 2A-2 | [sdAb]-[Z] | SEQ ID NO:142 |

In particular, the following aspects were evaluated: (1) Affinity assessment of AAV9 interaction, tested through high concentration injection of AAV9. (2) Alkaline stability, tested by reduction of AAV9 binding after increasing number of treatments with 0.5 M NaOH.

### Materials and methods

### Generation of MabSelect PrismA^{™} purified polypeptides

5 µl glycerol stock produced for the polypeptides 2A-1 and 2A-2 as described in Example 1B was inoculated in 4 ml LB culture medium supplemented with 100 µg/ml carbenicillin in 14 ml round-bottom tube and incubated at 37°C over night at 160 rpm.

Protein expression culture medium prepared from Terrific Broth (TB) culture medium supplemented with 100 µg/ml carbenicillin and 2 mM MgCl₂ was added to filled 500 ml baffled glass shake flasks (50 ml per flask). Each flask was inoculated with approximately 500 µl from the previous overnight culture to obtain a starting OD600 of 0.05. The flasks were incubated in an Infors HT shaking incubator for approximately 3.5 hours at 37°C and 140 rpm shaking until OD600 reached 1.0. Thereafter 50 µl of IPTG (1 M) was added to a final concentration of 1 mM and the flasks were incubated in Infors HT shaking incubator at 27°C and 140 rpm shaking for 18 hours, whereafter the cultures were transferred to Falcon tubes (50 ml). To generate crude variant lysates the Falcon tubes were incubated in 48°C water bath for 2 hours followed by pelleting of cell debris by centrifugation at 8000 g for 10 min and filtration of supernatant with a 0.22 µm filter.

Clarified lysate for each sample was loaded onto a 1 ml HiTrap MabSelect^{™} PrismA chromatography column (Cytiva^{™}), equilibrated in phosphate buffered saline (PBS) pH 7.4, allowing residence time of about 2.4 min. The column was then washed with 5 column volumes (CV) of PBS pH 7.4, followed by 5 CV of 50 mM sodium acetate pH 6. Protein was eluted using 50 mM sodium acetate pH 3.5 in a step gradient over 5 CV.

Concentration of the purified samples was measured using NanoDrop (ThermoFisher Scientific) according to manufacturer's instructions. Following purification, samples were kept in refrigerator in Eppendorf tubes until all measurements had been performed and were then kept in freezer until Biacore analysis. Protein mass was determined by liquid chromatography-mass spectrometry on a ACQUITY RDa Detector (Waters^{™}) using a BioRessolve column (Waters^{™}).

### Biacore analysis of binding affinity for AAV9

To assess binding to AAV9, the polypeptides 2A-1 and 2A-2 as described above were immobilized on a Biacore^{™} Series S CM5 chip using Amine coupling kit (Cytiva^{™}) and analyzed using Biacore^{™} 8K+ instrument (Cytiva^{™}). A sample containing AAV9 viral particles (vp) at a concentration of 2E12 vp/ml was used as analyte.

Immobilization was performed using a standard method in Biacore software with coupling of the polypeptides in Flow Cell 2 (FC2) and activation/inactivation in Flow cell 1 (FC1). Polypeptides were diluted in Biacore^{™} Acetate buffer pH 5.0 (Cytiva^{™}) at a concentration of 17-30 µg/ml, depending on their molecular weight. The immobilization levels varied between different polypeptide variants corresponding to their molecular weight. In each run the polypeptide was immobilized in FC2.

Biacore^{™} method for binding analysis: Running buffer: PBS-P+; Flow rate: 5 µl/min; Sample injection: 2400 s/40 min over both FC1 and FC2; Dissociation time: 2400 s/40 min; Regeneration: 10 mM Glycine-HCl pH 1.5, 30 µl/min, 2x30 s. Injections of analyte (AAV9) for each channel (cycles) were as follows: running buffer, AAV9 2E11 vp/ml, 5E11 vp/ml, 2E12 vp/ml.

All sensorgrams were generated as reference subtracted and the responses as the difference between baseline before injection and signal just before end of injection.

### Biacore analysis of alkaline stability

After evaluation of AAV9 binding (see above) the same chips were subjected to repeated cycles of binding to AAV9 (5E11 vp/ml) followed by injection of NaOH (0.5 M) for the assessment of alkaline stability.

Biacore method for alkaline stability (per cycle): Running buffer: PBS-P+; Flow rate: 10 µl/min; Sample injection 1 (AAV9 5E11 vp/ml): 300 s over both flow cells ; Dissociation time 1: 30 s; Sample injection 2 (0.5 M NaOH): 600 s over both flow cells ; Dissociation time 2: 0 s ; Regeneration: 10 mM Glycine-HCl pH 1.5, 30 µl/min, 2x30 s. This cycle was repeated 40 times to follow stability of AAV9 response values.

All sensorgrams were generated as reference subtracted and the responses as the difference between baseline before injection and signal just before end of injection.

### Results

Analysis of expression level by Bis-Tris PAGE indicated that expression levels of polypeptide 2A-1 (sdAb, no fusion) and 2A-2 (sdAb with C-terminal stabilizing polypeptide) were comparable (data not shown). This sdAb had previously been found to express well in monomeric form.

Immobilization levels on CM5 chip were 2134 or 3560 RU, respectively. The AAV9 binding capacity at the maximum viral concentration used (2E12 vp/ml) was about 10 % higher for the tested fusion protein 2A-2 compared to the single sdAb 2A-1.

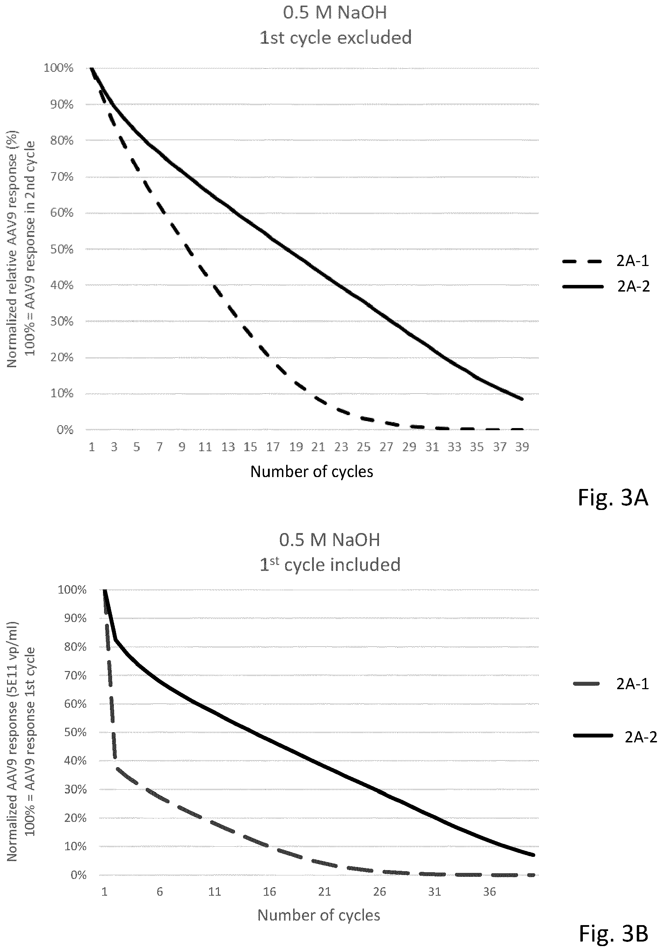

Fig. 3A plots the AAV9 binding capacity for increasing number of cycles involving 0.5 M NaOH exposure (sample injection 2 above) normalized to the response after completing the first cycle (i.e., excluding the first cycle). The first cleaning cycle may remove loosely bound polypeptides from the surface. As can be seen in this figure, the fusion protein 2A-2, comprising a single-domain antibody variant fused at its C-terminal to a stabilizing polypeptide, shows a drastic improvement in alkaline stability over the single-domain antibody 2A-1 alone. The improvement is even more pronounced if the response after each cycle is normalized to the response of the first cycle, before the first NaOH exposure, as shown in Fig. 3B. As can be seen in Fig. 3B, the drop in AAV9 binding capacity experienced between the first and second cycles is merely 20 % for fusion protein 2A-2, compared to the 60 % reduction in response for the sdAb which is not part of a fusion protein. It is hypothesized that the smaller drop between the first and second binding cycles may at least in part be due to the surface coupling of the fusion protein. Hence, it is believed that the second polypeptide moiety provides a coupling mechanism that is more resistant to alkali exposure than the coupling of the sdAb as such.

Table 4 summarizes the results of the AAV9 binding capacity and alkaline stability measurements.

**Table 4**

| Sample | AAV9 capacity (2E12 vp/ml) (RU) | No. of cycles with at least 50 % binding maintained (5E11 vp/ml, 100%=AAV9 response of 1^{st} cycle) | No. of cycles with at least 50 % binding maintained (5E11 vp/ml, 100%=AAV9 response of 2^{nd} cycle) |
|---|---|---|---|
| 2A-1 | 11294 | 1 | 10 |
| 2A-2 | 12421 | 14 | 19 |

### Example 2B: AAV9 binding polypeptide with Z fusion - multimeric constructs

Multimeric constructs of a single-domain antibody capable of binding AAV9 fused to a stabilizing polypeptide were investigated with respect to protein expression, antigen binding capacity and alkaline stability.

The polypeptides tested are outlined in Table 5 below. A single-domain antibody (sdAb) trimer was compared to trimeric fusion proteins containing one or three stabilizing polypeptides, respectively. The single domain antibody (denoted "sdAb") was an AAV9-binding VHH variant (SEQ ID NO:139). The stabilizing polypeptide (denoted "Z") was an SpA domain Z variant (SEQ ID NO:64) in which binding to the Fc and VH3 portions of IgG had been abolished. Each construct had a C-terminal linker followed by a (His)₆-Cys tag.

**Table 5**

| Candidate no. | Construct description | Construct sequence |
|---|---|---|
| 2B-1 | [sdAb]-[sdAb]-[sdAb] | SEQ ID NO:143 |
| 2B-2 | [sdAb]-[sdAb]-[sdAb]-[Z] | SEQ ID NO:144 |
| 2B-3 | [sdAb]-[Z]-[sdAb]-[Z]-[sdAb]-[Z] | SEQ ID NO:145 |

The following aspects were evaluated: (1) Protein expression analysis by PAGE. (2) Affinity assessment of AAV9 interaction (tested through high concentration injection of AAV9). (3) Alkaline stability (tested by reduction of AAV9 binding after increasing number of treatments with 0.3 M NaOH).

### Materials and methods

### Generation of MabSelect PrismA^{™} purified and biotinylated polypeptides

Candidate polypeptides were generated and purified according to the method described in Example 2A.

Concentration of MabSelect PrismA^{™} purified samples was measured using NanoDrop (Thermo Scientific) according to manufacturer's instructions. Following purification, samples were reduced by adding 10 µl 0.5 M DTT dissolved in sterile water to 490 µl sample to reach a final DTT concentration of 10 mM. The samples were incubated in room temperature for 1 hour, followed by buffer exchange to PBS with Amersham NAP-5 columns (Cytiva^{™}) according to protocol, resulting in 1 ml eluates. EZ-Link^{™} Maleimide-PEG2-Biotin, No weight^{™} format (Thermo Scientific) was dissolved in 190 µl PBS according to protocol and 5 µl dissolved biotin was added to each 1 ml eluate, to reach a final 2-5 molar excess of biotin. Samples were incubated on ice for 2 hours, followed by dialysis according to Slide-A-Lyzer^{™} G2 Dialysis Cassette (Thermo Scientific) protocol. The concentration of dialyzed samples was measured using NanoDrop (Thermo Scientific) according to manufacturer's instructions and kept in refrigerator in Eppendorf tubes until all measurements were performed and were then kept in freezer until further analysis. Protein mass was determined by liquid chromatography-mass spectrometry on a ACQUITY RDa Detector (Waters^{™}) using a BioRessolve column (Waters^{™}).

### Bis-Tris PAGE analysis

To evaluate the protein expression levels, a Bis-Tris PAGE was performed on filterered supernatant post heat treatment. Materials and equipment used were as follows: NuPAGE LDS sample buffer (x4), PageRuler prestained, 4-12% Bis-Tris minigel, Invitrogen NP0321BOX, Mini-gel tank (Invitrogen), 20x MES buffer (Invitrogen), QuickBlue Dye.

15 µl of filtered supernatant from post heat treatment was mixed with 5 µl LDS sample buffer. The sample mix was heated to 70°C for 10 minutes and lightly centrifuged to collect condensate from the tube cap. 15 µl sample mix for each expressed construct was loaded on the gel. 5 µl PageRuler was loaded as marker. The gel was run at 200 V for 35 minutes and stained in QuickBlue dye for 2 hours. The gel was destained in deionized water over night.

### Analysis of binding affinity for AAV9

To assess binding to AAV9, the polypeptides 2B-1, 2B-2 and 2B-3 as described above were immobilized on a Biacore^{™} Series S SA chip (Cytiva^{™}) and analyzed using Biacore^{™} 8K+ instrument (Cytiva^{™}). A sample containing AAV9 viral particles (vp) at a concentration of 2E12 vp/ml was used as analyte.

Immobilization was performed using a standard SA coupling method in Biacore software with coupling of the polypeptides in Flow Cell 2 (FC2) and activation/inactivation in Flow cell 1 (FC1). The polypeptides were diluted in PBS-P+ (Cytiva^{™}) at a concentration of approximately 25 µg/ml. The immobilization levels varied somewhat between different polypeptide variants corresponding to their molecular weight. In each run the polypeptide was immobilized in FC2.

Biacore^{™} method for binding analysis: Running buffer: PBS-P+ ; Flow rate: 5 µl/min ; Sample injection: 2400 s/40 min over both FC1 and FC2; Dissociation time: 2400 s/40 min ; Regeneration: 10 mM Glycine-HCl pH 1.5, 30 µl/min, 2x30 s. Injections of analyte (AAV9) for each channel (cycles) were as follows: running buffer, AAV9 2E11 vp/ml, 5E11 vp/ml, 2E12 vp/ml.

All sensorgrams were generated as reference subtracted and the responses as the difference between baseline before injection and signal just before end of injection.

### Analysis of alkaline stability

After evaluation of AAV9 binding (see above) the same chips were subjected to repeated cycles of binding to AAV9 (5E11 vp/ml) followed by injection of NaOH (0.3 M) for the assessment of alkaline stability.

Biacore^{™} method for alkaline stability (per cycle): Running buffer: PBS-P+ ; Flow rate: 10 µl/min ; Sample injection 1 (AAV9 5E11 vp/ml): 300 s over both flow cells ; Dissociation time 1: 30 s ; Sample injection 2 (0.3 M NaOH): 600 s over both flow cells ; Dissociation time 2: 0 s ; Regeneration: 10 mM Glycine-HCl pH 1.5, 30 µl/min, 2x30 s. This cycle was repeated 40 times to follow stability of AAV9 response values.

All sensorgrams were generated as reference subtracted and the responses as the difference between baseline before injection and signal just before end of injection.

### Results

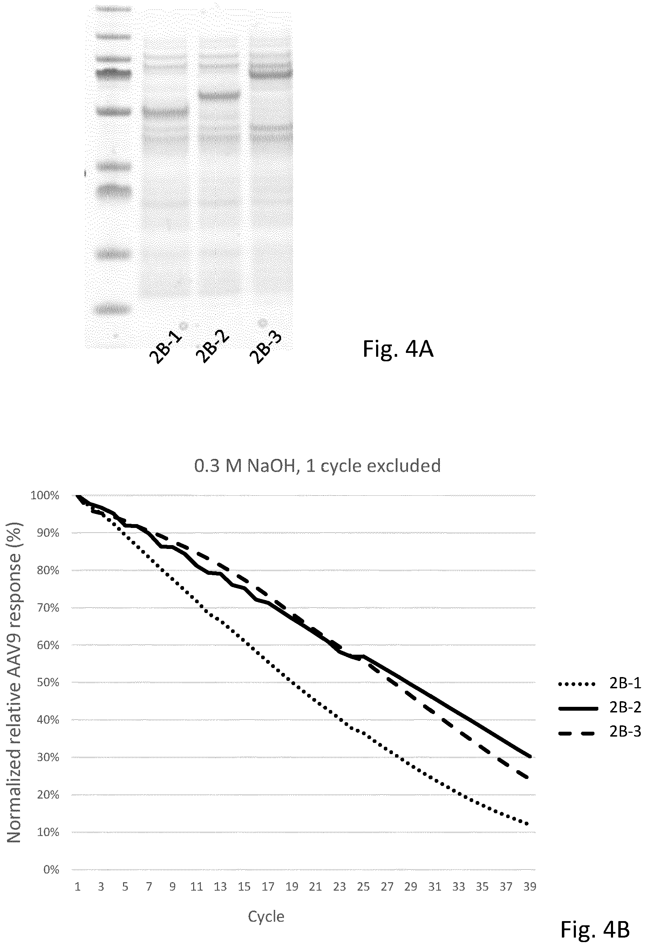

Analysis of expression level by Bis-Tris PAGE (Fig. 4A) indicated that the multimeric fusion proteins comprising a stabilizing polypeptide could improve protein expression.

Immobilization levels on SA chip were in the range of 3000-3500 RU. The AAV9 binding capacity at the maximum viral concentration used was higher for the tested fusion proteins 2B-2 and 2B-3 compared to the sdAb trimer without a stabilizing polypeptide.

Fig. 4B plots the AAV9 binding capacity for increasing number of cycles involving 0.3 M NaOH exposure (sample injection 2 above) normalized to the response after completing the first cycle (i.e., excluding the first cycle). As can be seen in this figure, the fusion proteins 2B-2 and 2B-3, both show notable improvement in alkaline stability over the sdAb trimer 2B-1. Normalizing the response to the response of the first cycle, it was found that 2B-3 experienced a larger drop in response between the first and second cycles than 2B-1 or 2B-2, but for the subsequent cycles remained more stable than 2B-1, which lost more of its binding capacity over the course of the test than the fusion proteins 2B-2 and 2B-3. Table 6 summarizes the results of the AAV9 binding capacity and alkaline stability measurements.

**Table 6**

| Sample | AAV9 capacity (2E12 vp/ml, RU) | No. of cycles with at least 50 % binding maintained (5E11 vp/ml, 100%=AAV9 response of 1^{st} cycle) | No. of cycles with at least 50 % binding maintained (5E11 vp/ml, 100%=AAV9 response of 2^{nd} cycle) |
|---|---|---|---|
| 2B-1 | 13410 | 12 | 20 |
| 2B-2 | 14328 | 21 | 30 |
| 2B-3 | 14991 | 11 | 28 |

### Example 3: GFP-binding polypeptides with different Z variants

In this example, 47 fusion proteins having the same GFP-binding VHH (SEQ ID NO:120) and different stabilizing polypeptides were evaluated. The stabilizing polypeptides were SpA domain Z variants having different sets of amino acids in positions 9, 10, 11, 13, 14, 17, 18, 28, 29, 33, 40, 51 as outlined in Table 7. The stabilizing polypeptide was fused to the C-terminus of the GFP-binding polypeptide via a peptide linker (QAS). The fusion protein had a C-terminal (His)s tag joined to the stabilizing polypeptide via a GS linker.

**Table 7. Details of stabilizing polypeptides used in fusion proteins.**

| Stabilizing polypeptide identifier | Fusion protein identifier | Stabilizing polypeptide SEQ ID NO: | Varying amino acid positions | | | | | | | | | | | |
|---|---|---|---|---|---|---|---|---|---|---|---|---|---|---|
| | | | 9 | 10 | 11 | 13 | 14 | 17 | 18 | 28 | 29 | 33 | 40 | 51 |
| Z0385 | pAM1032 | 65 | A | Y | R | L | A | A | R | L | A | S | G | V |
| Z0386 | pAM1033 | 66 | A | Y | R | L | A | A | R | E | A | S | G | V |
| Z0387 | pAM1034 | 67 | A | Y | R | L | A | A | R | R | A | S | G | V |
| Z0388 | pAM1035 | 68 | A | Y | R | L | A | A | R | A | L | S | G | V |
| Z0389 | pAM1036 | 69 | A | Y | R | L | A | A | R | A | E | S | G | V |
| Z0390 | pAM1037 | 70 | A | Y | R | L | A | A | R | A | R | S | G | V |
| Z0391 | pAM1038 | 71 | A | Y | R | L | A | A | R | A | A | T | E | R |
| Z0392 | pAM1039 | 72 | A | Y | R | L | A | A | R | A | A | A | R | I |
| Z0393 | pAM1040 | 73 | A | Y | R | L | A | A | R | A | A | A | G | R |
| Z0394 | pAM1041 | 74 | A | Y | R | L | A | A | R | A | A | Q | D | I |
| Z0395 | pAM1042 | 75 | A | Y | R | L | A | A | R | A | A | G | R | L |
| Z0396 | pAM1043 | 76 | A | Y | R | L | A | A | R | A | A | H | K | L |
| Z0397 | pAM1044 | 77 | A | Y | R | L | A | A | R | A | A | E | G | L |
| Z0127 | pAM1050 | 64 | A | Y | R | L | A | A | R | A | A | S | G | V |
| Z0421 | pAM1146 | 78 | R | Y | R | L | A | A | R | A | A | S | G | V |
| Z0439 | pAM1164 | 79 | A | Y | R | L | A | L | R | A | A | S | G | V |
| Z0440 | pAM1165 | 80 | A | Y | R | L | A | A | E | A | A | S | G | V |
| Z0441 | pAM1166 | 81 | A | Y | R | L | A | A | L | A | A | S | G | V |
| Z0443 | pAM1168 | 82 | A | Y | R | L | A | E | L | A | A | S | G | V |
| Z0444 | pAM1169 | 83 | A | Y | R | L | A | E | A | A | A | S | G | V |
| Z0445 | pAM1170 | 84 | A | Y | R | L | A | L | A | A | A | S | G | V |
| Z0446 | pAM1171 | 85 | A | Y | R | L | A | L | E | A | A | S | G | V |
| Z0447 | pAM1172 | 86 | A | Y | R | L | A | R | A | A | A | S | G | V |
| Z0448 | pAM1173 | 87 | A | Y | R | L | A | R | E | A | A | S | G | V |
| Z0449 | pAM1174 | 88 | A | Y | R | L | A | R | L | A | A | S | G | V |
| Z0450 | pAM1175 | 89 | A | Y | R | L | A | R | R | A | A | S | G | V |
| Z0451 | pAM1176 | 90 | A | Y | R | L | Q | A | R | A | A | S | G | V |
| Z0452 | pAM1177 | 91 | A | Y | R | E | L | A | R | A | A | S | G | V |
| Z0453 | pAM1178 | 92 | A | Y | R | R | A | A | R | A | A | S | G | V |
| Z0454 | pAM1179 | 93 | A | Y | R | A | R | A | R | A | A | S | G | V |
| Z0455 | pAM1180 | 94 | A | Y | R | Q | A | A | R | A | A | S | G | V |
| Z0456 | pAM1181 | 95 | A | Y | R | L | E | A | R | A | A | S | G | V |
| Z0457 | pAM1182 | 96 | A | Y | R | E | R | A | R | A | A | S | G | V |
| Z0458 | pAM1183 | 97 | A | Y | R | R | L | A | R | A | A | S | G | V |
| Z0459 | pAM1184 | 98 | A | Y | R | A | Q | A | R | A | A | S | G | V |
| Z0460 | pAM1185 | 99 | A | Y | R | Q | E | A | R | A | A | S | G | V |
| Z0461 | pAM1186 | 100 | A | Y | R | Q | Q | A | R | A | A | S | G | V |
| Z0462 | pAM1187 | 101 | A | Y | R | R | R | A | R | A | A | S | G | V |
| Z0463 | pAM1188 | 102 | R | Y | R | A | R | E | R | E | A | S | G | R |
| Z0464 | pAM1189 | 103 | E | R | L | Q | A | A | A | A | L | A | R | I |
| Z0465 | pAM1190 | 104 | Y | A | E | R | L | R | A | A | R | A | G | V |
| Z0466 | pAM1191 | 105 | E | R | A | E | R | A | R | R | R | S | G | V |
| Z0468 | pAM1193 | 106 | R | A | L | R | R | E | L | A | A | A | G | R |
| Z0469 | pAM1208 | 107 | L | Y | R | L | A | A | R | A | L | S | G | S |
| Z0470 | pAM1209 | 108 | A | Y | L | L | A | A | R | A | L | Q | S | G |
| Z0471 | pAM1210 | 109 | A | Y | R | L | A | A | R | A | A | R | H | V |
| Z0472 | pAM1211 | 110 | A | Y | A | A | K | A | R | A | A | S | Q | V |

The following aspects were evaluated:
(1) Affinity assessment of GFP target molecule interaction (tested through varying concentration injection of target molecule).
(2) Alkaline stability (assessed by decreased GFP binding after increasing number of NaOH (0.3 M) treatments).
(3) IgG interaction tested through injection of Gammanorm (Octapharma^{™}) and trastuzumab (prepared in-house).

### Materials and methods

### Plasmid preparation

Materials and equipment used were as follows: Plasmid pAM1050; G-blocks fusion protein candidates (IDT^{™}); Restriction enzymes Hindlll and BamHl (NEB^{™}); Antarcic phosphatease (NEB^{™}); GFX^{™} PCR DNA and Gel Band Purification Kit (Cytiva^{™}); T4 DNA ligase (Thermo Fisher^{™}); Top10 *E. coli* cells (One Shot^{™} TOP10 Chemically Competent *E. coli,* ThermoFisher^{™}); SOC-media (Invitrogen^{™}); Agar plates, 100 µl carbenicillin/ml.

The plasmid pAM1050 and G-blocks containing the DNA sequences for all candidates were digested with restriction enzymes Hindlll and BamHl. The plasmid was dephosphorylated using antarctic phosphatease and gel-purified according to manufacturer's protocols.

Restriction enzyme digested G-blocks were ligated into the gel-purified plasmid using T4 DNA ligase for 2h at room temperature and transformed into KCM competent Top10 *E. coli* cells. Briefly, cells and ligation mix were incubated for 30 min on ice and 10min at room temperature in KCM-buffer. Cells were then transferred to SOC media and incubated at 37°C for 1h and then seeded on agar plates containing 100 µl carbenicillin/ml and incubated at 37°C over night.

### Protein expression

Materials and equipment used were as follows: Transformed *E. coli* colonies for candidate fusion proteins; LB culture medium, Terrific Broth (TB) culture medium, carbenicillin, IPTG 1 M, 125 ml & 250 ml baffled glass shake flasks, Infors HT Shaking incubator; water bath; centrifuge (Beckman Coulter^{™}).

Single colonies were picked from agar plates and inoculated in 3 ml LB media containing 100µl carbenicillin/ml at 37°C over night (150 rpm agitation). The following day, 250 µl overnight culture was transferred to 25ml TB medium containing 100 µl carbenicillin/ml and grown at 37°C and 150 rpm shaking until OD600 reached 0.6-0.9. Thereafter IPTG was added to a final concentration of 1mM, and the flasks were incubated over night at 27°C and 150 rpm shaking. The following day the cultures were pelleted (3000g, 5 min) and re-suspended in 25ml PBS. The cell suspension was heated to 48°C for 2h, and then centrifugated 8000g for 10min. The supernatants were transferred to new tubes and stored at -20°C until purification.

### Protein purification

Materials and equipment used were as follows: ÄKTA Pure P150 system (Cytiva^{™}); HiTrap MabSelect PrismA chromatography column (Cytiva^{™}); NanoDrop One (Thermo Fisher^{™}); NuPAGE^{™} 4-12%, Bis-Tris Mini Protein Gel (Thermo Fisher^{™}); ACQUITY RDa Detector (Waters^{™}); BioRessolve column (Waters^{™}).

Supernatants from protein expression were filtered (0.45µm) and loaded on to a HiTrap MabSelect PrismA column using an ÄKTA Pure P150 system. Samples were washed with PBS and 50mM sodium acetate (pH 6) and eluted in 50mM sodium acetate pH 3.5. Protein concentration was determined by NanoDrop One and purity was assessed on a NuPAGE^{™} SDS-PAGE gel. Protein mass was determined by liquid chromatography-mass spectrometry on a ACQUITY RDa Detector system using a BioRessolve column.

### Biacore binding analysis

Materials and equipment used were as follows: Biacore^{™} 8K+ instrument (Cytiva^{™}); CM5 sensor chips (Cytiva^{™}), Biacore^{™} Amine coupling kit (Cytiva^{™}), fusion protein candidates produced as described above, Green fluorescent protein (GFP, Merck^{™}); Gammanorm (Octapharma^{™}); trastuzumab (produced and purified from in-house production cell line (CHO-K1 background)).

All purified candidates were diluted to a concentration of 25µg/ml in 10mM sodium acetate buffer pH 5. Immobilization on CM5 sensor chips was performed using Biacore^{™} Amine coupling kit and a standard immobilization method in the Biacore software. Candidates were immobilized in flow cell 2 (Fc2) and flow cell 1 (Fc1) was activated/inactivated and used as a reference surface.

Interactions between the candidates and trastuzumab, Gammanorm and GFP, respectively, were studied as follows:
One injection of Gammanorm 1µM and one injection of trastuzumab 1uM, contact time 240s, 240s dissociation time, flow rate 10µl/min.

Three concentrations of GFP were studied: 10nM, 25nM and 100nM. 600s association, 600s dissociation time, 10 µl/min flow rate.

After each cycle, the surface was regenerated with two injections (30 s, 30 µl/min) of 10mM glycine-HCl pH 1.5. All generated sensorgrams were reference subtracted and the final response was given as the signal difference between baseline before injection and at the end of sample injection.

### Biacore alkaline stability analysis

After the binding analysis (see above) the same CM5 chips having immobilized polypeptides were subjected to repeated cycles of binding to GFP followed by injection of 0.3 M NaOH for the assessment of alkaline stability.

Method for alkaline stability (per cycle):
Start up was performed with PBS-P+ (prior to first sample injection only).

Sample injection 1: 50nM GFP in cycle one and two. Then GFP injection every other cycle, alternating with PBSP+ (no analyte).

300 s association time, 30 s dissociation time, 10µl/min.

Sample injection 2: 0.3M NaOH. 600s contact time, 0s dissociation time.

Regeneration: Two consecutive injections with Glycine pH 1.5 30 s, 30µl/min.

The method was repeated for 40 cycles in total. All generated sensorgrams were reference subtracted and the final response was given as the signal difference between baseline before injection and at the end of sample injection.

### Results

All candidates were successfully cloned and transformed into Top10 *E. coli,* expressed and purified, with the expected protein mass (data not shown). Immobilization levels on chip were in the range 3000-4500 RU.

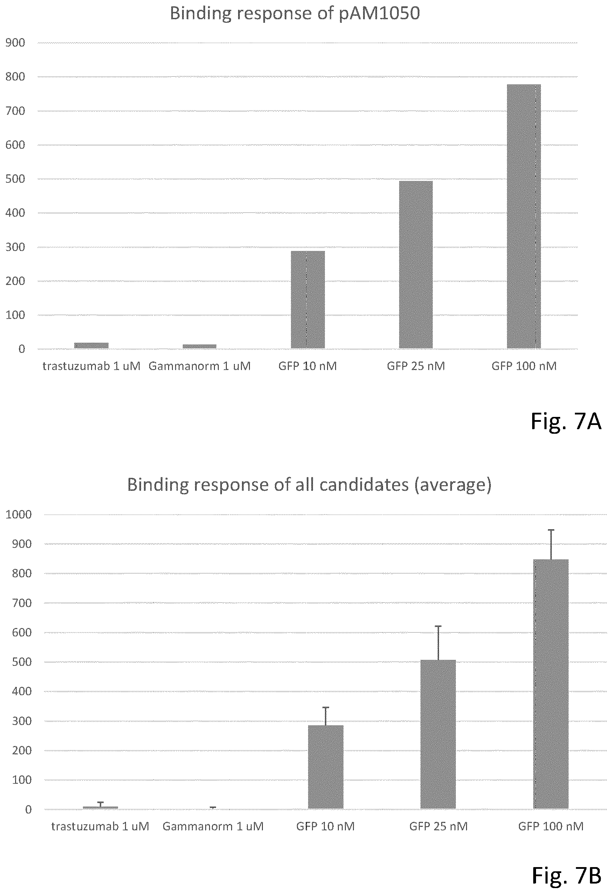

Fig. 7A-B show the detected Biacore binding responses (RU) to trastuzumab (1µM), Gammanorm (1µM) and GFP (10, 25 and 100 nM, respectively) for pAM1050 (Fig. 7A) and the average of all constructs (Fig. 7B). All of the fusion proteins exhibited affinity for GFP, and interaction with IgG was close to zero for all candidates. For reference, a fusion protein containing the same GFP-binding VHH fused to an SpA domain derivative (SEQ ID NO:9) having preserved Fc interaction but no VH3 interaction gave a trastuzumab (1µM) response of 9533 RU and a Gammanorm (1 µM) response of 8707 RU in an identical experimental setup (immobilization level: 3807 RU). Hence, the capability of the stabilizing polypeptides to interact with Fc and VH3 portions was abolished for the tested candidates.

The alkaline stability varied somewhat between tested candidates. Table 8 reports the GFP response (not normalized) of cycles 1, 2 and 20 of the alkaline stability assessment.

**Table 8. Response to GFP injection (50 nM) after repeated cycles of exposure to 0.3 M NaOH**

| **Fusion protein** | **GFP response [RU]** | | |
|---|---|---|---|
| | **Cycle 1** | **Cycle 2** | **Cycle 20** |
| pAM 1032 | 508 | 182 | 113 |
| pAM 1033 | 517 | 176 | 101 |
| pAM 1034 | 558 | 295 | 174 |
| pAM 1035 | 497 | 189 | 118 |
| pAM 1036 | 484 | 199 | 118 |
| pAM 1037 | 528 | 239 | 143 |
| pAM 1038 | 449 | 136 | 81 |
| pAM 1039 | 484 | 206 | 119 |
| pAM 1040 | 451 | 167 | 101 |
| pAM 1041 | 416 | 151 | 87 |
| pAM1042 | 439 | 189 | 113 |
| pAM1043 | 448 | 177 | 107 |
| pAM1044 | 412 | 167 | 94 |
| pAM1050 | 460 | 193 | 110 |
| pAM1146 | 294 | 157 | 85 |
| pAM1164 | 551 | 215 | 118 |
| pAM1165 | 402 | 147 | 90 |
| pAM1166 | 499 | 173 | 88 |
| pAM1168 | 453 | 157 | 89 |
| pAM1169 | 461 | 132 | 76 |
| pAM1170 | 487 | 111 | 66 |
| pAM1171 | 483 | 139 | 74 |
| pAM1172 | 559 | 205 | 121 |
| pAM1173 | 502 | 126 | 76 |
| pAM1174 | 569 | 237 | 134 |
| pAM1175 | 556 | 224 | 131 |
| pAM1176 | 454 | 185 | 101 |
| pAM1177 | 241 | 127 | 101 |
| pAM1178 | 561 | 261 | 148 |
| pAM1179 | 503 | 274 | 162 |
| pAM1180 | 521 | 251 | 143 |
| pAM1181 | 418 | 213 | 128 |
| pAM1182 | 537 | 229 | 125 |
| pAM1183 | 555 | 241 | 134 |
| pAM1184 | 456 | 194 | 107 |
| pAM1185 | 445 | 162 | 93 |
| pAM1186 | 453 | 197 | 116 |
| pAM1187 | 561 | 232 | 131 |
| pAM1188 | 523 | 227 | 128 |
| pAM1189 | 481 | 217 | 123 |
| pAM1190 | 459 | 188 | 103 |
| pAM1191 | 453 | 189 | 107 |
| pAM1193 | 533 | 158 | 96 |
| pAM1208 | 476 | 198 | 112 |
| pAM1209 | 562 | 181 | 105 |
| pAM1210 | 425 | 178 | 109 |
| pAM1211 | 516 | 197 | 127 |

The data suggest that several substitutions in the tested positions were tolerated and resulted in fusion proteins having high alkaline stability. After 20 cycles, all tested candidates still retained more than 50 % of the binding response recorded for the second cycle. pAM1178-80 which contained different amino acids in positions 13 and 14, performed particularly well. Results for pAM1034 and pAM1037 indicate that R in position 28 or 29 may be advantageous.

### Example 4A: Single domain antibodies with Z fusion, no IgG interaction

Different single domain antibodies were fused C-terminally via a peptide linker to a stabilizing polypeptide and the following aspects were evaluated: (1) Affinity assessment of target molecule interaction, tested through various concentration injection of target molecule. (2) Alkaline stability, tested by reduction of target binding after increasing number of treatments with 0.1 M or 0.3 M NaOH.

The single domain antibodies were VHH variants directed against AAV9, Green fluorescent protein (GFP) or epidermal growth factor receptor (EGFR), and were based on native camelid VHH frameworks or a modified framework to enhance alkaline stability of the VHH variant as such. The native camelid frameworks used corresponded to the framework sequences of the VHH "EgA1" or "9G8", respectively, described in Schmitz et al, 2013, Structure 21, 1214-1224, or to the framework sequence of a GFP-binding VHH "S-Nb3" described in Fleetwood et al., Cell. Mol. Life Sci. (2013) 70:1081-1093, except that in relation to the latter the first two amino acids were replaced by VD. CDR regions were the same for candidates binding the same target.

The stabilizing polypeptide was an SpA domain Z variant ("Z0127") (SEQ ID NO:64) in which binding to the Fc and VH3 portions of IgG had been abolished.

The tested polypeptide candidates (fusion proteins) are outlined in Table 9.

**Table 9.**

| Candidate no. | VHH variant | Target | Type of VHH framework | Stabilizing polypeptide | Full protein sequence |
|---|---|---|---|---|---|
| 4A-1 | vh342 | AAV9 | Native (EgA1) | Z0127 | SEQ ID NO:134 |
| 4A-2 | vh343 | AAV9 | native (9G8) | Z0127 | SEQ ID NO:136 |
| 4A-3 | vh118 | AAV9 | stabilized | Z0127 | SEQ ID NO:142 |
| 4A-4 | vh166 | EGFR | Native (9G8) | Z0127 | SEQ ID NO:131 |
| 4A-5 | vh167 | EGFR | stabilized | Z0127 | SEQ ID NO:132 |
| 4A-6 | vh25 | GFP | Modified native (S-Nb3) | Z0127 | SEQ ID NO:112 |
| 4A-7 | vh60 | GFP | native (EgA1) | Z0127 | SEQ ID NO:116 |
| 4A-8 | vh113 | GFP | stabilized | Z0127 | SEQ ID NO:123 |

### Materials and methods

### Generation of Immobilized Metal Affinity Chromatography (IMAC) purified candidate polypeptides

For each candidate 5 µl glycerol stock produced as described in Example 1B was inoculated in 4 ml LB culture medium supplemented with 100 µg/ml carbenicillin in 14 ml round-bottom tube and incubated at 37°C over night at 160 rpm.

Protein expression culture medium (TB medium supplemented with 100 µg/ml carbenicillin and 2 mM MgCl₂) was prepared and added to filled 500 ml baffled glass shake flasks (50 ml per flask). Each flask was inoculated with approximately 500 µl from the previous overnight culture to obtain a starting OD600 of 0.05. The flasks were incubated in Infors HT shaking incubator for approximately 3.5 hours at 37°C and 140 rpm shaking until OD600 reached 1.0. Thereafter 50 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the Falcon tubes were incubated in 48°C water bath for 2 hours followed by pelleting of cell debris by centrifugation at 8000 g for 10 min and filtration of supernatant with a 0.22 µm filter.

The samples were purified using HisTrap^{™} FF 1 ml chromatography column (Cytiva). Clarified lysate was loaded on the chromatography column, equilibrated in 50 mM sodium phosphate pH 7.5, 500 mM NaCl, allowing a residence time of about 2 min. The column was then washed with 15 column volumes (CV) of 50 mM sodium phosphate pH 7.5, 500 mM NaCl. Protein was eluted using 0.5 M imidazole pH 7.5 - 8, linear gradient (0-100 %) over 10 CV. Eluted protein was buffer-exchanged into phosphate buffered saline (Medicago) pH 7.4 using gravity flow columns prepacked with Sephadex^{™} G-25 resin (Cytiva) prior to further analysis.

Concentration of purified and buffer exchanged samples was measured using NanoDrop (Thermo Scientific) according to manufacturer's instructions. Following purification, samples were kept in refrigerator in Eppendorf tubes until all measurements were performed and were then kept in freezer until Biacore analysis.

### Biacore analysis of binding affinity for target molecules

To assess binding to target molecule (AAV9, GFP or EGFR), candidate polypeptides were immobilized on a Biacore^{™} Series S CM5 chip (Cytiva) using Biacore^{™} Amine coupling kit (Cytiva) and analyzed with a Biacore^{™} 8K+ instrument (Cytiva). The respective analytes used were AAV9 (2E12 vp/mL), GFP (1 g/L) and EGFR (0.2 g/L).

Immobilization was performed using a standard method in Biacore software with coupling of polypeptide variants in Flow Cell 2 (FC2) and activation/inactivation in Flow cell 1 (FC1). Polypeptide variants were diluted in Biacore^{™} Acetate buffer pH 5.0 (Cytiva^{™}) at a concentration of 25 µg/ml. The immobilization levels on CM5 chip ranged between about 1000 and 3300 RU but were generally around 3000 RU.

In each run the polypeptide was immobilized in FC2. Multiple sensor chips were used until all candidates were tested.

Biacore^{™} method for binding analysis to AAV9: Running buffer: PBS-P+; Flow rate: 5 µl/min ; Sample injection: 2400 s/40 min over both FC1 and FC2 ; Dissociation time: 2400 s/40 min ; Regeneration: 10 mM Glycine-HCl pH 1.5, 30 µl/min, 2x30 s. Injections of analyte (AAV9) for each channel (cycles) were as follows: running buffer, 2E11 vp/ml, 5E11 vp/ml, 2E12 vp/ml.

Biacore^{™} method for binding analysis to GFP or EGFR: Running buffer: PBS-P+ ; Flow rate: 10 µl/min ; Sample injection: 600 s/10 min over both FC1 and FC2; Dissociation time: 600 s/10 min ; Regeneration: 10 mM Glycine-HCl pH 1.5, 30 µl/min, 2x30 s. Injections of analyte (GFP) for each channel immobilized with GFP binding polypeptides (cycles) were as follows: running buffer, 10 nM, 25 nM and 100 nM. Injections of analyte (EGFR) for each channel immobilized with EGFR binding polypeptides (cycles) were as follows: running buffer, 6.25 nM, 12.5 nM, 25 nM, 50 nM, 100 nM and 200 nM.

All sensorgrams were generated as reference subtracted and the responses as the difference between baseline before injection and signal just before end of injection.

### Biacore analysis of alkaline stability

After the binding analysis (see above) the same CM5 chips having immobilized polypeptides were subjected to repeated cycles of binding to the respective analyte followed by injection of NaOH for the assessment of alkaline stability (0.3 M for AAV9 binding polypeptides and 0.1 M for GFP and EGFR binding polypeptides, respectively).

Biacore method for alkaline stability (per cycle): Running buffer: PBS-P+ ; Flow rate: 10 µl/min ; Sample injection 1 (AAV9 5E11 vp/ml or GFP 50 nM or EGFR 75 nM): 300 s over both flow cells ; Dissociation time 1: 30 s ; Sample injection 2 (0.1 M or 0.3 M NaOH): 600 s over both flow cells; Dissociation time 2: 0 s ; Regeneration: 10 mM Glycine-HCl pH 1.5, 30 µl/min, 2x30 s. This cycle was repeated 40 times to follow stability of target response values.

All sensorgrams were generated as reference subtracted and the responses as the difference between baseline before injection and signal just before end of injection.

### Results

All fusion proteins showed satisfactory binding to the respective target.

Tables 10a-c summarize the results of the binding capacity and alkaline stability measurements.

**Table 10a. AAV9 binding candidates**

| Sample | Binding capacity AAV9 (2E12 vp/ml) (RU) | Alkaline stability (0.3 M NaOH) | |
|---|---|---|---|
| | | No. of cycles with at least 50 % binding maintained (100% = response of 1^{st} cycle) | No. of cycles with at least 50 % binding maintained (100% = response of 2^{nd} cycle) |
| 4A-1 | 9924 | 1 | 2 |
| 4A-2 | 12361 | 1 | 2 |
| 4A-3 | 12443 | 26 | 34 |

**Table 10b. EGFR binding candidates**

| Sample | Binding capacity EGFR (200 nM) (RU) | Alkaline stability (0.1 M NaOH) | |
|---|---|---|---|
| | | No. of cycles with at least 50 % binding maintained (100% = response of 1^{st} cycle) | No. of cycles with at least 50 % binding maintained (100% = response of 2^{nd} cycle) |
| 4A-4 | 2813 | 1 | 2 |
| 4A-5 | 2352 | 6 | 28 |

**Table 10c. GFP binding candidates**

| Sample | Binding capacity GFP (100 nM) (RU) | Alkaline stability (0.1 M NaOH) | |
|---|---|---|---|
| | | No. of cycles with at least 50 % binding maintained (100% = response of 1^{st} cycle) | No. of cycles with at least 50 % binding maintained (100% =response of 2^{nd} cycle) |
| 4A-6 | 1125 | 1 | 2 |
| 4A-7 | 1124 | 1 | 8 |
| 4A-8 | 868 | 26 | >40 |

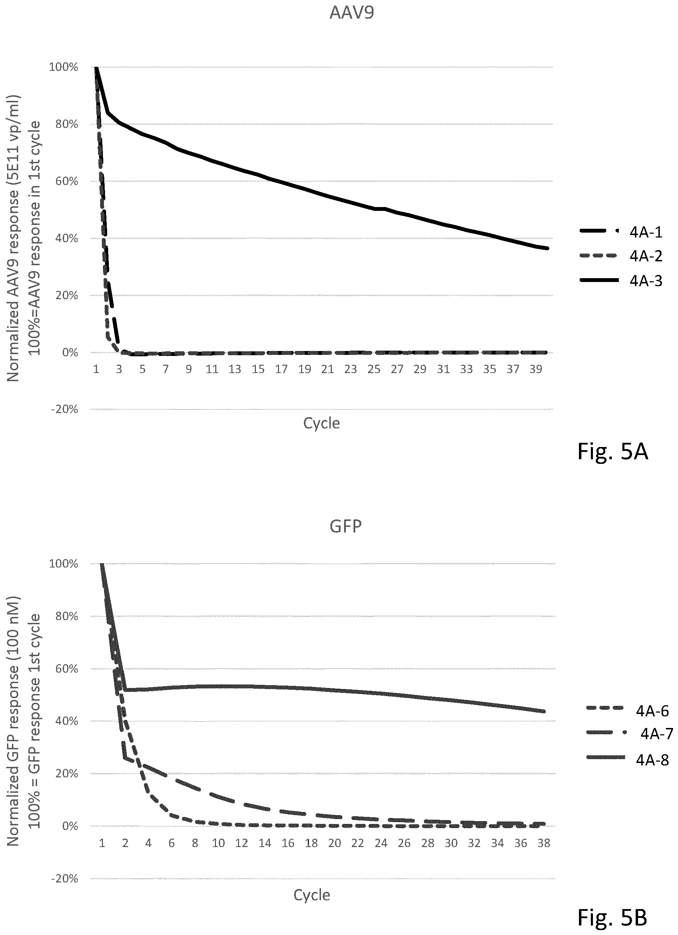

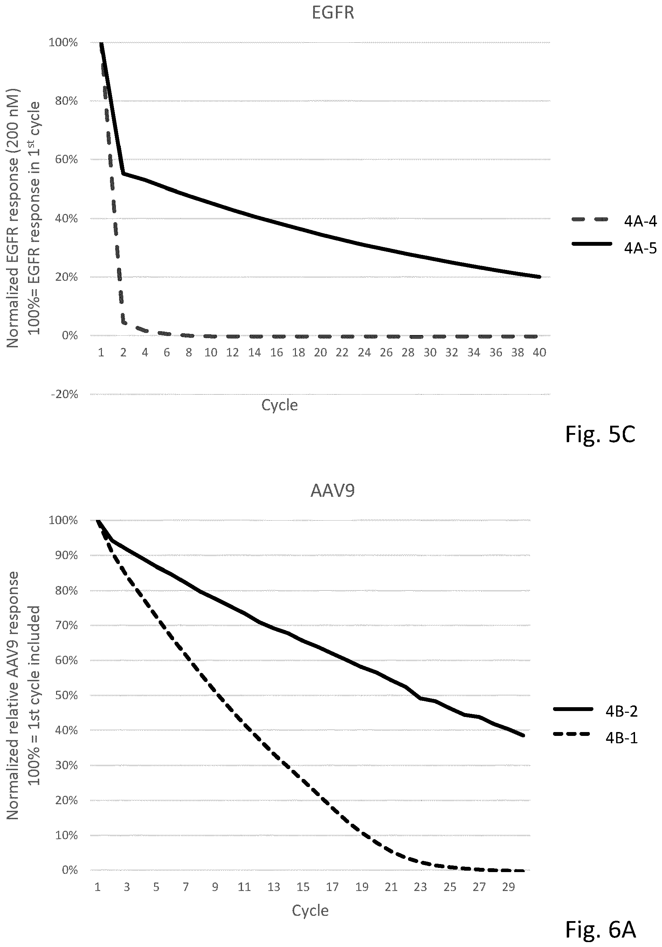

Figs. 5A-C show the binding capacities obtained for increasing number of cycles involving NaOH exposure (sample injection 2 above) as normalized to the response of the first cycle for the AAV9 binding fusion proteins (Fig. 5A), GFP-binding fusion proteins (Fig 5B) and EGFR-binding fusion proteins (Fig. 5C). As can be seen in these figures, the fusion proteins in which the VHH affinity ligand was based on stabilized VHH framework (4A-3, 4A-5, 4A-8) showed improved alkaline stability in relation to the corresponding fusion protein incorporating a VHH having a native camelid VHH framework.

### Example 4B: Single domain antibodies with Z fusion

Different single domain antibodies were fused C-terminally to a stabilizing polypeptide in the form of an SpA domain Z variant ("Z0091") (SEQ ID NO:9) capable of binding to immunoglobulin Fc but not VH3. The following aspects were evaluated: (1) Affinity assessment of target molecule interaction, tested through high concentration injection of target molecule. (2) Alkaline stability, tested by reduction of target binding after increasing number of treatments with 0.1 M, 0.3 or 0.5 M NaOH.

The single domain antibodies were VHH variants directed against AAV9, green fluorescent protein (GFP) or epidermal growth factor receptor (EGFR), and either were based on a native camelid VHH framework or were based on a modified framework to enhance alkaline stability of the VHH variant as such. The native camelid frameworks corresponded to the framework sequences of the VHH "EgA1" or "9G8", respectively, described in Schmitz et al. CDR regions were the same for candidates binding the same target.

The tested polypeptide candidates (fusion proteins) are outlined in Table 11.

**Table 11**

| Candidate no. | VHH variant | Target | Type of VHH framework | Stabilizing polypeptide | Full protein sequence |
|---|---|---|---|---|---|
| 4B-1 | vh97 | AAV9 | synthetic, partly stabilized | Z0091 | SEQ ID NO:138 |
| 4B-2 | vh118 | AAV9 | Synthetic, stabilized | Z0091 | SEQ ID NO:141 |
| 4B-3 | vh166 | EGFR | Native (9G8) | Z0091 | SEQ ID NO:129 |
| 4B-4 | vh167 | EGFR | Synthetic, stabilized | Z0091 | SEQ ID NO:130 |
| 4B-5 | vh60 | GFP | native (EgA1) | Z0091 | SEQ ID NO:115 |
| 4B-6 | vh113 | GFP | Synthetic, stabilized | Z0091 | SEQ ID NO:122 |

### Materials and methods

### Generation of IgG Sepharose purified candidate polypeptides

For each candidate 5 µl glycerol stock produced as described in Example 1A was inoculated in 4 ml LB culture medium supplemented with 50 µg/ml kanamycin in 14 ml round-bottom tube and incubated at 37°C over night at 160 rpm.

Protein expression culture medium (TB medium supplemented with 50 µg/ml kanamycin and 2 mM MgCl₂ was prepared and added to filled 100 ml baffled glass shake flasks (20 ml per flask). Each flask was inoculated with approximately 100 µl from the previous overnight culture to obtain a starting OD600 of 0.05. The flasks were incubated in Infors HT shaking incubator for approximately 3 hours at 37°C and 140 rpm shaking until OD600 reached 1.0. Thereafter 20 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the Falcon tubes were incubated in 48°C water bath for 2 hours followed by pelleting of cell debris by centrifugation at 8000 g for 10 min and filtration of supernatant with a 0.22 µm filter.

The samples were purified using gravity flow in PD-10 chromatography columns (Cytiva^{™}) packed in-house with IgG Sepharose 6FF chromatography resin (Cytiva^{™}). Clarified lysate was loaded on the chromatography column, equilibrated in 50 mM Tris, 150 mM NaCl, 0.05% Tween20 (TST buffer). The column was washed with 10 column volumes (CV) of TST buffer followed by 2 CV of 2 CV 5 mM NH₄Ac pH 5. Protein was eluted using 2.5 ml 0.5 M HAc. Eluted protein was buffer-exchanged into phosphate buffered saline (Medicago) pH 7.4 using gravity flow columns PD-10 (Cytiva) prior to further analysis.

Concentration of IgG Sepharose purified samples was measured using NanoDrop (Thermo Scientific) according to manufacturer's instructions. Following purification, samples were kept in refrigerator in Eppendorf tubes until all measurements were performed and were then kept in freezer until Biacore analysis. Protein mass was determined by liquid chromatography-mass spectrometry on a ACQUITY RDa Detector (Waters^{™}) using a BioRessolve column (Waters^{™}).

### Biacore analysis of binding affinity for target molecules

To assess binding to target molecule (AAV9, GFP or EGFR), candidate polypeptides were immobilized on a Biacore^{™} Series S CM5 chip (Cytiva) using Amine coupling kit (Cytiva) and analyzed with a Biacore^{™} 8K+ instrument (Cytiva). The respective analytes used were AAV9 (prepared in-house), trastuzumab-GFP (prepared in-house) and EGFR (SinoBiological, 10001-H08H), Fc from mAb (prepared inhouse).

Immobilization was performed using a standard method in Biacore software with coupling of polypeptide variants in Flow Cell 2 (FC2) and activation/inactivation in Flow cell 1 (FC1). AAV9 binding candidates were diluted in Biacore^{™} Acetate buffer pH 5.0 (Cytiva^{™}) at a concentration of 25 µg/ml, and the average immobilization levels was 4319 RU +SD 640 RU. GFP binding polypeptide variants were diluted in Biacore^{™} Acetate buffer pH 4.5 (Cytiva^{™}) at a concentration of 25 µg/ml, and average immobilization level was 3803 RU +SD 302 RU). EGFR binding polypeptide variants were diluted in Biacore^{™} Acetate buffer pH 4.5 (Cytiva^{™}) at a concentration of 25 µg/ml, and average immobilization level was 3719 RU +/- 463 RU). The polypeptides were immobilized in FC2.

Biacore^{™} method for AAV9 binding analysis: Running buffer: PBS-P+ ; Flow rate: 5 µl/min ; Sample injection: 2400 s/40 min over both FC1 and FC2; Dissociation time: 2400 s/40 min ; Regeneration: 10 mM Glycine-HCl pH 1.5, 30 µl/min, 2x30 s. Injections of analyte (AAV9) for each channel (cycles) were as follows: running buffer, 1.25E11 vp/ml, 5E11 vp/ml, 2E12 vp/ml.

Biacore^{™} method for GFP binding analysis: Running buffer: PBS-P+ ; Flow rate: 10 µl/min ; Block injection: 90 s over both FC1 and FC2; Sample injection: 600 s over both FC1 and FC2; Dissociation time: 1800 s ; Regeneration: 10 mM Glycine-HCl pH 1.5, 30 µl/min, 2x30 s. Injections of block (Fc) for each channel (cycles) were 2 µM. Injections of analyte (trastuzumab-GFP) for each channel (cycles) were as follows: running buffer, 1, 10, 100, 1000 nM.

Biacore^{™} method for EGFR binding analysis: Running buffer: PBS-P+ ; Flow rate: 10 µl/min ; Sample injection: 600 s over both FC1 and FC2; Dissociation time: 1800 s; Regeneration: 10 mM Glycine-HCl pH 1.5, 30 µl/min, 2x30 s. Injections of analyte (EGFR) for each channel (cycles) were as follows: running buffer, 0.1-200 nM in 1:1 dilution steps.

All sensorgrams were generated as reference subtracted and the responses as the difference between baseline before sample injection and signal just before end of injection.

### Biacore analysis of alkaline stability

After the binding analysis (see above) the same CM5 chips having immobilized polypeptides were subjected to repeated cycles of binding to the respective analyte followed by injection of NaOH for the assessment of alkaline stability (0.1/0.3/0.5 M).

Biacore method for AAV9 alkaline stability (per cycle): Running buffer: PBS-P+ ; Flow rate: 10 µl/min ; Sample injection 1 (AAV9 3E11 vp/ml): 300 s over both flow cells; Dissociation time 1: 30 s; Sample injection 2 (0.5 M NaOH): 600 s over both flow cells; Dissociation time 2: 0 s; Regeneration: 10 mM Glycine-HCl pH 1.5, 30 µl/min, 2x30 s.

Biacore method for GFP alkaline stability (per cycle): Running buffer: PBS-P+ ; Flow rate: 10 µl/min; Block injection: (Fc from mAb 2 µM) 90 s over both flow cells; Dissociation time block: 0 s; Sample injection 1 (trastuzumab-GFP 2µM ): 300 s over both flow cells ; Dissociation time 1: 30 s; Sample injection 2 (0.3 M NaOH): 600 s over both flow cells ; Dissociation time 2: 0 s; Regeneration: 10 mM Glycine-HCl pH 1.5, 30 µl/min, 2x30 s.

Biacore method for EGFR alkaline stability (per cycle): Running buffer: PBS-P+ ; Flow rate: 10 µl/min; Sample injection 1 (EGFR 0.1 M): 300 s over both flow cells; Dissociation time 1: 30 s; Sample injection 2 (0.1 M NaOH): 600 s over both flow cells; Dissociation time 2: 0 s; Regeneration: 10 mM Glycine-HCl pH 1.5, 30 µl/min, 2x30 s.

These cycles were repeated 30 times to follow stability of target response values.

All sensorgrams were generated as reference subtracted and the responses as the difference between baseline before injection and signal just before end of injection.

### Results

All fusion proteins showed satisfactory binding to the respective target.

Tables 12a-c summarize the results of the binding capacity and alkaline stability measurements.

**Table 12a. AAV9 binding candidates**

| Sample | Binding capacity AAV9 (2E12 vp/ml) (RU) | Alkaline stability (0.5 M NaOH) | |
|---|---|---|---|
| | | No. of cycles with at least 50 % binding maintained (100% = response of 1^{st} cycle) | No. of cycles with at least 50 % binding maintained (100%=response of 2^{nd} cycle) |
| 4B-1 | 14983 | 9 | 10 |
| 4B-2 | 12193 | 22 | 24 |

**Table 12b. EGFR binding candidates**

| Sample | Binding capacity EGFR (200 nM) (RU) | Alkaline stability (0.1 M NaOH) | |
|---|---|---|---|
| | | No. of cycles with at least 50 % binding maintained (100% = response of 1^{st} cycle) | No. of cycles with at least 50 % binding maintained (100%=response of 2^{nd} cycle) |
| 4B-3 | 2234 | 1 | 2 |
| 4B-4 | 1896 | 13 | 24 |

**Table 12c. GFP binding candidates**

| Sample | Binding capacity trastuzumab-eGFP (1 µM) (RU) | Alkaline stability (0.3 M NaOH) | |
|---|---|---|---|
| | | No. of cycles with at least 50 % binding maintained (100% = response of 1^{st} cycle) | No. of cycles with at least 50 % binding maintained (100%=response of 2^{nd} cycle) |
| 4B-5 | 4304 | 1 | 5 |
| 4B-6 | 3906 | >30 | >30 |

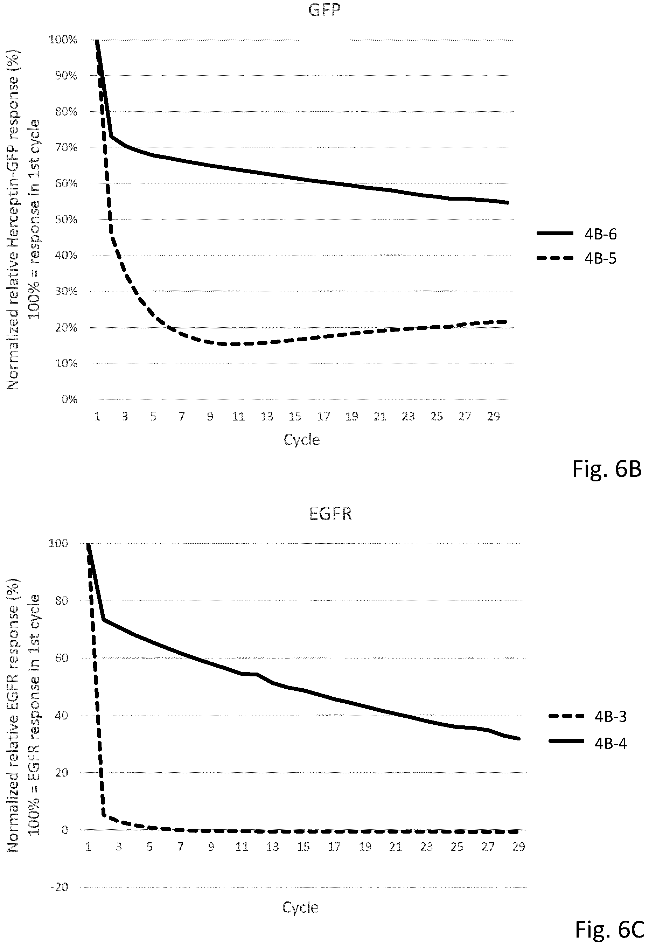

Figs. 6a-c show the binding capacities obtained for increasing number of cycles involving NaOH exposure (sample injection 2) as normalized to the response of the first cycle (i.e., including the first cycle) for the AAV9 binding fusion proteins (Fig. 6a), GFP-binding fusion proteins (Fig 6b) and EGFR-binding fusion proteins (Fig. 6c). As can be seen in these figures, fusion proteins in which the VHH affinity ligand was based on stabilized VHH framework (4B-2, 4B-4, 4B-6) showed improved alkaline stability in relation to the corresponding fusion protein incorporating a VHH having a native camelid VHH framework (4B-3, 4B-5) or a synthetic but not optimally stabilized framework (4B-1).

### Example 5: IgG binding fusion proteins

An IgG-binding polypeptide (representing the first polypeptide moiety) was expressed and evaluated alone or in fusion with a C-terminal stabilizing polypeptide. The IgG-binding polypeptide was protein L domain B3 and the C-terminal fusion partner was an SpA derived polypeptide (Z0127) (SEQ ID NO:64) in which binding to the Fc and VH3 portions of IgG had been abolished. The following constructs were tested:

**Table 13.**

| Protein | Stabilizing polypeptide | Full protein sequence |
|---|---|---|
| pL1 | - | SEQ ID NO:146 |
| PL1-Z0127 | Z0127 | SEQ ID NO:147 |

The effect of the stabilizing fusion partner on alkaline stability was assessed by measuring binding after repeated exposure to NaOH 0.3 M.

### Materials and methods

### Generation of Immobilized Metal Affinity Chromatography (IMAC) purified candidate polypeptides

For each variant 1 µl glycerol stock produced as described in Example 1B was inoculated in 3 ml LB culture medium supplemented with 100 µg/ml carbenicillin in 14 ml round-bottom tube and incubated at 37°C over night at 180 rpm.

Protein expression culture medium (TB medium supplemented with 100 µg/ml carbenicillin and 2 mM MgCl₂) was prepared and added to filled 100 ml baffled glass shake flasks (25 ml per flask). Each flask was inoculated with 250 µl from the previous overnight culture to obtain a starting OD600 of approximately 0.05. The flasks were incubated in Infors HT shaking incubator for approximately 3.5 hours at 37°C and 150 rpm shaking until OD600 reached 1.0. Thereafter 25 µl IPTG (1 M) was added to a final concentration of 1 mM and the flasks were incubated in Infors HT shaking incubator at 27°C and 150 rpm shaking for 18 hours, whereafter the cultures were transferred to 50 ml polypropylene centrifuge tubes. To generate crude variant lysates the centrifuge tubes were incubated in 48°C water bath for 2 hours followed by pelleting of cell debris by centrifugation at 8000 g for 10 min and filtration of supernatant with a 0.22 µm filter.

The samples were purified using HisTrap^{™} FF 1mL chromatography column (Cytiva). Clarified lysate was loaded on the chromatography column, equilibrated in 50 mM sodium phosphate pH 7.5, 500 mM NaCl, allowing a residence time of about 2 min. The column was then washed with 15 column volumes (CV) of 50 mM sodium phosphate pH 7.5, 500 mM NaCl. Protein was eluted using 0.5 M imidazole pH 7.5 - 8, linear gradient (0-100 %) over 10 CV. Eluted protein was buffer-exchanged into phosphate buffered saline (Medicago) pH 7.4 using gravity flow columns prepacked with Sephadex^{™} G-25 resin (Cytiva) prior to further analysis.

Concentration of purified and buffer exchanged samples was measured using NanoDrop (Thermo Scientific) according to manufacturer's instructions. Following purification, samples were kept in freezer until Biacore analysis.

### Biacore binding and alkaline stability

The binding interaction of Protein L to IgG was assessed by measuring binding to adalimumab (prepared in-house) on a Biacore 8K system (Cytiva). The proteins to be tested were amine coupled to a series S CM 5 sensor chip using the Biacore Amine coupling kit (Cytiva). Each protein was diluted in NaAc pH 4.5 to 10 µg/ml and immobilized in FC2 using the standard immobilization protocol. FC1 was activated and deactivated and was used as reference for all runs.

Alkaline stability was assessed by performing 20 cycles of the following consecutive steps:
(1) Binding of adalimumab at 200 nM for 180 s at 10 µl/min followed by 100 s dissociation.
(2) Wash with 0.3 M NaOH for 600 s at 10 µl/min.
(3) Regeneration with glycine pH 1.5 for 30 s at 30 µl/min.

The binding was recorded using the report point "binding late" and plotted against the number of cycles.

### Results

Table 14 shows immobilization levels obtained for each protein.

**Table 14.**

| Protein | Molecular weight (g/mol) | Immobilization level (RU) | Normalized immobilization level (g/(mol*RU)) |
|---|---|---|---|
| PL1-Z0127 | 15816 | 1795 | 8.8 |
| pL1 | 9347 | 1133 | 8.3 |

Taking the molecular weight into account, immobilization resulted in similar levels of protein on the surface, although slightly higher for the fusion protein.

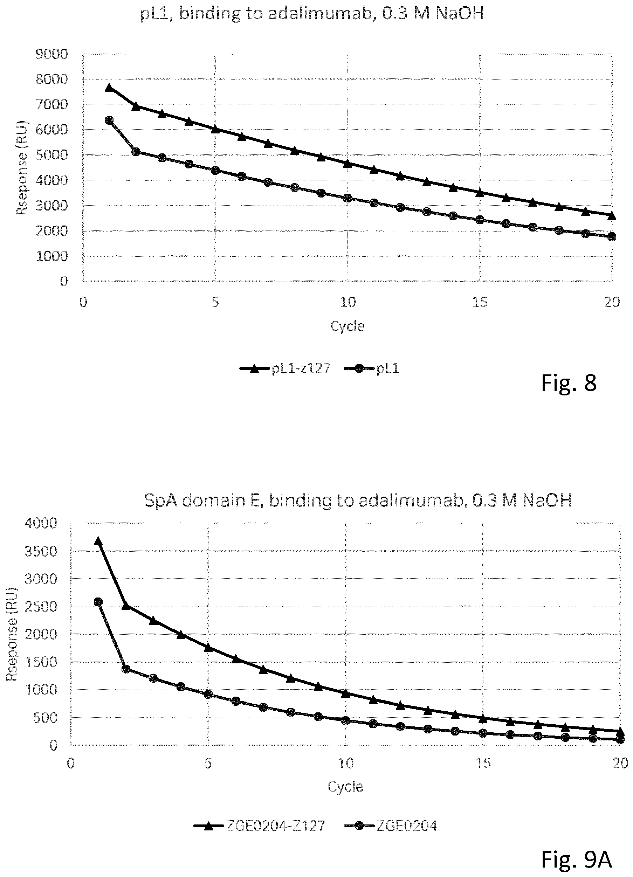

Fig 8 shows the binding of adalimumab to the surface over 20 cycles with 0.3M NaOH exposure. As can be seen in this graph, the fusion protein (triangles) initially produces a slightly higher binding signal than pL1 alone (dots), owing to the higher immobilization level, and deteriorates at a slower rate upon repeated alkali exposure.

### Example 6: IgG binding fusion proteins

This example aimed at expressing and evaluating IgG-binding polypeptides (each representing the first polypeptide moiety) alone or in fusion with a C-terminal stabilizing polypeptide. The IgG-binding polypeptides were SpA domains A, B, C, D and E, respectively, and the C-terminal fusion partner was an SpA derived polypeptide (Z0127, SEQ ID NO:64) in which binding to the Fc and VH3 portions of IgG had been abolished. The effect of the stabilizing fusion partner was evaluated for the following aspects: (1) Soluble expression yield from *E. coli* cultures, (2) Alkaline stability, assessed by measuring binding after repeated exposure to NaOH 0.3 M.

### Materials and methods

All experiments were performed as detailed in Example 5.

For alkaline stability testing, each tested protein was immobilized in separate flow cells in a Biacore instrument, on which binding and alkaline stability was assessed as described in Example 5.

### Results

It was found that fusion with the stabilizing polypeptide improved expression levels for Z0204 (domain E) and Z0205 (domain D). For Z0208 (domain C) and the corresponding fusion Z0208-Z0127, expression levels were similar. No expressed protein was obtained for Z0206 (domain A) and Z0207 (domain B) alone, possibly due to problems with the expression vectors. Since no reference was available, the fusion proteins Z0206-Z127 and Z0207-Z127 were not included in the alkaline stability test.

Table 15 identifies the evaluated proteins.

**Table 15**

| Protein | IgG binding polypeptide | Stabilizing polypeptide | Full protein sequence |
|---|---|---|---|
| Z0204-Z127 | SpA domain E (SEQ ID NO: 7) | Z0127 | SEQ ID NO:148 |
| Z204 | | - | SEQ ID NO:232 |
| Z0205-Z127 | SpA domain D (SEQ ID NO: 6) | Z0127 | SEQ ID NO:149 |
| Z0205 | | - | SEQ ID NO:233 |
| Z0208-Z127 | SpA domain C (SEQ ID NO: 5) | Z0127 | SEQ ID NO:150 |
| Z0208 | | - | SEQ ID NO:234 |
| Z0207-Z127 | SpA domain B (SEQ ID NO: 4) | Z0127 | SEQ ID NO:151 |
| Z0206-Z127 | SpA domain A(SEQ ID NO: 3) | Z0127 | SEQ ID NO:152 |

Table 16 shows immobilization levels obtained for each protein. The immobilization resulted in similar levels of protein on the surface when the molecular weights are taken into account).

**Table 16**

| Protein | Molecular weight (g/mol) | Expression yield (mg/L) | Immobilization level (RU) | Normalized immobilization level (g/(mol*RU)) |
|---|---|---|---|---|
| Z0204-Z127 | 14199 | 59 | 1336 | 10.6 |
| Z0204 | 8522 | 15 | 655 | 13.0 |
| Z0205-Z127 | 14911 | 61 | 1445 | 10.3 |
| Z0205 | 9235 | 25 | 781 | 11.8 |
| Z0208-Z127 | 14628 | 29 | 1609 | 9.1 |
| Z0208 | 8952 | 26 | 987 | 9.0 |

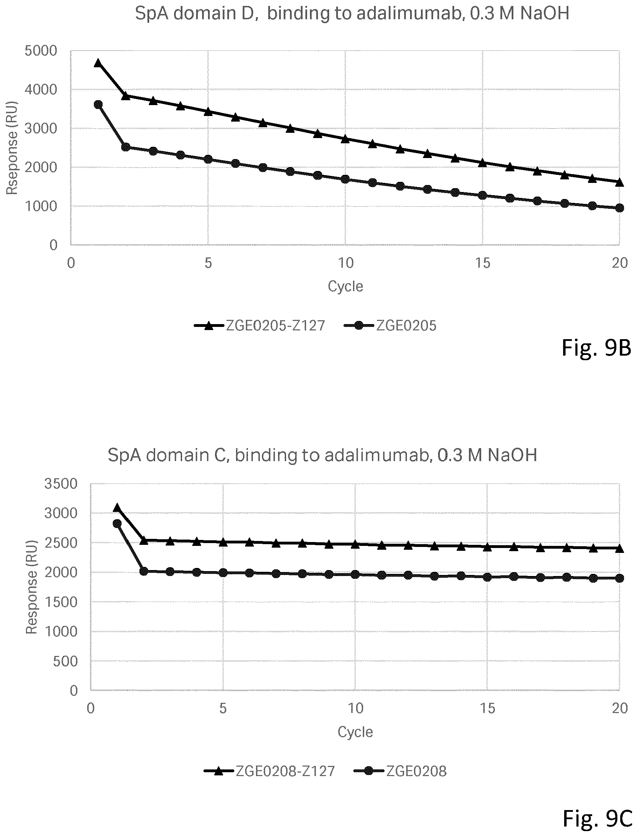

Figures 9A-C show the binding of adalimumab to each protein over 20 cycles involving 10 min of exposure to 0.3 M NaOH. In these figures, the SpA domains are referred to using the format "ZGE020X". All fusion proteins consistently gave a higher binding response the respective reference, despite being immobilized at similar levels or slightly lower, as in the case of domain C. Also, for domain D (Fig. 9B) and domain C (Fig. 9C), the fusion proteins experienced a less pronounced reduction in response between the first and second cycles compared to the respective reference SpA domain without a stabilizing fusion partner. In total, the fusion with a stabilizing polypeptide contributed to an improved alkaline stability of the immobilized protein, especially over the first 15 cycles for the three tested SpA domains.

### Example 7: HSA-binding fusion proteins

A single-chain variable fragment (scFv) representing the first polypeptide moiety was expressed and evaluated alone or in fusion with a C-terminal stabilizing polypeptide. The scFv had affinity for human serum albumin (HSA), and the C-terminal fusion partner was an SpA derived polypeptide (Z0127, SEQ ID NO:64) in which binding to the Fc and VH3 portions of IgG had been abolished. Both constructs were expressed with a C-terminal (His)s tag. The following constructs were tested:

**Table 17**

| Protein | Stabilizing polypeptide | Full protein sequence |
|---|---|---|
| scFvHSA | - | SEQ ID NO:153 |
| scFvHSA-Z127 | Z0127 | SEQ ID NO:154 |

The effect of the stabilizing fusion partner on alkaline stability was assessed by measuring binding after repeated exposure to NaOH 0.3 M.

### Materials and methods

All methods were done as detailed in Example 5. Briefly, the scFv was expressed with or without fusion to the stabilizing polypeptide and immobilized in flow cells in Biacore analysis. Binding and alkaline stability was assessed over 20 cycles. Two different NaOH concentrations, 0.01 M and 0.05 M were used separately to facilitate comparison between the constructs. Therefore, each protein was immobilized on two surfaces each using identical conditions.

### Results

Table 18 shows immobilization levels obtained for each protein. When the molecular weights (MW) are taken into account the immobilization resulted in similar levels, although slightly lower for the fusion proteins.

**Table 18**

| Protein | Surface | MW (g/mol) | Immobilization level (RU) | Normalized immobilization level (g/(mol*RU)) |
|---|---|---|---|---|
| scFvHSA-Z127 | Surface 1 | 33822 | 3778 | 9.0 |
| | Surface 2 | 33822 | 3844 | 8.8 |
| scFvHSA | surface 1 | 27339 | 2819 | 9.7 |
| | surface 2 | 27339 | 2811 | 9.7 |

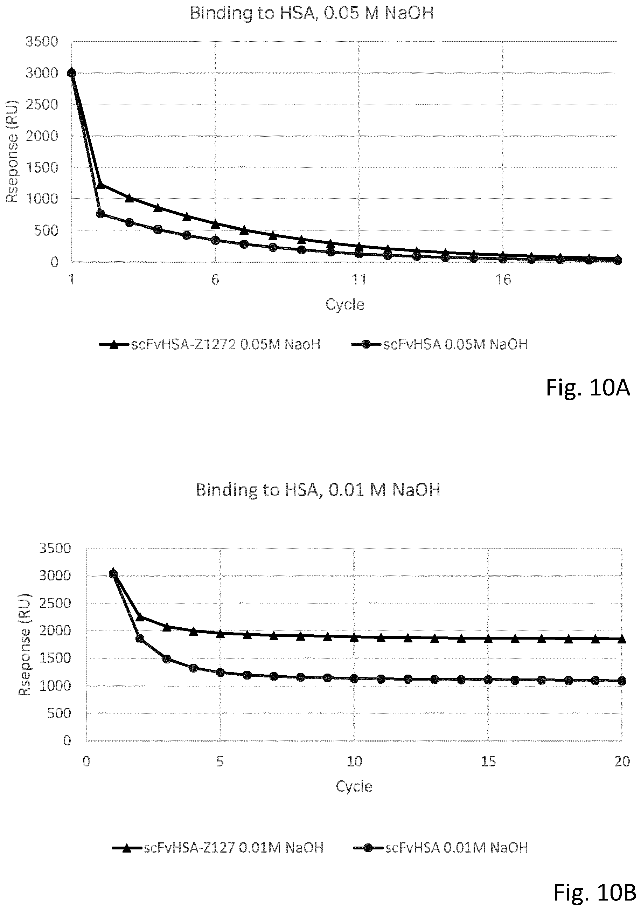

Figures 10A-B show results from the alkaline stability assessment at 0.05 or 0.01 M NaOH, respectively. The proteins had identical initial binding signals despite the fusion proteins being immobilized at slightly lower molar immobilization level. For the fusion protein, the drop in response between the first and second cycles was smaller than for the scFv reference, and with the higher NaOH concentration (0.05 M, Fig. 10A), it is also seen that the binding signal for the fusion protein declines at a slower rate than for the scFv alone. At 0.01 M NaOH (Fig. 10B), the binding signal declines and stabilizes for both constructs, but at a lower level for the construct without a stabilizing polypeptide. In conclusion, fusion with the stabilizing polypeptide improved alkaline stability of the anti-HSA scFv.

### Example 8: Functional evaluation

This example demonstrates with a basic experiment the functionality of a polypeptide immobilized on a support to form an affinity separation matrix. The polypeptide was a fusion of an AAV9 binding sdAb variant and a stabilizing polypeptide (SEQ ID NO: 64).

### Materials and methods

The fusion protein (SEQ ID NO:155) was produced and purified according to the method described in Example 2A.

The fusion protein was immobilized on epoxy activated chromatography resin (highly crosslinked agarose beads) using cysteine coupling. 0.2 ml of the resin was packed in Tricorn 5/20 columns (Cytiva). Using an ÄKTA pure 25 system (Cytiva), 50 ml of tangential flow filtrated AAV9 material (prepared in-house) was loaded on the column with four minutes residence time and eluted with 100 mM citrate buffer pH 2.5.

Collected eluate fraction was analysed on a Coomassie SDS-PAGE gel. The low pH elution sample was neutralized with 1:1 volume of Tris buffer (400 mM). 15 µl of sample and 5 µl sample buffer + DTT was heated to 70 °C for 10 min. 5 µl sample was loaded and the gel was run for 35 minutes at 200V and then soaked in Coomassie solution over night. The gel was then de-stained with water until satisfactory staining was achieved. The gel was photographed using ImageQuant 800.

To assess the alkaline stability of the immobilized fusion protein, the chromatography resin was subjected to cleaning-in-place (CIP) with 10 mM Glycine-HCl pH 1.5, followed by an alkaline stability study involving exposure to 0.5 M NaOH. The full stability study consisted of 40 cycles where each cycle involved contacting the chromatography material with 0.5M NaOH for 30 min. A solution containing pure AAV9 particles was added every 10th cycle with 15 seconds residence time. PBS buffer was used for all other cycles.

### Results

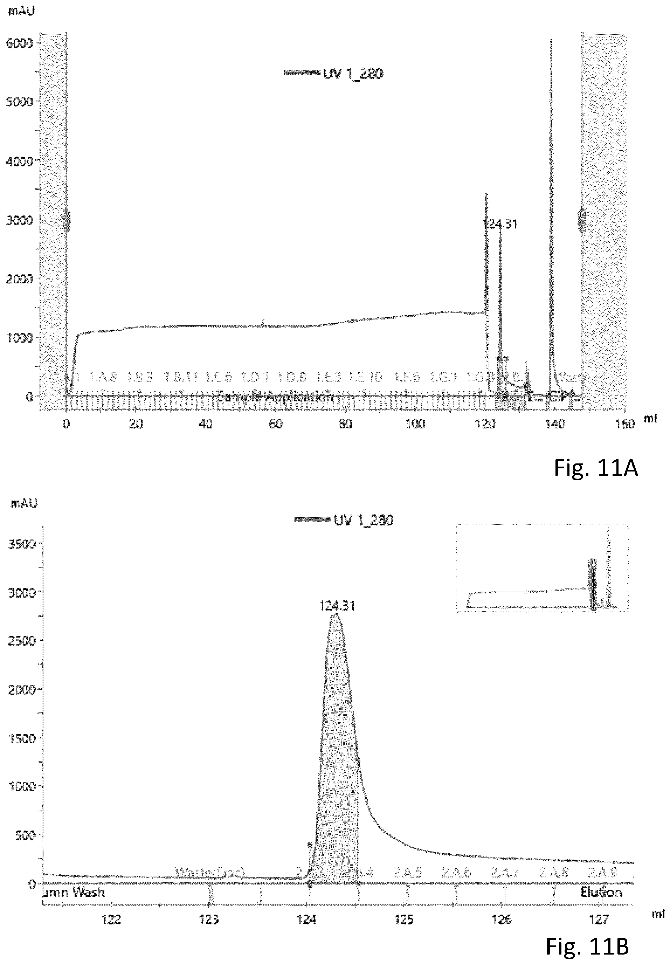

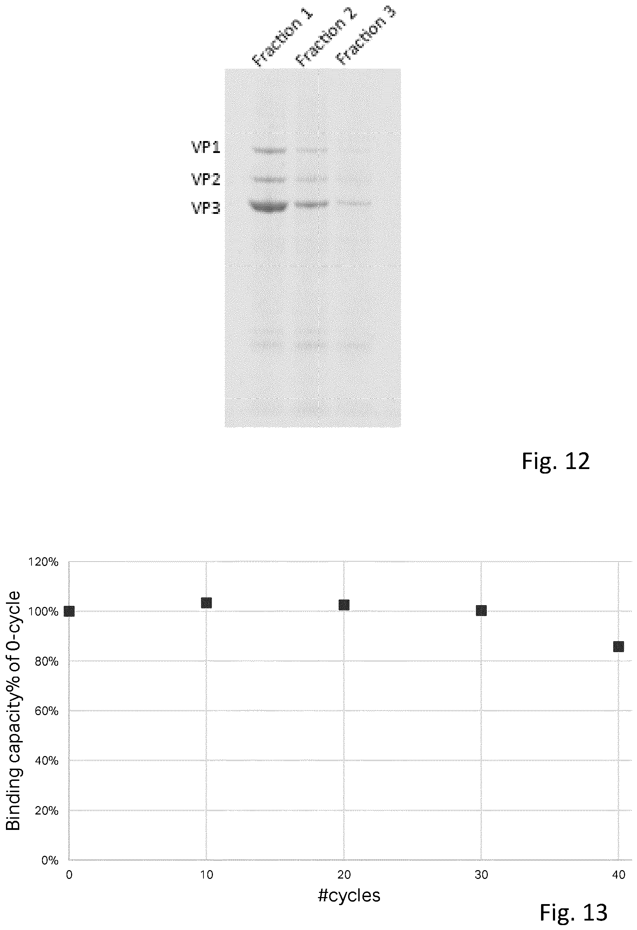

Fig. 11A shows the chromatogram obtained for purification of AAV9 TFF material using the candidate vh118 as an affinity ligand. The elution peak is marked with a rectangle. Fig. 11B shows only the region of the elution peak. Fig. 12 shows the photograph of the gel. VP1, VP2 and VP3 denotes the AAV9 virion proteins 1, 2 and 3, respectively.

The results show that the present candidate was able to successfully purify AAV9 viral particles, with an estimated dynamic binding capacity (QB10%) of about 3E+14 viral particles/ml resin and estimated recovery of loaded virus particles of 70-80%.

The result of the alkaline stability assessment is shown in Fig. 13 which plots the binding capacity normalized to the binding capacity before the first NaOH exposure. Over the 40 cycles of exposure to 0.5 M NaOH, which corresponded to an accumulated exposure time of 20 hours, the target binding capacity was reduced by less than 20 %.

### LIST OF ITEMIZED EMBODIMENTS

1. A fusion protein comprising at least one first polypeptide moiety and at least one second polypeptide moiety, wherein
the first polypeptide moiety is a single-chain polypeptide capable of binding a target entity, and
the second polypeptide moiety comprises a single-chain, α-helix-containing domain, wherein the second polypeptide moiety does not have binding affinity for said target entity,
and wherein presence of the second polypeptide moiety optionally improves at least one property of the fusion protein as compared to the property of the first polypeptide moiety alone, wherein said improved property is selected from the group consisting of: alkaline stability, recombinant protein expression and coupling to a support.
2. The fusion protein according to item 1 wherein improved alkaline stability is represented by the fusion protein as a whole having an alkaline stability that is increased as compared to the alkaline stability of the first polypeptide moiety alone.
3. The fusion protein according to item 1 or 2, wherein the first polypeptide moiety is a polypeptide domain.
4. The fusion protein according to any one of items 1-3, wherein the first polypeptide moiety is not an α-helix bundle domain.
5. The fusion protein according to any one of items 1-4, wherein the first polypeptide moiety has a secondary structure comprising beta sheets.
6. The fusion protein according to any one of items 1-5, wherein the first polypeptide moiety is a natural or synthetic antibody domain or antibody fragment or a modified variant thereof.
7. The fusion protein according to item 6, wherein the first polypeptide moiety comprises an antibody heavy chain variable domain.
8. The fusion protein according to item 6 or 7, wherein the first polypeptide moiety lacks antibody heavy chain constant domains.
9. The fusion protein according to any one of items 1-8, wherein the first polypeptide moiety is selected from the group consisting of an immunoglobulin heavy chain variable domain, a single-domain antibody (sdAb), a variable domain of a heavy chain of a heavy chain antibody (VHH), a VNAR, an immunoglobulin single chain variable fragment (scFv), and a synthetic variant of an antibody heavy chain variable domain in which a natural VH/VL interaction site has been modified to mimic a naturally occurring sdAb.
10. The fusion protein according to any one of items 1-8, wherein the single-chain polypeptide is a single-chain variable fragment or a single-domain antibody or a variant thereof.
11. The fusion protein according any one of items 1-10, wherein the single-chain polypeptide comprises a complementarity determining region 1 (CDR1), a complementarity determining region 2 (CDR2), and a complementarity determining region 3 (CDR3).
12. The fusion protein according to any one of items 1-3, wherein the first polypeptide moiety has a secondary structure comprising at least one beta sheet and at least one α-helix.
13. The fusion protein according to item 12, wherein the first polypeptide moiety is or is derived from a protein domain of Protein L or Protein G.
14. The fusion protein according to any one of items 1-3, wherein the first polypeptide moiety comprises an α-helix bundle domain.
15. The fusion protein according to item 14, wherein the first polypeptide moiety comprises a polypeptide which is or is derived from a protein domain of staphylococcal protein A (SpA).
16. The fusion protein according to item 15 wherein the first polypeptide moiety comprises an amino acid sequence selected from the group consisting of SEQ ID NOs:1-7 and amino acid sequences having at least 80 % identity to any one of SEQ ID NOs:1-7.
17. The fusion protein according to item 14 wherein the first polypeptide moiety comprises an albumin-binding domain (ABD).
18. The fusion protein according to any one of items 1-17, wherein the first polypeptide moiety alone has an alkali stability that is lower than the second polypeptide moiety alone.
19. The fusion protein according to any one of items 1-18, wherein the first polypeptide moiety alone retains 75 % or less, such as 50 % or less, of its target binding capacity after at most 20 hours, such as after 10 hours, of exposure to 0.5 M NaOH, or after at most 120 cycles, such as after 60 cycles, of repeated NaOH exposure wherein a cycle of NaOH exposure involved contact with 0.5 M NaOH for 10 minutes.
20. The fusion protein according to any one of items 1-19, wherein the first polypeptide moiety has a binding affinity for a biological entity.
21. The fusion protein according to item 20, wherein the biological entity is a virus particle or a viral vector, such as an adenovirus, a retrovirus (y-retroviruses and lentiviruses), a poxvirus, an adeno-associated virus (AAV), a baculovirus, or a herpes simplex virus.
22. The fusion protein according to item 21, wherein the biological entity is an adeno-associated virus (AAV), such as an AAV serotype 1, 2, 3, 4, 5, 6, 7, 8, 9, 10 (AAVrh10), 11 or 12.
23. The fusion protein according to 20, wherein the biological entity is an antibody, such as a monoclonal antibody, or an antibody fragment.
24. The fusion protein according to item 20, wherein the biological entity is an exosome or a lipid nanoparticle.
25. The fusion protein according to any one of items 1-24, wherein the second polypeptide moiety is a polypeptide domain.
26. The fusion protein according to any one of items 1-25, wherein the second polypeptide moiety is an α-helix-containing domain.
27. The fusion protein according to any one of items 1-26, wherein the second polypeptide moiety comprises at least 1 α-helix, such as at least 2 α-helices, such as 3, 4, 5 or 6 α-helices.
28. The fusion protein according to item 27, wherein the second polypeptide moiety comprises up to 10 α-helices.
29. The fusion protein according to any one of items 1-28, wherein the second polypeptide moiety is an α-helix bundle domain.
30. The fusion protein according to any one of item 1-29, wherein the second polypeptide comprises a three-helix bundle domain.
31. The fusion protein according to any one of items 1-30, wherein the second polypeptide is a single-domain polypeptide.
32. The fusion protein according to any one of items 1-31, wherein the second polypeptide is or is derived from a protein domain of SpA.
33. The fusion protein according to item 32, wherein the second polypeptide moiety comprises an amino acid sequence selected from the group consisting of SEQ ID NOs: 7-9, 28-30 and 61-110 and amino acid sequences having at least 80 % identity thereto, preferably at least 85 % or at least 90 % identity thereto.
34. The fusion protein according to item 33, wherein the second polypeptide moiety comprises an amino acid sequence having at least 85 %, 90 % or 95 % identity to SEQ ID NO: 8 or 64.
35. The fusion protein according to any one of items 1-34, wherein the second polypeptide moiety is capable of binding to the Fc domain of immunoglobulin G (IgG), and/or is capable of binding to the VH3 domain of immunoglobulin G (IgG).
36. The fusion protein according to any one of items 32-34, wherein the second polypeptide moiety has lower binding affinity for an Fc region of trastuzumab compared to the binding of SEQ ID NO:8 for the same Fc region.
37. The fusion protein according to any one of items 32-34, wherein the second polypeptide moiety has lower binding affinity for the VH3 region of trastuzumab compared to the binding of SEQ ID NO:6 for the same VH3 region.
38. The fusion protein according to claim 36 and 37, wherein the second polypeptide moiety has lower binding affinity for an Fc region of trastuzumab compared to the binding of SEQ ID NO:8 for the same Fc region, and has lower binding affinity for the VH3 region of trastuzumab compared to the binding of SEQ ID NO:6 for the same VH3 region.
39. The fusion protein according to item 38, wherein the second polypeptide moiety comprises an amino acid sequence wherein the amino acid in the position corresponding to position X₃₃ of SEQ ID NO:64 is selected from T, S, G, Q, A, E, H, R; the amino acid in the position corresponding to position X₄₀ of SEQ ID NO:64 is selected from E, G, R, D, K, Q, H and S; and the amino acid in the position corresponding to X₅₁ is selected from L, V, S, I, R and G; with the proviso that when X₅₁ is L then X₃₃X₄₀ is selected from AD, HK, EG, ER, GR, AK, AR, PK, RR and KK and when X₅₁ is G then X₃₃X₄₀ is TK.
40. The fusion protein according to any one of items 32-39 wherein the second polypeptide moiety comprises an amino acid sequence derived from a protein domain of SpA and in which, independently,
the amino acid residue in the position corresponding to position 9 of SEQ ID NO: 64 is selected from L, E, R, A and Y, and preferably is A;
the amino acid residue in the position corresponding to position 10 of SEQ ID NO: 64 is selected from L, E, R, A and Y, and preferably is Y
the amino acid residue in the position corresponding to position 11 of SEQ ID NO: 64 is selected from L, E, R, A and Y, and preferably is R;
the amino acid residue in the position corresponding to position 13 of SEQ ID NO: 64 is selected from L, E, R, A and Q, and preferably is L, R, A or Q;
the amino acid residue in the position corresponding to position 14 of SEQ ID NO: 64 is selected from L, E, R, A, Q and K, and preferably is A or R;
the amino acid residue in the position corresponding to position 17 of SEQ ID NO: 64 is selected from L, E, R and A, and preferably is A;
the amino acid residue in the position corresponding to position 18 of SEQ ID NO: 64 is selected from L, E, R and A, and preferably is R;
the amino acid residue in the position corresponding to position 28 of SEQ ID NO: 64 is selected from L, E, R and A, and preferably is R or A;
the amino acid residue in the position corresponding to position 29 of SEQ ID NO: 64 is selected from L, E, R and A, and preferably is R or A;
the amino acid residue in the position corresponding to position 33 of SEQ ID NO: 64 is selected from T, S, G, Q, A, E, H and R, and preferably is S;
the amino acid residue in the position corresponding to position 40 of SEQ ID NO: 64 is selected from E, G, R, D, K, Q, H and S, and preferably is G; and
the amino acid residue in the position corresponding to position 51 of SEQ ID NO: 64 is selected from L, V, S, I, R and G, and preferably is V.
41. The fusion protein according to any one of items 32-39 wherein the second polypeptide moiety comprises an amino acid sequence according to
VDAKFDKEX₉X₁₀X₁₁AX₁₃X₁₄EIX₁₇X₁₈LPNLTEEQRX₂₈X₂₉FIQX₃₃LKDDPSX₄₀SKAILAEAKKX₅₁NDAQAPK (SEQ ID NO: 235)
in which, independently,
X₉ is selected from L, E, R, A and Y, and preferably is A;
X₁₀ is selected from L, E, R, A and Y, and preferably is Y
X₁₁ is selected from L, E, R, A and Y, and preferably is R;
X₁₃ is selected from L, E, R, A and Q, and preferably is L, R, A or Q;
X₁₄ is selected from L, E, R, A, Q and K, and preferably is A or R;
X₁₇ is selected from L, E, R and A, and preferably is A;
X₁₈ is selected from L, E, R and A, and preferably is R;
X₂₈ is selected from L, E, R and A, and preferably is R or A;
X₂₉ is selected from L, E, R and A, and preferably is R or A;
X₃₃ is selected from T, S, G, Q, A, E, H and R, and preferably is S;
X₄₀ is selected from E, G, R, D, K, Q, H and S, and preferably is G;
X₅₁ is selected from L, V, S, I, R and G, and preferably is V.
42. The fusion protein according to item 40 or 41, wherein the second polypeptide moiety comprises, or consists of an amino acid sequence selected from the group consisting of SEQ ID NOs: 7-110 or preferably SEQ ID NOs: 64-110.
43. The fusion protein according to any one of items 1-42, wherein the at least one second polypeptide moiety is located C-terminally of the first polypeptide.
44. The fusion protein according to any one of items 1-43, wherein the at least one second polypeptide, including any additional amino acids, is located at the C-terminus of the fusion protein.
45. The fusion protein according to any one of items 1-44, wherein the second polypeptide is not located at the N-terminus of the fusion protein.
46. The fusion protein according to any one of items 1-45, comprising at least two of said first polypeptide moiety.
47. The fusion protein according to item 46, comprising at least 3, such as up to 6, such as up to 10, of said first polypeptide moiety.
48. The fusion protein according to item 47, wherein said first polypeptide moieties have amino acid sequences that are identical.
49. The fusion protein according to item 47, wherein said first polypeptide moieties have amino acid sequences that are different from each other.
50. The fusion protein according to item 47, wherein said first polypeptide moieties are capable of binding different target entities.
51. The fusion protein according to any one of items 1-50, comprising at least two of said second polypeptide moiety, of which at least one is located at the C terminus of the fusion protein.
52. The fusion protein according to item 51, comprising at least 3, such as up to 10 of said second polypeptide moiety.
53. The fusion protein according to item 51 or 52, wherein said at least two second polypeptide moieties are arranged sequentially at the C terminus of the fusion protein.
54. The fusion protein according to any one of items 51-53, wherein said second polypeptide moieties have amino acid sequences that are identical.
55. The fusion protein according to any one of items 51-53, wherein said second polypeptide moieties have amino acid sequences that are different from each other.
56. The fusion protein according to any one of items 1-55, comprising a further amino acid sequence, optionally selected from the group consisting of: a leader peptide, a signal peptide, an affinity tag, a coupling peptide, a linker peptide, a spacer peptide.
57. The fusion protein according to item 56, wherein said further amino acid sequence is positioned N-terminally or C-terminally of the fusion protein.
58. The fusion protein according to item 56, wherein said further amino acid sequence is a leader peptide or signal peptide, such as a PelB or OmpA signal peptide.
59. The fusion protein according to item 56, wherein said further amino acid sequence comprises a plurality of histidines, such as a (His)₆ sequence.
60. The fusion protein according to any one of items 1-11 and 18-59, wherein the first polypeptide comprises framework regions (FWRs) and complementary determining regions in the following order from N-terminus to C-terminus: [FWR1]-[CDR1]-[FWR2]-[CDR2]-[FWR3]-[CDR3]-[FWR4].
61. The fusion protein according to item 60, wherein the first polypeptide comprises a plurality of framework regions, and each of said framework regions has an amino acid sequence of at least 70 %, such as at least 75%, such as at least 80 %, such as at least 85 % sequence identity to a corresponding framework region of SEQ ID NO:127, 133 or 137.
62. The fusion protein according to any one of items 1-61, wherein the alkaline stability is measured as the ability of the first polypeptide moiety to maintain target binding capacity after exposure to alkaline conditions.
63. The fusion protein according to item 62, wherein exposure to alkaline conditions is repeated exposure to a solution of at least 0.1 M NaOH, such as 0.5 M NaOH, for a time period of 10 min.
64. The fusion protein according to 62 or 63, wherein the target binding capacity is evaluated after repeated cycles of exposure, such as after at least 10 cycles.
65. An isolated nucleic acid sequence encoding a fusion protein according to any one of items 1-64.
66. An expression vector comprising the nucleic acid sequence of item 65.
67. A recombinant host cell for the production of the fusion protein of any one of items 1-64, comprising the expression vector of item 67.
68. A method of producing the fusion protein of any one of items 1-64, comprising
i. providing recombinant host cells according to item 67;
ii. culturing the host cell under conditions enabling expression of the fusion protein; and
iii. isolating the fusion protein.
69. The method of item 68, wherein the cells are prokaryotic cells, such as *E. coli* cells.
70. The method of item 68, wherein the cells are eukaryotic cells, such as mammalian cells, insect cells or yeast cells.
71. The method of item 70, wherein the mammalian cells are Chinese hamster ovary (CHO) cells or human embryonic kidney (HEK) cells.
72. The method of item 70, wherein the yeast cells are *Pichia pastoris* or *Saccharomyces cerevisiae* cells.
73. The method of any one of items 68-72, wherein the fusion protein comprises a histidine tag and step iii comprises purifying the fusion protein by immobilized metal affinity chromatography (IMAC).
74. The method of any one of items 68-72, wherein step iii comprises purifying the fusion protein by affinity chromatography.
75. The method of item 74, wherein the affinity chromatography uses an affinity ligand that binds to a framework region of the first polypeptide moiety.
76. The method of item 75, wherein the affinity chromatography uses an affinity ligand based on a protein domain of SpA, wherein the affinity ligand binds to a VH region, such as VH3, of the first polypeptide moiety.
77. The method of item 74, wherein the affinity chromatography uses an affinity ligand that binds to the second polypeptide moiety of the fusion protein.
78. The method of item 77, wherein the affinity ligand binds to an affinity tag included in or added to the amino acid sequence of the second polypeptide moiety.
79. Use of the fusion protein according to any one of items 1-64 for *in vitro* capture of said target entity.
80. Use according to item 79 for the detection of said target entity within a sample.
81. Use according to item 79 for separation of said target entity from other components of a sample.
82. Use according to item 81 for analytical separation of the target entity.
83. Use according to item 81 for preparative purification of the target entity.
84. An adsorbent material comprising the fusion protein according to any one of items 1-64 coupled to a support.
85. The adsorbent material according to item 84, wherein the fusion protein is coupled to the support via the second polypeptide.
86. The adsorbent material according to item 84 or 85, wherein the support is a surface.
87. The adsorbent material according to any one of items 84-86, wherein the support is selected from a chip, a plate, a well, a sheet, a fiber, a particle and a bead.
88. The adsorbent material according to any one of items 84-87, wherein the support comprises a polymeric material.
89. The adsorbent material according to any one of items 84-88, wherein the support comprises a polysaccharide-based material, such as agar, agarose or an agarose derivative.
90. The adsorbent material according to item 89, wherein the support comprises crosslinked agarose.
91. The adsorbent material according to any one of items 84-88, wherein the support is selected from the group consisting of a fibrous matrix, a membrane, a filter and a monolith.
92. The adsorbent material according to item 91, wherein the support material is a fibrous material.
93. The adsorbent material according to item 92, wherein the support material is a fibrous matrix, such as a nonwoven fibrous matrix.
94. The adsorbent material according to any one of items 84-93, wherein the support is a chromatography matrix.
95. Use of the adsorbent material according to any one of items 84-94 for binding of the target entity.
96. Use according to item 95 for the detection of said target entity within a sample.
97. Use according to item 95 for separation of said target entity from other components of a sample.
98. Use according to item 97 for analytical separation of the target entity.
100. Use according to item 97 for preparative purification of the target entity.
101. Use of an α-helix-containing polypeptide domain to improve the *in vitro* alkaline stability of an antigen-binding polypeptide by fusing the α-helix-containing polypeptide to the C-terminus of said antigen-binding polypeptide.
102. Use according to item 101, wherein the α-helix-containing polypeptide domain is a second polypeptide moiety as defined in any one of items 1-64.
103. Use according to item 101, wherein the antigen-binding polypeptide is a first polypeptide moiety as defined in any one of items 1-64.
104. Use according to any one of items 101-103, wherein the antigen-binding polypeptide alone retains 75 % or less, such as 50 % or less, of its target binding capacity after at most 20 hours, such as after 10 hours, of exposure to 0.5 M NaOH, or after at most 120 cycles, such as after 60 cycles, of repeated NaOH exposure wherein a cycle of NaOH exposure involves contact with 0.5 M NaOH for 10 minutes.
105. A method of separation, comprising the steps of
(a) Providing an adsorbent material according to any one of items 84-94, wherein the fusion protein has a binding capacity for a target entity,
(b) Contacting the adsorbent material with a liquid sample comprising said target entity under conditions allowing the target entity to bind to the first polypeptide of the fusion protein,
(c) Optionally washing the adsorbent material,
(d) Eluting the target entity from the adsorbent material, and
(e) Cleaning the adsorbent material with a cleaning liquid.
106. The method of item 105, wherein the cleaning liquid is alkaline, and preferably comprises from 0.05 to 0.5 M of NaOH or KOH.
107. The method of item 105 or 106, wherein steps (a)-(e) are repeated at least 10 times, such as at least 20 times.
108. The method of item 107, wherein after 10 cycles of contact with alkaline cleaning liquid, the fusion protein retains at least 50 % such as at least 60 %, such as at least 80 %, such as at least 90 %, of the initial target entity binding capacity.
109. The method of item 107, wherein after 20 cycles of contact with alkaline cleaning liquid, the fusion protein retains at least 50 %, such as at least 60 %, such as at least 80 %, such as at least 90 %, of its initial target entity binding capacity.
110. The method of item 107, wherein, after 10 cycles of contact with alkaline cleaning liquid, the fusion protein retains at least 50 % such as at least 60 %, such as at least 80 %, such as at least 90 %, such as at least 95 %, such as at least 98 %, of the target entity binding capacity of the 2^{nd} cycle.
111. The method of item 107, wherein after 20 cycles of contact with alkaline cleaning liquid, the fusion protein retains at least 50 %, such as at least 60 %, such as at least 80 %, such as at least 90 %, such as at least 95 %, of the target entity binding capacity of the 2^{nd} cycle.

**Table 19. Exemplary amino acid sequences**

| **SEQ ID NO** | **Amino acid sequence** |
|---|---|
| 1 | ADNNFNKEQQNAFYEILNMPNLNEEQRNGFIQSLKDDPSQSANLLAEAKKLNESQAPK |
| 2 | ADNKFNKEQQNAFYEILHLPNLNEEQRNGFIQSLKDDPSQSANLLAEAKKLNDAQAPK |
| 3 | ADNKFNKEQQNAFYEILHLPNLTEEQRNGFIQSLKDDPSVSKEILAEAKKLNDAQAPK |
| 4 | ADAQQNKFNKDQQSAFYEILNMPNLNEEQRNGFIQSLKDDPSQSTNVLGEAKKLNESQAPK |
| 5 | AQHDEAQQNAFYQVLNMPNLNADQRNGFIQSLKDDPSQSANVLGEAQKLNDSQAPK |
| 6 | VDNKFNKEQQNAFYEILHLPNLNEEQRNAFIQSLKDDPSQSANLLAEAKKLNDAQAPK |
| 7 | VDAKFDKEQQNAFYEILHLPNLTEEQRNAFIQSLKDDPSQSANLLAEAKKLNDAQAPK |
| 8 | VDAKFDKEAQEAFYEILHLPNLTEEQRNAFIQSLKDDPSVSKAILAEAKKLNDAQAPK |
| 9 | VDAKFDKEAQEAFYEILHLPNLTEEQRNAFIQSLKDDPSGSKAILAEAKKVNDAQAPK |
| 28 | VDAKFDKEQQNAFYEILHLPNLTEEQRNAFIQALKDDPSRSANLLAEAKKINDAQAPK |
| 29 | VDAKFDKEQQNAFYEILHLPNLTEEQRNAFIQSLKDDPSGSANLLAEAKKVNDAQAPK |
| 30 | VDAKFDKEQQNAFYEILHLPNLTEEQRNAFIQALKDDPSGSANLLAEAKKRNDAQAPK |
| 61 | VDAKFDKEAYRALAEIARLPNLTEEQRAGFIQSLKDDPSQSANLLAEAKKLNDAQAPK |
| 62 | VDAKFDKEAYRALAEIARLPNLTEESRAGFIQSLKDDPSQSANLLAEAKKLNDAQAPK |
| 63 | VDAKFDKEAYRALAEIARLPNLTEEQRAGFIQSLKDDPSVSKAILAEAKKLNDAQAPK |
| 64 | VDAKFDKEAYRALAEIARLPNLTEEQRAAFIQSLKDDPSGSKAILAEAKKVNDAQAPK |
| 65 | VDAKFDKEAYRALAEIARLPNLTEEQRLAFIQSLKDDPSGSKAILAEAKKVNDAQAPK |
| 66 | VDAKFDKEAYRALAEIARLPNLTEEQREAFIQSLKDDPSGSKAILAEAKKVNDAQAPK |
| 67 | VDAKFDKEAYRALAEIARLPNLTEEQRRAFIQSLKDDPSGSKAILAEAKKVNDAQAPK |
| 68 | VDAKFDKEAYRALAEIARLPNLTEEQRALFIQSLKDDPSGSKAILAEAKKVNDAQAPK |
| 69 | VDAKFDKEAYRALAEIARLPNLTEEQRAEFIQSLKDDPSGSKAILAEAKKVNDAQAPK |
| 70 | VDAKFDKEAYRALAEIARLPNLTEEQRARFIQSLKDDPSGSKAILAEAKKVNDAQAPK |
| 71 | VDAKFDKEAYRALAEIARLPNLTEEQRAAFIQTLKDDPSESKAILAEAKKRNDAQAPK |
| 72 | VDAKFDKEAYRALAEIARLPNLTEEQRAAFIQALKDDPSRSKAILAEAKKINDAQAPK |
| 73 | VDAKFDKEAYRALAEIARLPNLTEEQRAAFIQALKDDPSGSKAILAEAKKRNDAQAPK |
| 74 | VDAKFDKEAYRALAEIARLPNLTEEQRAAFIQQLKDDPSDSKAILAEAKKINDAQAPK |
| 75 | VDAKFDKEAYRALAEIARLPNLTEEQRAAFIQGLKDDPSRSKAILAEAKKLNDAQAPK |
| 76 | VDAKFDKEAYRALAEIARLPNLTEEQRAAFIQHLKDDPSKSKAILAEAKKLNDAQAPK |
| 77 | VDAKFDKEAYRALAEIARLPNLTEEQRAAFIQELKDDPSGSKAILAEAKKLNDAQAPK |
| 78 | VDAKFDKERYRALAEIARLPNLTEEQRAAFIQSLKDDPSGSKAILAEAKKVNDAQAPK |
| 79 | VDAKFDKEAYRALAEILRLPNLTEEQRAAFIQSLKDDPSGSKAILAEAKKVNDAQAPK |
| 80 | VDAKFDKEAYRALAEIAELPNLTEEQRAAFIQSLKDDPSGSKAILAEAKKVNDAQAPK |
| 81 | VDAKFDKEAYRALAEIALLPNLTEEQRAAFIQSLKDDPSGSKAILAEAKKVNDAQAPK |
| 82 | VDAKFDKEAYRALAEIELLPNLTEEQRAAFIQSLKDDPSGSKAILAEAKKVNDAQAPK |
| 83 | VDAKFDKEAYRALAEIEALPNLTEEQRAAFIQSLKDDPSGSKAILAEAKKVNDAQAPK |
| 84 | VDAKFDKEAYRALAEILALPNLTEEQRAAFIQSLKDDPSGSKAILAEAKKVNDAQAPK |
| 85 | VDAKFDKEAYRALAEILELPNLTEEQRAAFIQSLKDDPSGSKAILAEAKKVNDAQAPK |
| 86 | VDAKFDKEAYRALAEIRALPNLTEEQRAAFIQSLKDDPSGSKAILAEAKKVNDAQAPK |
| 87 | VDAKFDKEAYRALAEIRELPNLTEEQRAAFIQSLKDDPSGSKAILAEAKKVNDAQAPK |
| 88 | VDAKFDKEAYRALAEIRLLPNLTEEQRAAFIQSLKDDPSGSKAILAEAKKVNDAQAPK |
| 89 | VDAKFDKEAYRALAEIRRLPNLTEEQRAAFIQSLKDDPSGSKAILAEAKKVNDAQAPK |
| 90 | VDAKFDKEAYRALQEIARLPNLTEEQRAAFIQSLKDDPSGSKAILAEAKKVNDAQAPK |
| 91 | VDAKFDKEAYRAELEIARLPNLTEEQRAAFIQSLKDDPSGSKAILAEAKKVNDAQAPK |
| 92 | VDAKFDKEAYRARAEIARLPNLTEEQRAAFIQSLKDDPSGSKAILAEAKKVNDAQAPK |
| 93 | VDAKFDKEAYRAAREIARLPNLTEEQRAAFIQSLKDDPSGSKAILAEAKKVNDAQAPK |
| 94 | VDAKFDKEAYRAQAEIARLPNLTEEQRAAFIQSLKDDPSGSKAILAEAKKVNDAQAPK |
| 95 | VDAKFDKEAYRALEEIARLPNLTEEQRAAFIQSLKDDPSGSKAILAEAKKVNDAQAPK |
| 96 | VDAKFDKEAYRAEREIARLPNLTEEQRAAFIQSLKDDPSGSKAILAEAKKVNDAQAPK |
| 97 | VDAKFDKEAYRARLEIARLPNLTEEQRAAFIQSLKDDPSGSKAILAEAKKVNDAQAPK |
| 98 | VDAKFDKEAYRAAQEIARLPNLTEEQRAAFIQSLKDDPSGSKAILAEAKKVNDAQAPK |
| 99 | VDAKFDKEAYRAQEEIARLPNLTEEQRAAFIQSLKDDPSGSKAILAEAKKVNDAQAPK |
| 100 | VDAKFDKEAYRAQQEIARLPNLTEEQRAAFIQSLKDDPSGSKAILAEAKKVNDAQAPK |
| 101 | VDAKFDKEAYRARREIARLPNLTEEQRAAFIQSLKDDPSGSKAILAEAKKVNDAQAPK |
| 102 | VDAKFDKERYRAAREIERLPNLTEEQREAFIQSLKDDPSGSKAILAEAKKRNDAQAPK |
| 103 | VDAKFDKEERLAQAEIAALPNLTEEQRALFIQALKDDPSRSKAILAEAKKINDAQAPK |
| 104 | VDAKFDKEYAEARLEIRALPNLTEEQRARFIQALKDDPSGSKAILAEAKKVNDAQAPK |
| 105 | VDAKFDKEERAAEREIARLPNLTEEQRRRFIQSLKDDPSGSKAILAEAKKVNDAQAPK |
| 106 | VDAKFDKERALARREIELLPNLTEEQRAAFIQALKDDPSGSKAILAEAKKRNDAQAPK |
| 107 | VDAKFDKELYRALAEIARLPNLTEEQRALFIQSLKDDPSGSKAILAEAKKSNDAQAPK |
| 108 | VDAKFDKEAYLALAEIARLPNLTEEQRALFIQSLKDDPSSSKAILAEAKKGNDAQAPK |
| 109 | VDAKFDKEAYRALAEIARLPNLTEEQRAAFIQRLKDDPSHSKAILAEAKKVNDAQAPK |
| 110 | VDAKFDKEAYAAKAEIARLPNLTEEQRAAFIQSLKDDPSQSKAILAEAKKVNDAQAPK |
| 127 | EVQLVESGGGLVQAGGSLRLSCAASGRTFSSYAMGWFRQAPGKEREFVVAINWSSGSTYYADSV KGRFTISRDNAKNTMYLQMNSLKPEDTAVYYCAAGYQINSGNYNFKDYEYDYWGQGTQVTVSS |
| 133 | QVQLQESGGGLVQPGGSLRLSCAASGRTLSDYTMGWFRQAPGKQREFVAAISWSGAYTKYTDS VKGRFTISRDNAKTTVYLQMNSLKPEDTAVYYCAAGPTGLLSKKATPADYDYWGQGTQVTVSS |
| 137 | QVQLQESGGGSVQAGGSLRLSCVASGRTLSDYTMGWFRQPPGKAREFVAAISWSGAYTKYADS VKGRFTISRDNAKNTVYLQMNSLKPEDTAVYYCAAGPTGLLSKKATPADYDYWGQGTQVTVSS |
| 139 | QVQLQQSGGGSVQAGGSLRLSCTISGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |

### REFERENCES

WO2003080655A1
WO 2016/079033A1
WO2022/013272
WO 2023/046886
PCT/EP23/056401
S Hjerten: Biochim Biophys Acta 79(2), 393-398 (1964)
US6602990
US7396467
WO2019/137869
WO2018/011600
Schmitz et al, Structure 21, 1214-1224 (2013)
Fleetwood et al., Cell.Mol. Life Sci. 70:1081-1093 (2013)

## Claims

1. A fusion protein comprising at least one first polypeptide moiety and at least one second polypeptide moiety, wherein the first polypeptide moiety is a single-chain polypeptide capable of binding a target entity, and the second polypeptide moiety comprises a single-chain α-helix-containing domain, wherein the second polypeptide moiety does not have binding affinity for said target entity, and wherein presence of the second polypeptide moiety improves at least one property of the fusion protein as compared to the property of the first polypeptide moiety alone, wherein said improved property is selected from the group consisting of: alkaline stability, recombinant protein expression and coupling to a support.

2. The fusion protein according to claim 1, wherein improved alkaline stability is represented by the fusion protein as a whole having an alkaline stability that is increased as compared to the alkaline stability of the first polypeptide moiety alone.

3. The fusion protein according to claim 1 or 2, wherein the first polypeptide moiety is a natural or synthetic antibody domain or antibody fragment or a modified variant thereof, such as is a single-chain variable fragment or a single-domain antibody (sdAb), or a variant thereof.

4. The fusion protein according to claim 1 or 2, wherein the first polypeptide moiety comprises an α-helix bundle domain, such as an albumin-binding domain or a polypeptide which is or is derived from a protein domain of staphylococcal protein A (SpA).

5. The fusion protein according to any one of claims 1-4, wherein the first polypeptide moiety alone has an alkali stability that is lower than the second polypeptide moiety alone.

6. The fusion protein according to any one of claims 1-5, wherein the second polypeptide moiety is an α-helix bundle domain.

7. The fusion protein according to claim 6, wherein the second polypeptide is or is derived from a protein domain of SpA.

8. The fusion protein according to claim 7, wherein the second polypeptide moiety comprises an amino acid sequence selected from the group consisting of SEQ ID NOs: 7-9, 28-30 and 61-110 and derivatives thereof having at least 80 % identity thereto, preferably at least 85 % or at least 90 % identity thereto.

9. The fusion protein according to claim 7 or 8, wherein the second polypeptide moiety has lower binding affinity for an Fc region of trastuzumab compared to the binding of SEQ ID NO:8 for the same Fc region, and has lower binding affinity for the VH3 region of trastuzumab compared to the binding of SEQ ID NO:6 for the same VH3 region.

10. The fusion protein according to any one of claims 7-9, wherein the second polypeptide moiety comprises an amino acid sequence derived from a protein domain of SpA and in which
the amino acid residue in the position corresponding to position 9 of SEQ ID NO: 64 is selected from L, E, R, A and Y, and preferably is A;
the amino acid residue in the position corresponding to position 10 of SEQ ID NO: 64 is selected from L, E, R, A and Y, and preferably is Y
the amino acid residue in the position corresponding to position 11 of SEQ ID NO: 64 is selected from L, E, R, A and Y, and preferably is R;
the amino acid residue in the position corresponding to position 13 of SEQ ID NO: 64 is selected from L, E, R, A and Q, and preferably is L, R, A or Q;
the amino acid residue in the position corresponding to position 14 of SEQ ID NO: 64 is selected from L, E, R, A, Q and K, and preferably is A or R;
the amino acid residue in the position corresponding to position 17 of SEQ ID NO: 64 is selected from L, E, R and A, and preferably is A;
the amino acid residue in the position corresponding to position 18 of SEQ ID NO: 64 is selected from L, E, R and A, and preferably is R;
the amino acid residue in the position corresponding to position 28 of SEQ ID NO: 64 is selected from L, E, R and A, and preferably is R or A;
the amino acid residue in the position corresponding to position 29 of SEQ ID NO: 64 is selected from L, E, R and A, and preferably is R or A;
the amino acid residue in the position corresponding to position 33 of SEQ ID NO: 64 is selected from T, S, G, Q, A, E, H and R, and preferably is S;
the amino acid residue in the position corresponding to position 40 of SEQ ID NO: 64 is selected from E, G, R, D, K, Q, H and S, and preferably is G;
the amino acid residue in the position corresponding to position 51 of SEQ ID NO: 64 is selected from L, V, S, I, R and G, and preferably is V.

11. The fusion protein according to any one of claims 1-10, wherein the at least one second polypeptide, including any additional amino acids, is located at the C-terminus of the fusion protein.

12. The fusion protein according to any one of claims 1-11, which is a multimeric fusion protein comprising at least two of said first polypeptide moiety and/or at least two of said second polypeptide moiety.

13. Use of the fusion protein according to any one of claims 1-12 for *in vitro* capture of said target entity, such as for the detection of said target entity within a sample.

14. Use according to claim 13 for separation of said target entity from other components of a sample.

15. An adsorbent material comprising the fusion protein according to any one of claims 1-12 coupled to a support.

16. The adsorbent material according to claim 15, wherein the support is selected from a chip, a plate, a well, a sheet, a fiber, a particle, a bead, a fibrous matrix, a membrane, a filter and a porous monolith.

17. The adsorbent material according to claim 15 or 16, wherein the support is a chromatography matrix.

18. Use of the adsorbent material according to any one of claims 15-17 for detection of said target entity within a sample.

19. Use the adsorbent material according to any one of claims 15-17 for separation of said target entity from other components of a sample.

20. Use of an alkaline stable α-helix-containing polypeptide domain to improve the *in vitro* alkaline stability of an antigen-binding polypeptide by fusing the α-helix-containing polypeptide to the C-terminus of said antigen-binding polypeptide.

21. Use according to claim 20, wherein the α-helix-containing polypeptide domain is a second polypeptide moiety as defined in any one of claims 1-12, and/or the antigen-binding polypeptide is a first polypeptide moiety as defined in any one of claims 1-12.

22. A method of separation, comprising the steps of
(a) Providing an adsorbent material according to any one of claims 15-17, wherein the fusion protein has a binding capacity for a target entity,
(b) Contacting the adsorbent material with a liquid sample comprising said target entity under conditions allowing the target entity to bind to the first polypeptide of the fusion protein,
(c) Optionally washing the adsorbent material,
(d) Eluting the target entity from the adsorbent material, and
(e) Cleaning the adsorbent material with a cleaning liquid, such as an alkaline cleaning liquid.

23. The method of claim 22, wherein steps (a)-(e) are repeated at least 10 times, such as at least 20 times.
